# EUROPÄISCHE PATENTSCHRIFT

(11) **EP 1 232 257 B1**
(45) Veröffentlichungstag und Bekanntmachung des Hinweises auf die Patenterteilung: **14.10.2009**
(21) Anmeldenummer: 00983106.6
(22) Anmeldetag: 03.11.2000
(51) Int. Cl.: C12N 15/00

(54) **MODULARE TRANSPORTSYSTEME FÜR MOLEKULARE SUBSTANZEN UND DEREN HERSTELLUNG UND VERWENDUNG**
MODULAR TRANSPORT SYSTEMS FOR MOLECULAR SUBSTANCES AND PRODUCTION AND USE THEREOF
SYSTEMES DE TRANSPORT MODULAIRES POUR SUBSTANCES MOLECULAIRES ET FABRICATION ET UTILISATION DE CES SYSTEMES

(30) Priorität: 03.11.1999 DE 19952957
(43) Veröffentlichungstag der Anmeldung: 21.08.2002
(73) Patentinhaber: ACGT Progenomics AG, 06120 Halle (Saale) (DE)
(72) Erfinder: BÖHM, Gerald, 06114 Halle (Saale) (DE); RUDOLPH, Rainer, 06120 Halle (Saale) (DE); SCHMIDT, Ulrich, West Leederville, WA 6007 (AU); ESSER, Dirk, Cambridge CB1 9EW (GB)
(74) Vertreter: Behnisch, Werner
(86) Internationale Anmeldenummer: PCT/EP2000/010876
(87) Internationale Veröffentlichungsnummer: WO 2001/032851

(56) Entgegenhaltungen:
- WO-A-00/00224
- WO-A-96/13599
- WO-A-97/43431
- WO-A-98/48841
- WO-A-99/13905
- WO-A-99/50424
- US-A- 5 766 897
- AN K. ET AL: "USE OF THE BACULOVIRUS SYSTEM TO ASSEMBLE POLYOMAVIRUS CAPSID-LIKE PARTICLES WITH DIFFERENT POLYOMAVIRUS STRUCTURERAL PROTEINS: ANALYSIS OF THE RECOMBINANT ASSEMBLED CAPSID-LIKE PARTICLES" J GENERAL VIROLOGY, Bd. 80, April 1999 (1999-04), Seiten 1009-1016, XP002176023
- MÜLLER M ET AL: "Chimeric papillomavirus-like particles" VIROLOGY, ACADEMIC PRESS,ORLANDO, US, Bd. 234, 21. Juli 1997 (1997-07-21), Seiten 93-111, XP002091857 ISSN: 0042-6822
- FORSTOVA J ET AL: "POLYOMA VIRUS PSEUDOCAPSIDS AS EFFICIENT CARRIERS OF HETEROLOGOUS DNA INTO MAMMALIAN CELLS" HUMAN GENE THERAPY, Bd. 6, Nr. 3, 1. März 1995 (1995-03-01), Seiten 297-306, XP000645479 ISSN: 1043-0342
- TANAKA S, MIKHAILOV M, ROY P: "Synthesis of bluetongue virus chimeric VP3 molecules and their interactions with VP7 protein to assemble into virus" VIROLOGY , Bd. 214, 20. Dezember 1995 (1995-12-20), Seiten 593-601, XP000986265
- LOUDON PT, ROY P: "Interaction of nucleic acids with core-like and subcore-like particles of bluetongue virus." VIROLOGY, Bd. 191, November 1992 (1992-11), Seiten 231-236, XP000986273
- LIU HM, BOOTH TF, ROY P: "Interactions between bluetongue virus core and capsid proteins translated in vitro." J GEN VIROL , Bd. 73, Oktober 1993 (1993-10), Seiten 2577-2584, XP000999048
- REYNOLDS PN, DMITRIEV I, CURIEL DT: "insertion of rgd motif into the hi loop of adenovirus fiber protein alters the distribution of transgene expression of the systemically administered vector" GENE THERAPY, Bd. 6, Juli 1999 (1999-07), Seiten 1336-1339, XP000996471
- LI M. ET AL: "POLYOMAVIRUS VP1 PHOSPHORYLATION: COEXPRESSION WITH THE VP2 CAPSID PROTEIN MODULATES VP1 PHOSPHORYLATION IN SF9 INSECT CELLS." PNAS, Bd. 92, Juni 1995 (1995-06), Seiten 5992-5996, XP002176024
- DELOS S. ET AL: "EXPRESSION OF THE POLYOMAVIRUS CAPSID PROTEINS VP2 AND VP3 IN ESCHERICHIA COLI: IN VITRO INTERACTIONS WITH RECOMBINANT VP1 CAPSOMERS" J VIROLOGY, Bd. 69, Nr. 12, Dezember 1995 (1995-12), Seiten 7734-7742, XP002176025
- SCHMIDT U. ET AL: "MECHANISM OF ASSEMBLY OF RECOMBINANT MURINE POLYOMAVIRUS-LIKE PARTICLES" J VIROLOGY, Bd. 74, Nr. 4, Februar 2000 (2000-02), Seiten 1658-1662, XP002176026
- TOMILSON E, ROLLAND AP: "Controllable gene therapy Pharmaceutics of non-viral gene delivery systems" JOURNAL OF CONTROLLED RELEASE, Bd. 39, 1996, Seiten 357-372, XP004037341

## Beschreibung

Die vorliegende Erfindung betrifft Transportsysteme für molekulare Substanzen, wobei die Transportsysteme mosaikartig aus separat rekombinant hergestellten Teileinheiten (Einzelbausteinen) zusammengesetzt sind, sowie Verfahren zur Herstellung der modularen Transportsysteme und deren Verwendung.

### Gebiet der Erfindung und Stand der Technik

Die medizinische Gentherapie ermöglicht eine dauerhafte und schonende Therapie für eine Reihe von schweren Erkrankungen, und stellt nach allgemeiner Ansicht eine wichtige Alternative zu traditionellen medizinischen Methoden wie beispielsweise Chemotherapie dar. Das grundsätzliche Verfahren basiert auf dem gezielten Einbringen von therapeutisch wirksamen, meist nukleinsäurebasiertem Material in somatische Zellen. Das Ziel gentherapeutischer Heilmethoden ist dabei entweder eine Therapie von angeborenen genetischen Defekten (klassische Gentherapie), eine Therapie von durch Infektion erworbenen Erkrankungen (beispielsweise EBV-Infektion, HIV-Infektion), oder eine Tumortherapie. Unter dieser Prämisse werden die verschiedenartigen Konzepte zur Therapie schwerer Erkrankungen als gentherapeutische Heilmethoden zusammengefaßt.

Die *klassische Gentherapie* befaßt sich mit (vererbten) genetischen Defekten und den damit assoziierten Erkrankungen, die auf eine meist singuläre Ursache (in der Regel ein disfunktionales Protein) zurückgeführt werden können. Zu diesen monokausalen Erkrankungen zählen beispielsweise ADA-Defizienz, Hämophilie, Duchenne-Muskeldystrophie, und Cystische Fibrose, für deren Therapie seit etwa 1990 gentherapeutische Methoden erprobt werden. Ziel ist der Ersatz bzw. die Komplementierung eines fehlenden Proteins nach spezifischem Einbringen von geeignetem genetischen Material in Körperzellen. *Infektiologische Gentherapie* versucht demgegenüber die Therapie von viralen oder bakteriellen Infektionen durch Eliminierung des entsprechenden Erregers; die von Viren befallenen Zellen sollen dabei in der Regel therapiert oder abgetötet werden, bevor es zur Reifung neuer infektiöser Viren kommt. Hauptzielrichtung derzeitiger Forschungsansätze ist die HIV-Infektion. Die *Gentherapie von Tumorerkrankungen* beabsichtigt im Gegensatz dazu das Einbringen von toxischen Substanzen spezifisch in neoplastische Zellen, oder den Einsatz analoger Prinzipien (Apoptose, Immunstimulation) zur selektiven Eliminierung von malignen Zellen.

Grundsätzlich werden bei der Gentherapie nach dem Stand der Technik zwei methodisch unterschiedliche Ansätze diskutiert: (i) Isolierte Zellen werden extrakorporal (*in vitro*) mit dem genetischen Material transformiert, oft durch zelltyp-unspezifische Retroviren; danach werden die transformierten Zellen in den Spender-Körper reimplantiert. (ii) Die Zielzellen werden *in vivo* mit spezifischen Vektoren infiziert; hier werden insbesondere replikationsdefiziente Retroviren oder Adenoviren bzw. Adeno-assoziierte Viren verwendet. Aber auch physikalische Systeme wie kondensierte DNA, virusähnliche Partikel, und andere werden eingesetzt.

Das eingebrachte genetische Material, in der Hauptsache DNA, kann entweder ins Chromosom integrieren (permanente Expression, beispielsweise für die Therapie angeborener, monokausaler Erkrankungen) oder transient exprimiert werden; dies ist beispielsweise bei einer infektiologischen Therapie oder einer Tumor-Therapie ausreichend. Anstelle von DNA ist in diesen Fällen auch das Einbringen von antisense-RNA oder von Ribozymen möglich, oder es werden therapeutische Wirkstoffe wie Peptide oder Proteine verwendet

Trotz erfolgreicher experimenteller Ansätze zur Gentherapie nach dem Stand der Technik sind jedoch auch eine Reihe von Problemen bekannt. So haben beispielsweise replikationskompetente Retroviren als Vektoren zu schweren Erkrankungen in Tiermodellen geführt (W.F. Anderson, Hum. Gene Ther. 4, 1-2, 1993; Otto, Jones-Trower, Vanin, Stambaugh, Mueller, Andersen & McGarrity, Hum. Gene Ther. 5, 567-575, 1994). Die Effizienz der Zelltransformation bei in vivo-Methoden ist oft nur gering, und die Spezifität des zellulären Targetings bei Verwendung von Retroviren als Vektoren in der Regel nicht gegeben. Die Systeme sind aufwendig bezüglich der Herstellung nach GMP-Bedingungen; die Produktion von viralen Vektoren mit Hilfe von Verpackungszellinien zieht wiederum eine aufwendige Analytik der Präparationen nach sich. Diese und andere, nachfolgend beschriebene Nachteile nach dem Stand der Technik sollen erfindungsgemäß durch neuartige, modulare Vektorsysteme als Transportvehikel für molekulare Substanzen beseitigt werden.

Nahezu alle bekannten Viren und Phagen besitzen ein aus mindestens einem oder mehreren Proteinen aufgebautes Kapsid, in dem das virale Genom eingeschlossen ist. Die Kapside weisen eine definierte, für ein Virus bzw. einen Phagen charakteristische Morphologie auf. Besonders häufig werden ikosaedrische oder filamentöse Kapside gebildet. Einen Überblick über die Morphologien bekannter Viren gibt Tabelle 1. Es gibt zahlreiche Beispiele, daß derartige Kapside *in vitro* aus isolierten Virusproteinen aufgebaut werden können, ohne daß das Genom des Virus oder zelluläre Faktoren anwesend sein müssen. Die daraus resultierenden Strukturen, bestehend aus leeren oder gefüllten Proteinhüllen, werden als virusähnliche oder virusanaloge Partikel bezeichnet.

**Tabelle 1. Morphologie bekannter Viren und Virusfamilien**

| Morphologie | Vertreter (Viren bzw. Phagen) |
|---|---|
| Amorph bzw. unbekannt | Umbravirus; Tenuivirus |
| bacilliform | Baculoviridae; Badnavirus; Barnaviridae; Filoviridae; Rhabdoviridae |
| filamentös | Capillovirus, Carlavirus; Closterovirus; Furovirus; Inoviridae; Lipothrixviridae; Potexvirus; Potyviridae; Tobamovirus; Tobravirus; Polydnaviridae |
| hellkal | Hordeivirus; Paramyxoviridae; Trichovirus |
| ikosaedrisch | Adenoviridae; Astroviridae; Birnaviridae; Bromoviridae; Caliciviridae; Caulimovirus; Circoviridae; Comoviridae; Corticoviridae; Dianthovirus; Enamovirus; Hepadnaviridae; Herpesviridae; Idaeovirus; Iridoviridae; Lviviridae; Luteovirus; Machlomovirus; Marafivirus; Microviridae; Necrovirus; Nodaviridae; Papovaviridae; Partitiviridae; Parvoviridae; Phycodnaviridae; Picornaviridae; Reoviridae; Rhizidiovirus; Sequiviridae; Sobemovirus; Tectiviridae; Tetraviridae; Tombusviridae; Totiviridae; Tymovirus |
| isometrisch | Cystoviridae; Geminiviridae |
| oval | Poxviridae |
| pleomorph | Coronaviridae; Hypoviridae; Plasmaviridae |
| sphärisch | Arenaviridae; Arterivirus; Bunyaviridae; Flaviviridae; Orthomyxoviridae; Retroviridae; Togaviridae |
| zitronenförmig | Fuselloviridae |
| unklassifizierte hyperthermophile Phagen und Viren | Bacilloviridae; Guttaviridae |
| Phagen mit Schwanzfortsatz | Myoviridae; Podoviridae; Siphoviridae |

Bei der Verwendung solcher virusähnlicher Partikel als Transportvehikel müssen die verwendeten Hüllproteine in einem geeignetem Expressionssystem produziert werden. Dabei werden vor allem eukaryontische Systeme, wie beispielsweise baculovirusinfizierte Insektenzellen oder prokaryontische Systeme, wie beispielsweise rekombinante *E. coli*, verwendet. Mit eukaryontischen Expressionssystemen werden komplette virusähnliche Partikel innerhalb der Zellen gebildet; darüberhinaus lassen sich Virushüllen herstellen, die aus verschiedenen Virusproteinen, beispielsweise Polyomavirus-Hüllproteinen VP1 und VP2 aufgebaut sind (An, Gillock, Sweat, Reeves & Consigli, J. Gen. Virol. 80, 1009-1016, 1999). Nachteile dieser Verfahren ist vor allem die aufwendige, kostenintensive Herstellung der Viruskapside. Bei der Expression von Virushüllproteinen in *E. coli* werden in den Zellen keine kompletten Viruskapside, sondern Kapsomere gebildet, die aus den Zellen isoliert und *in vitro* zu virusähnlichen Partikeln assembliert werden müssen. Zwar besteht hier die Möglichkeit, große Mengen der Hüllproteine zu gewinnen, jedoch muß ein geeignetes Protokoll zur *in vitro-*Assemblierung des jeweiligen Hüllproteins gefunden werden. Bei diesen Verfahren besteht weiterhin die Möglichkeit, Viruskapside aus verschiedenen natürlich in dem jeweiligen Virus vorkommenden Virushüllproteinen aufzubauen, beispielsweise können Herpes-Simplex-Viruskapside aus VP5, VP19C und VP23 (Newcomb, Homa, Thomsen, Trus, Cheng, Steven, Booy & Brown, J. Virol. 73, 4239-4250, 1999) aufgebaut werden. Die Möglichkeit, Viruskapside aus verschiedenen, jeweils modifizierten Teileinheiten aufzubauen, ist jedoch im Stand der Technik nicht beschrieben.

Das VP1-Protein von Polyomavirus assembliert *in vitro* unter geeigneten Lösungsmittelbedingungen spontan zu einer virusähnlichen Hülle; diese Eigenschaft des Wildtyp-Proteins ist nach dem Stand der Technik bereits bekannt. Dieser Prozeß kann grundsätzlich dazu verwendet werden, ein molekulares Transportvehikel für den gezielten Transfer von Molekülen (beispielsweise von Therapeutika), die in die Hülle des virusähnlichen Partikels eingeschlossen werden, herzustellen. Neben wichtigen strukturellen Arbeiten ist das Polyoma-VP1-Protein bezüglich seiner molekularbiologischen und pathologischen Eigenschaften außerordentlich gut untersucht. Die publizierten Arbeiten schließen sowohl die Herstellung, Reinigung und Charakterisierung, als auch die Struktur und die Assemblierung des Proteins ein (Rayment, Baker & Caspar, Nature 295, 110-115, 1982; Garcea & Benjamin, Proc. Natl. Acad. Sci. U.S.A. 80, 3613-3617, 1983, Slilaty & Aposhian, Science 220, 725-727, 1983; Leavitt, Roberts & Garcea, J. Biol. Chem. 260, 12803-12809, 1985; Moreland, Montross & Garcea, J. Virol. 65, 1168-1176, 1991; Griffith, Griffith, Rayment, Murakami & Casper, Nature 355, 652-654, 1992). Insbesondere die Assemblierung *in vitro* ist nach dem Stand der Technik ausführlich dokumentiert (Slilaty, Berns & Aposhian, J. Biol. Chem. 257, 6571-6575, 1982; Salunke, Caspar & Garcea, Cell 604, 895-904, 1986; Garcea, Salunke & Caspar, Nature 329, 86-87, 1987, Salunke, Caspar & Garcea, Biophys. J. 56, 887-900, 1989). Der potentielle Einsatz dergestalt konstruierter Vehikel, bestehend jeweils aus Untereinheiten des natürlich vorkommenden Proteins, für Gentransfer ist prinzipiell beschrieben (Forstovä, Krauzewicz, Sandig, Elliott, Palkovä, Strauss, & Griffin, Hum. Gene Therapy 6, 297-306, 1995). In der WO 97/43431 ist ein Vehikel zum Transport von molekularer Substanz beschrieben, umfassend mindestens ein von einem Virus abgeleitetes oder stammendes Kapsomer, das an seiner einen Seite eine Modifikation aufweist, so daß die molekulare Substanz an das Kapsomer bind- bzw. anlagerbar ist.

Bislang wurden im Stand der Technik jedoch noch keine Verfahren zur mosaikartigen *in vitro*-Assemblierung von Polyomaviruskapsiden oder anderen virusanalogen Partikeln beschrieben, die aus unterschiedlichen modifizierten Teileinheiten oder aus gleichen mehrfach modifizierten Teileinheiten zusammengesetzt sind, da Modifikationen von Teileinheiten häufig dazu führen, daß die Assemblierungskompetenz der Teileinheiten verlorengeht.

Aufgabe der vorliegenden Erfindung ist es daher, modulare Transportsysteme für molekulare Substanzen bereitzustellen, die aus aus unterschiedlichen modifizierten Teileinheiten oder aus gleichen mehrfach modifizierten Teileinheiten zusammengesetzt sind und die beschriebenen Nachteile des Stand der Technik nicht aufweisen.

Zur Lösung der Aufgabe werden erfindungsgemäß nach Anspruch 1 Transportsysteme für molekulare Substanzen bereitgestellt, enthaltend rekombinant hergestellte Teileinheiten auf Aminosäurebasis, umfassend:
- mindestens zwei voneinander einfach verschieden modifizierte Teileinheiten, und/oder
- eine oder mehrere zumindest zweifach verschieden modifizierte Teileinheiten, und
- wahlweise unmodifizierte Teileinheiten,
wobei die Teileinheiten mosaikartig zu dem Transportsystem zusammensetzbar sind und molekulare Substanzen in das Transportsystem einschließbar sind, und
wobei die Teileinheiten mosaikartig zu dem Transportsystem zusammensetzbar sind und molekulare Substanzen in das Transportsystem einschließbar sind, und
wobei es sich bei den modifizierten Teileinheiten um Polyomavirus VP1-Protein Teileinheiten handelt, die als eine Modifikation eine Einführung von ein oder mehreren Aminosäuren, Peptid- oder Proteinsequenzen oder Proteindomänen in einer Loop-Region an der Außenseite des Proteins zwischen den Aminosäurepositionen 148 und 149 und/ oder 293 und 295 aufweist.

Vorteilhafte Ausführungsformen der Transportsysteme sowie Herstellungsverfahren und Verwendungen der Transportsysteme ergeben sich aus den Unteransprüchen und aus der Beschreibung.

### Beschreibung

Die Verwendung von natürlichen Viren oder virusanalogen Systemen zum Transfer von Nukleinsäuren in Zellen (Gentherapie) ist ein wichtiges Forschungsgebiet im Bereich der molekularen Medizin. Eine besondere Herausforderung liegt dabei in der Herstellung eines Vektorsystems (Transportsystems), das Nachteile nach dem Stand der Technik zu gentherapeutischen Heilverfahren ausschließt oder minimiert.

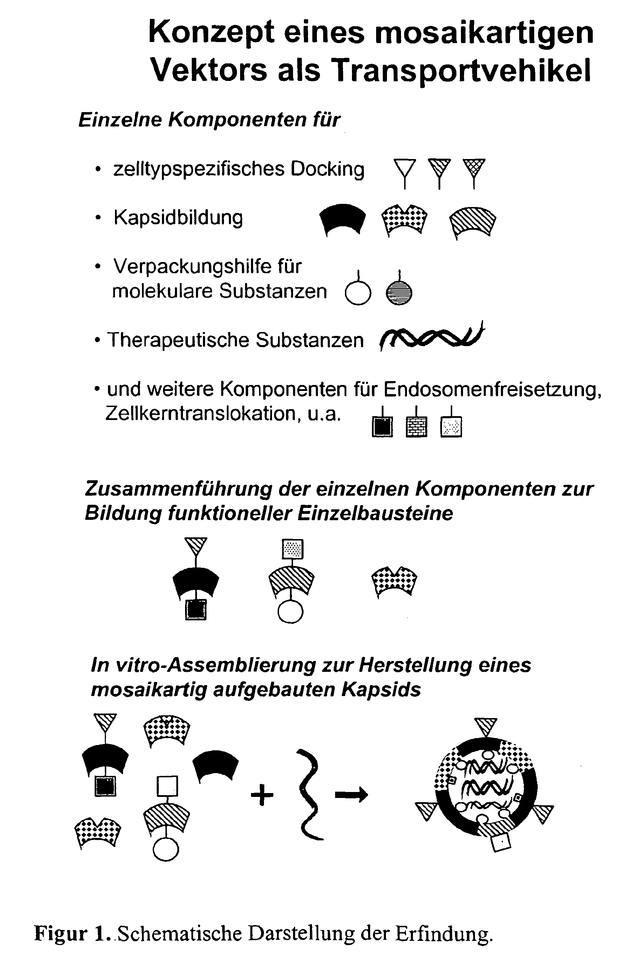

In der vorliegenden Erfindung wird ein Transportsystem für molekulare Substanzen beschrieben, das *in vitro* aus unterschiedlichen Einzelkomponenten zusammengesetzt sein kann wobei jeweils die Merkmale des Anspruchs 1 erfüllt sein müssen. Dies wird erreicht durch die Verwendung molekularer Komponenten bzw. Teileinheiten ("Module"), die erfindungsgemäß aus Proteinen bestehen. Diese Teileinheiten können erfindungsgemäß auf verschiedene Weise modifiziert sein, d.h. die Aminosäuresequenzen der Teileinheiten können verändert, ergänzt oder verkürzt werden, um gewünschte Eigenschaften mit den Modulen in das Transportsystem zu integrieren. Insbesondere können die Module durch Fusionen und Insertionen auch funktionelle Domänen aus anderen Proteinen enthalten. Die einzelnen Funktionsmodule können *in vitro* zusammengesetzt (assembliert) werden, entweder direkt aufgrund ihrer molekularen Eigenschaften, oder durch, beispielsweise für den Fall, daß die Module nicht assemblierungskompetent sind, Kopplung an spezielle Module, welche die erforderliche Assemblierungskompetenz aufweisen. Im Rahmen der Erfindung können virusähnliche Partikel mosaikartig aufgebaut werden, die aufgrund ihrer Zusammensetzung bestimmte Funktionen besitzen Ein besonderer Vorteil besteht dann, daß die molekulare Zusammensetzung der Transportsysteme stöchiometrisch festlegbar ist. Die entstehenden virusähnlichen Partikel können dazu verwendet werden, molekulare Substanzen wie Nukleinsäuren, Peptide, oder Proteine effizient und gezielt in das Innere von eukaryontischen Zellen zu transportieren. Eine Ausführungsform der Erfindung ist schematisch in Fig. 1 dargestellt.

Erfindungsgemäß können die Transportsysteme modifizierte Teileinheiten der in Tabelle 1 aufgeführten Viren und Phagen oder von makromolekularen Proteinassemblaten mit innerem Hohlraum, wie Proteasomen oder Chaperone und wahlweise unmodifizierte Teileinheiten davon enthalten. Erfindungsgemäß können die Transportsysteme Monomere, Dimere oder Oligomere von Teileinheiten enthalten. Bevorzugt sind erfindungsgemäß Transportsysteme, deren Teileinheiten aus dem Polyomavirus VP1-Protein abgeleitet sind bzw. modifizierte Teileinheiten hiervon. Weiterhin bevorzugt sind Transportsysteme, deren Teileinheiten aus Phagenproteinen abgeleitet sind, insbesondere solcher Phagen, die Wirte thermophilen oder hyperthermophilen Ursprungs aufweisen und die damit auch bei hohen Umgebungstemperaturen (≥ 70 °C) noch stabile Strukturen ausbilden. Hierbei ist das SSV1-Partikel (Fuseolloviridae) hervorzuheben, das das Archaebakterium *Sulfolobus shibatae* infiziert. Dieser Vertreter der Phagen ist aufgrund seiner Wirtsspezifität hyperthermophil, mithin auch bei hohen Temperaturen stabil und kann daher für eine Vielzahl von Anwendungen im Bereich der Biotechnologie und auch der Medizin vorteilhaft genutzt werden. Es ist in der Lage, eine sehr stabile Proteinhülle auszubilden, wobei die Bausteine einfach rekombinant herstellbar sind. Ähnliche Vertreter thermophiler bzw. hyperthermophiler Phagen finden sich beispielsweise auch bei den Lipothrixviridae (Vertreter: TTT1, TTV2, TTV3). Noch nicht weiter klassifiziert sind die thermophilen und hyperthermophilen Vertreter der Bacilloviridae (Beispiel: TTV4, SIRV) und der Guttaviridae (Beispiel: SNDV), die ebenfalls in solchen Prozessen, wo unter anderem die Stabilität einer Proteinhülle (gebildet aus den Phagenproteinen) relevant ist, eingesetzt werden können.

Die modifizierten Teileinheiten werden erfindungsgemäß bevorzugt rekombinant hergestellt.

Die Transportsysteme enthalten erfindungsgemäß mindestens zwei voneinander verschieden modifizierte Teileinheiten, wobei unter "verschieden modifiziert" zu verstehen ist, daß die Teileinheiten unterschiedliche Modifikationen aufweisen, oder die Teileinheiten die gleiche Modifikation an verschiedenen Positionen der Teileinheit aufweisen.

Die Transportsysteme können gemäß der vorliegenden Erfindung auch ein oder mehrere zumindest zweifach modifizierte Teileinheiten, bevorzugt zweifach verschieden modifizierte Teileinheiten, enthalten.

Wahlweise können die Transportsysteme erfindungsgemäß zusätzlich unmodifizierte Teileinheiten enthalten.

Erfindungsgemäß können die rekombinant hergestellten Teileinheiten durch Punktmutationen oder durch Einführung, Entfernung oder Austausch von ein oder mehreren Aminosäuren, Peptid- oder Proteinsequenzen oder Proteindomänen an dem Terminus/den Termini und/oder in der Sequenz der Teileinheit modifiziert sein.

Die Modifikationen können beispielsweise Markierungen sein, also beispielsweise Fluoreszenzfarbstoffe, Polyethylenglycol, Oligonukleotide, Nukleinsäuren, Peptide, Peptidhormone, Lipide, Fettsäuren oder Kohlenhydrate.

Die Teileinheiten können auch Modifikationen aufweisen, durch die eine verbesserte Bindungsaffinität der Teileinheiten an molekulare Substanzen bewirkt wird, beispielsweise prolinreiche Sequenzen, WW-Sequenzen, SH3-Domänen, Biotin, Avidin, Streptavidin, oder polyionische Sequenzen. Derartige Modifikationen befinden sich bevorzugt an der Innenseite des Transportsystems.

Weiterhin sind erfindungsgemäß Modifikationen vorgesehen, durch die eine verbesserte Aufnahme in gewünschte Zielzellen erreicht werden kann, beispielsweise Kohlenhydratstrukturen, Proteine oder Proteindomänen, Antikörper oder modifizierte Antikörper, Antigene, oder isolierte Rezeptorbindungsdomänen von Liganden oder andere Substanzen oder Sequenzen, die jeweils eine Bindung an Rezeptoren auf der Oberfläche der Zielzellen vermitteln können.

Weiterhin können die Teileinheiten erfindungsgemäß Modifikationen aufweisen, durch die ein Transport in bestimmte Zellorganellen der Zielzellen (beispielsweise Zellkern, Mitochondrien, Endoplasmatisches Reticulum) oder ein Transport aus den Zielzellen hinaus ermöglicht wird. Diese Modifikation, die eine verbesserte Aufnahme in Zielzellen, Zellorganellen oder einen Transport aus den Zielzellen hinaus bewirken, befinden sich bevorzugt an der Außenseite des Transportsystems oder sind Bestandteil der zu transportierenden molekularen Substanz.

Das Verfahren zur Herstellung der erfindungsgemäßen Transportsysteme umfaßt die folgenden Schritte:
- Rekombinante Expression der Polyomavirus VP1-Protein-Teileinheiten,
- Freisetzung der Teileinheiten durch Lyse der Wirtszellen,
- Inkontaktbringen der Teileinheiten in den gewünschten stöchiometrischen Verhältnissen, um das Transportsystem mosaikartig zusammenzusetzen (zu assemblieren), und
- wahlweise vor oder während der Assemblierung Inkontaktbringen mit molekularen Substanzen, um die molekularen Substanzen in das Transportsystem einzuschließen.

Der in der vorliegenden Erfindung beschriebene Ansatz ist eine vorteilhafte Alternative zu dem derzeit üblichen Verfahren der experimentellen Gentherapie, also der Verwendung von Viren, Liposomen, oder von physikalischen Systemen. Bei Verwendung replikationsdefizienter Viren beispielsweise sind beträchtliche Untersuchungen notwendig, um die biologische und therapeutische Sicherheit dieser Vektoren zu garantieren. Im Gegensatz zu diesen Systemen beschreibt die vorliegende Erfindung ein Verfahren, das einen einfachen, schrittweisen *in vitro*-Aufbau eines virusanalogen Partikels, bestehend aus mosaikartig zusammengesetzten Teilen, zur Grundlage hat und dadurch besonders sicher bezüglich einer medizinischen oder therapeutischen Anwendung ist.

Die Vorteile der in der vorliegenden Erfindung beschriebenen modularen Konstruktion von artifiziellen viralen Vektorsystemen gegenüber traditionellen, vorwiegend retroviralen Systemen, sind nachfolgend zusammengefaßt.
- Oft diskutierte Sicherheits-Probleme der Vektoren, wie sie bei der Konstruktion von künstlich replikationsinkompetenten Viren (Retroviren, Adenoviren) auftreten können, werden vermieden, da hier nicht komplexe, potentiell pathogene Viren um einige Eigenschaften reduziert werden, sondern lediglich artifizielle Assoziate, beispielsweise aus Proteinen aufgebaut, um erforderliche Eigenschaften erweitert werden. Die vollständige Synthese der Kapside *in vitro* ermöglicht die Umsetzung maximaler Anforderungen an ein sicheres System für gentherapeutische Anwendungen. Die Einzelkomponenten sind vollständig vom therapeutischen Ansatz entkoppelt; die zu transferierende, therapeutisch wirksame Substanz (DNA, RNA, oder analoge Moleküle) enthält keinerlei Informationen zur Herstellung des molekularen Vehikels. Somit ist das Auftreten replikationskompetenter Spezies völlig auszuschließen. Weiterhin enthalten die fertigen Vektoren keinerlei genetische Information über den Bauplan des Partikels, nachteilige potentielle Gefährdungen durch Rekombinationsereignisse sind somit vollständig ausgeschlossen.
- Eine hohe Reinheit und Homogenität der Systeme wird durch die *in vitro*-Assemblierung von Komponenten gewährleistet, die separat in hoher Qualität nach dem Stand der Technik herstellbar sind. Über hochspezifische Affinitäts-Reinigungsschritte (derzeit beispielsweise durch ein selbstspleißendes Protein an einer Affinitätsmatrix realisiert) der isolierten Bestandteile können alle unerwünschten, problematischen Komponenten (kontaminierende DNA, bakterielle Proteine und Endotoxine) effizient entfernt werden.
- Die synthetisch (rekombinant) hergestellten Viruskapside können mit Hilfe eines singulären Cysteinrestes in jeder Untereinheit einer besonderen Variante jeweils fluoreszenzmarkiert oder mit einer für PET (Positron-Emissions-Tomograpie) und anderen Lokalisationsverfahren geeigneten molekularen Markierung versehen werden. Diese Markierungen erlauben den Nachweis der Vektoren sowohl innerhalb von (nichtfixierten, d. h. lebenden) Zellen mittels konfokaler Fluoreszenzmikroskopie, wie auch - zeitaufgelöst - innerhalb von kompletten, lebenden Organismen.
- Die Fluoreszenzmarkierung aller Komponenten des Systems erlaubt die Qualitätskontrolle bezüglich der Zusammensetzung der Präparationen durch FACS-Analysen der Kapside, bei der die Zusammensetzung durch statistisches Auszählen von Einzelpartikeln präzise nachgewiesen werden kann.
- Sowohl die *in vitro-* als auch die *in vivo*-Applikation der Vektoren ist grundsätzlich möglich.

Vorteile von erfindungsgemäß modular aufgebauten, artifiziellen virusanalogen Vektorsystemen gegenüber Systemen, die aus einer einheitlichen Komponente aufgebaut sind (beispielsweise in der Literatur beschriebene virusähnliche Partikel) sind nachstehend zusammengefaßt.
- Eine (Entkopplung) der minimal erforderlichen Einzelschritte gentherapeutischer Verfahren wird ermöglicht: (i) die gezielte Verpackung eines krankheitsspezifischen Therapeutikums, (ii) das zellspezifische Targeting (Zelltropismus), (iii) Aufnahme und Endosomenfreisetzung, (iv) kompartiment-spezifische Translokation innerhalb der Zelle, und (v) selektive Wirkungsinitiierung der therapeutischen Substanz.
- Der modulare Aufbau des Vehikels erlaubt die selektive Integration aller notwendigen Funktionen in das synthetische Partikel, wobei jeweils nur die Funktionen berücksichtigt werden müssen, die für die Art der Anwendung erforderlich sind. Beispielsweise erfordert der Transport eines krankheitsspezifischen Therapeutikums (beispielsweise DNA, die die Expression des therapeutischen Gens mittels eines gewebespezifischen Promoters erlaubt) nicht unbedingt eine Domäne für zelltypspezifisches Targeting.
- Die genau dosierbare Zusammensetzung des Partikels ermöglicht eine Einbindung der verschiedenen erforderlichen Funktionen in der exakt dafür notwendigen Dosis. Dadurch wird eine Verminderung bzw. Vermeidung von unerwünschten Nebenwirkungen bei hoher Therapeutikadosierung erreicht.
- Unterschiedliche therapeutische Zielrichtungen erfordern nicht die Erarbeitung völlig neuartiger Systeme und Herstellungsverfahren, sondern jeweils nur das Einbringen einzelner neuer Bausteine oder eine Modifikation bestehender Komponenten des Gesamtsystems. Beispielsweise kann im Rahmen einer Tumortherapie ein Anti-Tumor-Therapeutikum in Tumorgewebe transportiert werden, wobei durch eine entsprechende Rezeptor-Bindungsdomäne spezifisch Tumorzellen eines bestimmten Typs adressiert werden. Eine Variation der Rezeptor-Bindungsdomäne ermöglicht den Transport desselben Therapeutikums in Tumorzellen eines anderen Typs. Außerdem können beispielsweise durch Variation bestimmter Domänen zur spezifischen Verpackung eines Therapeutikums (beispielsweise DNA, RNA, Peptide oder Proteine) unterschiedliche therapeutisch wirksame Substanzen in einem ansonsten unveränderten System appliziert werden.
- Potentielle Schwachstellen des therapeutischen Ansatzes können durch vergleichendes Austesten von verschiedenen funktionellen Modulen leicht identifiziert und anschließend beseitigt werden, und es kann ein besseres Verständnis für die grundlegenden molekularbiologischen Vorgänge bei natürlichen Viren erworben werden.

Die einzelnen Bausteine (Teileinheiten) der Transportsysteme können so ausgestaltet sein, daß sie individuelle funktionelle Eigenschaften besitzen. Die mosaikartige Zusammensetzung (Assemblierung) wird *in vitro* durchgeführt und kann durch stöchiometrische Zugaben der Bausteine und geeignete Assemblierungsbedingungen festgelegt werden. Die Bausteine des Transportsystems werden üblicherweise rekombinant hergestellt. Die jeweils entstehenden virusähnlichen Hüllstrukturen (Kapside) können so die für den jeweiligen Anwendungsfall gewünschten Eigenschaften und Funktionen aufweisen. Neue Funktionen und Anwendungsgebiete lassen sich durch das Hinzufügen weiterer Module bereitstellen und ergänzen, wobei die einzelnen Module bezüglich ihrer funktionellen und molekularen Eigenschaften unabhängig voneinander herstellbar sind. Solchermaßen hergestellte Transportsysteme für molekulare Substanzen eignen sich insbesondere für Anwendungen im Bereich der Gentherapie, auch zum gezielten Einbringen von Wirkstoffen wie beispielsweise DNA oder Proteine in eukaryontische Zellen.

Gemäß der vorliegenden Erfindung wird das Polyomavirus VP1-Protein in seinen natürlichen Eigenschaften verändert und ein Transportsystem mit Eigenschaften bereitgestellt, die im Stand der Technik nicht beschrieben sind. Das VP1-Protein kann beispielsweise so verändert werden, daß unerwünschte natürliche Eigenschaften wie die Bindung an einen spezifischen Rezeptor auf der Oberfläche von Zellen, beispielsweise Nierenepithelzellen der Maus, entfernt werden, ohne daß die Assemblierung davon betroffen ist. Andererseits kann durch die Einführung spezifischer neuer Sequenzen der Aufnahmemechanismus in eukaryontische Zellen moduliert werden, bestimmte Sequenzmotive stimulieren dabei die Aufnahme in Zellen.

Die dreidimensionale Struktur des Proteins ist bekannt (Stehle, Yan, Benjamin & Harrison, Nature 369, 160-163, 1994). Im Rahmen der Erfindung konnte gezeigt werden, daß in mindestens zwei Loopsegmente an der Außenseite des Proteins (Aminosäurepositionen 148 und 293) ein funktionelles Modul in Form einer Domäne, z. B. für das rezeptor-spezifische Docking (zelltypspezifische Targeting), inseriert werden kann (vgl. Beispiel 4).

Weiterhin konnte gezeigt werden, daß durch Veränderung der Cystein-Zusammensetzung der Untereinheiten sowie der assemblierten Kapside eine Modulation der Disulfidbrückenmuster erfolgen kann. Auf diese Weise kann die biologische Stabilität der Partikel variiert werden.

Erfindungsgemäß werden Varianten des VP1-Proteins mit besonderen, neuen Eigenschaften hergestellt, die das natürlich vorkommende Wildtyp-Protein nicht besitzt. Als besonders vorteilhaft hat sich dabei erwiesen, daß eine Herstellung und Reinigung der modifizierten VP1-Proteine über ein im Beispiel 1 beschriebenes Verfahren erfolgen kann. Weiterhin können durch gentechnische Methoden Veränderungen vorgenommen werden (Punktmutationen, siehe Beispiel 2, 3, und 5) und weiterhin zusätzliche (funktionelle) Domänen, Peptide, oder Proteine gentechnisch an den Termini des VP1-Proteins angebracht oder in die Sequenz des VP1-Proteins implantiert werden (vgl. Beispiel 4). Diese Funktionseinheiten können die Eigenschaften des Hüllproteins beispielsweise um Funktionen zum spezifischen Rezeptor-Docking, zur effizienten Aufnahme in die Zielzellen, oder zur Bindung und Verpackung des zu transportierenden Moleküls erweitern. Insbesondere lassen sich die einzelnen Funktionseinheiten innerhalb einer Virushülle kombinieren, indem die verschiedenen Hüllproteine mosaikartig miteinander assembliert werden, so daß multifunktionelle virusähnliche Partikel gebildet werden. Dabei kann entsprechend der Art der Anwendung die optimale Menge jeder Funktionseinheit innerhalb eines einzelnen Viruskapsids eingestellt werden. Ein solchermaßen konstruiertes, artifizielles virusanaloges Partikel kann vielfach verwendbar sein, insbesondere aber für den gezielten Transfer von therapeutisch wirksamen molekularen Substanzen in Zielzellen eingesetzt werden.

Die vorliegende Erfindung beschreibt mosaikartig, modular aufgebaute Transportsysteme für therapeutische Substanzen, wobei eine einfache und schnelle Anpassung des Systems an die jeweilige Anwendung ermöglicht wird. Ein Anwendungsgebiet der Erfindung kann die Therapie von Infektionskrankheiten wie beispielsweise AIDS sein. Dabei erfolgt eine Vermehrung des Virus HIV in CD4⁺-Lymphzyten, was zu den beschriebenen Krankheitssymptomen führt. Die infizierten Zellen präsentieren auf ihrer Oberfläche in der späten Infektionsphase das virale Protein gp120, das an den natürlichen Rezeptor CD4 bindet und die Aufnahme des Virus in die Zelle vermittelt. Dieser Mechnismus kann zur zellspezifischen Adressierung ausgenutzt werden, indem die Oberfläche des in der vorliegenden Erfindung beschriebenen Transportsystems modifiziert wird, entweder mit dem Rezeptor CD4 oder mit einzelnen CD4-Domänen, die zur Bindung an gp120 notwendig sind. Da die Wechselwirkung von CD4 mit gp120 hochspezifisch ist und an keiner anderen Stelle im Körper auftritt, interagiert ein solches Transportvehikel nur mit Lymphozyten, die bereits erfolgreich von HIV infiziert wurden, das heißt, die therapeutische Substanz wird wie gewünscht ausschließlich in infizierte Zellen transportiert.

Als therapeutische Substanz kann DNA verwendet werden, die intrazellulär wirkende Antikörper codiert, die wiederum spezifisch an HIV-Proteine binden und diese somit in ihrer Funktion neutralisieren. Die therapeutische DNA kann einzel- oder doppelsträngig in die Zelle eingeführt werden. Im Falle doppelsträngiger DNA kann der Einschluß in die Partikel erfolgen, indem einzelne, modifizierte Module eingesetzt werden, die mit dsDNA wechselwirken. Ein solches Modul kann basische Sequenzen auf der Innenseite der Partikel tragen, die mit DNA wechselwirken. Weiter wäre eine Kopplung DNA-interkalierender Substanzen zur Bindung doppelsträngiger DNA möglich. Einzelsträngige DNA wiederum kann auch durch Verwendung von Modulen mit ssDNA-Bindungsproteinen in die Partikel dirigiert werden Ebenfalls wäre eine Kopplung sequenzspezifischer Oligonukleotide an die Partikelinnenseite denkbar, die durch Hybridisierung mit der therapeutischen ssDNA eine Verpackung vermitteln.

Ein weiterer Ansatzpunkt für die HIV-Therapie wäre die Verpackung von Ribozymen, die eine spezifische Erkennungssequenz für HIV-RNA besitzen. Die virale RNA wird durch die Bindung der Ribozyme katalytisch gespalten und inaktiviert. Die Verpackung von Ribozymen kann in diesem Fall durch Module, die RNA-Bindungsdomänen besitzen oder analog zum Einschluß von ssDNA mit Oligonukleotid-modifizierten Vehikelbausteinen, realisiert werden Die Therapie kann alternativ zu Nukleinsäuren auch durch eingebrachte Proteine oder Peptide erfolgen. Eingebrachte trans-dominante (modifizierte) Proteine können mit nativen HIV-Proteinen in der Zelle konkurrieren und somit ihre Funktion inhibieren. Ebenfalls können Peptide oder synthetisch modifizierte Peptide die Wirkung bestimmter HIV Proteine, beispielsweise der HIV-Protease, inhibieren. Weiterhin ist es möglich, die Proteine durch entsprechende Signalsequenzen innerhalb der Zelle zu dirigieren, beispielsweise in den Nukleus mit Hilfe einer Kemtranslokationssequenz des großen T-Antigens des Virus SV40. Dies ist für eine Interaktion mit nuklear lokalisierten Faktoren erforderlich, wie beispielsweise dem HIV-Tat-Protein, das im Zellkern unter anderem als Transkriptionsfaktor dient und die Transkription viraler Proteine drastisch erhöht. Proteine lassen sich in die Transportvehikel einschließen, durch Bindung an Module, die sequenzspezifische Bindungsdomänen in der Art enthalten, daß die gebundenen Proteine in das Vehikelinnere gebracht werden. Außerdem können die genannten Proteine oder Peptide direkt an Vehikelbausteine fusioniert werden, in der Weise, daß sich dazwischen eine Erkennungssequenz für HIV-Protease oder eine zelluläre Protease befindet, die das Protein oder Peptid intrazellulär und wiederum spezifisch in infizierten Zellen freisetzt.

Eine weitere Anwendung der vorliegenden Erfindung ist die Applikation von Anti-Tumor-Wirkstoffen bei malignen Erkrankungen. Dafür müssen die Vehikel Bausteine enthalten, die den Transport des Wirkstoffes in Tumorgewebe gewährleisten. Je nach Art des Tumors geschieht dies beispielsweise durch auf der Partikeloberfläche lokalisierte Antikörper, die an Tumorantigene binden, die ausschließlich oder weitestgehend nur auf Tumorzellen vorhanden sind. Solide Tumore benötigen eine ausreichende Blutversorgung und sekretieren deshalb Wachstumsfaktoren, die die Bildung neuer Blutgefäße im Tumorgewebe initiieren. Die Epithelzellen neu gebildeter Blutgefäße exprimieren verstärkt plasmamembrangebundene Integrinrezeptoren. Diese Rezeptoren erkennen spezifisch die Sequenzabfolge RGD (Arginin-Glycin-Aspartat) und bewirken eine rezeptorvermittelte Endocytose RGD-haltiger Liganden. Diese Eigenschaft kann ebenfalls zur Adressierung von Tumorzellen und damit verbundenem epithelialen Gewebe genutzt werden, indem RGD-exponierende Module in das Transportvehikel integriert werden, so daß eine Aufnahme der therapeutischen Substanz in das Tumorgewebe erfolgt. Eine Kombination verschiedener Rezeptorbindungseigenschaften bewirkt neben einer verbesserten Gewebespezifität eine Therapie, die gleichzeitig an mehreren Stellen den Tumors angreift und die Bildung wirkstoffresistenter Zellen verringert.

Als Wirkstoffe können Nukleinsäuren, wie einzel- und doppelsträngige DNA oder RNA eingesetzt werden. Die darauf codierten Proteine können beispielsweise in der Zelle Apoptose auslösen, indem sie an den entsprechenden Stellen in den zellulären Signaltransduktionskaskaden eingreifen. Zu einer erweiterten Tumorspezifität und somit höheren Sicherheit lassen sich zur Transkription Promotoren verwenden, die bevorzugt in Tumorzellen aktiv sind. In gleicher Weise können Peptide eingesetzt werden, die eine Inhibition von Matrix-Metalloproteinasen bewirken. Insbesondere die Inhibition von MMP-2 und MMP-9 durch spezifische, kurze Peptidsequenzen kann hier effektive Wirkung zeigen.

Außer den genannten Nukleinsäuren können auch Proteine oder Peptide verpackt werden, die Apoptose oder Nekrose auslösen. Geeignet sind beispielsweise katalytische Domänen bakterieller Toxine (beispielsweise Diphtheria-Toxin, Cholera-Toxin, Botulinus-Toxin, und andere), die mit hoher Effizienz die Proteinbiosynthese der Zelle hemmen und dadurch Nekrose auslösen. Vorteilhaft kann sich dabei auswirken, daß nur wenige Moleküle nötig sind, um eine Zelle abzutöten. Ein weiterer therapeutischer Ansatz stellt der Transport der Thymidinkinase von Herpes-Simplex-Virus in Tumorzellen dar. Dieses Enzym phosphoryliert Nukleotidbausteine und weist dabei eine verringerte Substratspezifität gegenüber den zellulären Kinasen auf, so daß auch künstliche Nukleotide, wie beispielsweise Ganciclovir, phosphoryliert werden. Phosphoryliertes Ganciclovir wird bei der DNA-Replikation in neusynthetisierte DNA-Stränge mit eingebaut und führt zum Abbruch der Replikation, was wiederum die Zellteilung verhindert.

Grundsätzlich läßt sich die hier beschriebene Erfindung auch zur Korrektur angeborener genetischer Defekte anwenden, wie beispielsweise ADA-Defizienz, Hämophilie, Duchenne-Muskeldystrophie, und Cystische Fibrose. Diese Erkrankungen sind monokausal, das heißt, sie lassen sich auf einen Defekt eines einzelnen Gens zurückführen. Das Einbringen dieses Gens in korrekter Form ist damit in der Regel ausreichend, um die Krankheitssymptome aufzuheben oder zu vermindern. Für diese Anwendungen muß eine stabile Genexpression erreicht werden, entweder durch stabile episomale Vektoren oder eine Integration der therapeutischen DNA in zelluläre Chromosomen. Dafür können die übertragenen Nukleinsäuren Sequenzen enthalten, die eine Intgration erleichtern. Beispielsweise kann einzelsträngige DNA verwendet werden, die an ihren Enden ITR-Sequenzen (Inverted Terminal Repeats) des Adenoassoziierten Virus trägt, die zur chromosomalen Integration beitragen. Außerdem können neben der therapeutischen DNA oder RNA Proteine mit in die Zelle transportiert werden, die eine Integration aktiv katalysieren, wie beispielsweise HIV-Integrase, oder Rep78 und Rep68 des Adenoassoziierten Virus.

Die Expression korrigierender Gene kann idealerweise unter Kontrolle der natürlichen Promotoren erfolgen, wodurch gleichzeitig eine angepaßte Regulation gewährleistet wird. Ein zelltypspezifisches Targeting des Transportvehikels ist daher in vielen Fällen nicht notwendig. Beispielsweise können bei Hämophilie-Patienten die fehlenden Faktoren der Blutgerinnungskaskade in Muskelgewebe produziert werden, wobei die Faktoren mit einer geeigneten Signalsequenz fusioniert werden, so daß sie aus der Zelle sekretiert werden und an ihren Wirkungsort, die Blutbahn, gelangen.

In allen Fällen einer praktischen Anwendung ist eine effiziente Freisetzung der therapeutischen Substanzen innerhalb der Zelle notwendig, das heißt, die Substanz muß die endosomale Membran erfolgreich passieren. Diese Funktion läßt sich durch Hämolysine, insbesondere thiolaktivierte Cytolysine, Translokationsdomänen bakterieller Toxine, oder bestimmte virale Proteine, wie beispielsweise das Adenovirus-Pentonprotein, realisieren. Diese Funktionen können als Teil des in der vorliegenden Erfindung beschriebenen Vektorsystems im mosaikartig zusammengesetzten Transportvehikel enthalten sein. Des weiteren kann diese Funktion auch von chemischen Substanzen, wie beispielsweise Polykationen oder Dendrimeren, übernommen werden. Die entsprechende Komponente muß entweder auf die Oberfläche der Partikel gebracht oder mit in die Partikel eingeschlossen werden.

Ebenfalls kann es für viele Anwendungen notwendig sein, die Immunogenität des Transportsystems sowie des Therapeutikums so gering wie möglich zu halten. Die humorale Immunogenität der Transportvehikel selbst und die Erkennung und Eliminierung durch Makrophagen kann erfindungsgemäß durch eine Maskierung mit Polyethylenglycol oder eine Umhüllung mit einer Lipiddoppelschicht erreicht werden. Polyethylenglycol kann chemisch so modifiziert werden, daß es kovalent an spezifische SH-Gruppen auf der Partikeloberfläche gebunden wird. Die Immunogenität des therapeutischen Wirkstoffes, das heißt, der direkt eingeführten Proteine oder der von therapeutischen Nukleinsäuren transkribierten und/oder translatierten Proteine, kann mit einer Fusion von 35 bis 40 GA- (Glycin-Alanin)-repetitiven Sequenzen reduziert werden. GA-reiche Sequenzen kommen natürlicherweise bei dem EBNA1-Protein des humanen Eppstein-Barr-Virus vor und schützen das virale Protein vor einem Abbau durch das zelluläre Proteasom und einer Präsentation auf MHC-Klasse-I-Rezeptoren. Diese Schutzfunktion kann für die verschiedenen verwendeten Proteine und Peptide wiederum als Teil des mosaikartigen Vektorsystems ausgeführt sein, wobei hier die *in vitro*-Assemblierung eine positive Rolle spielt.

### Beispiele

Die folgenden Beispiele zeigen Anwendungen der Erfindung, sollen den Schutzbereich der Erfindung jedoch nicht beschränken. 19a. In den Beispielen der Beschreibung wird auf die folgenden Figuren Bezug genommen.

Figur 1 ist eine schematische Darstellung der Erfindung mit möglichen Ausgestaltungsformen. Es wird mosaik-artig ein Kapsid, bestehend aus identischen zumindest zweifach modifizierten Untereinheiten oder verschiedenen Teileinheiten (Komponenten) aufgebaut. Das assemblierte Kapsid kann bestimmte, vorher ausgewählte Eigenschaften aufweisen, die es beispielsweise für Gentransfer geeignet erscheinen lassen.

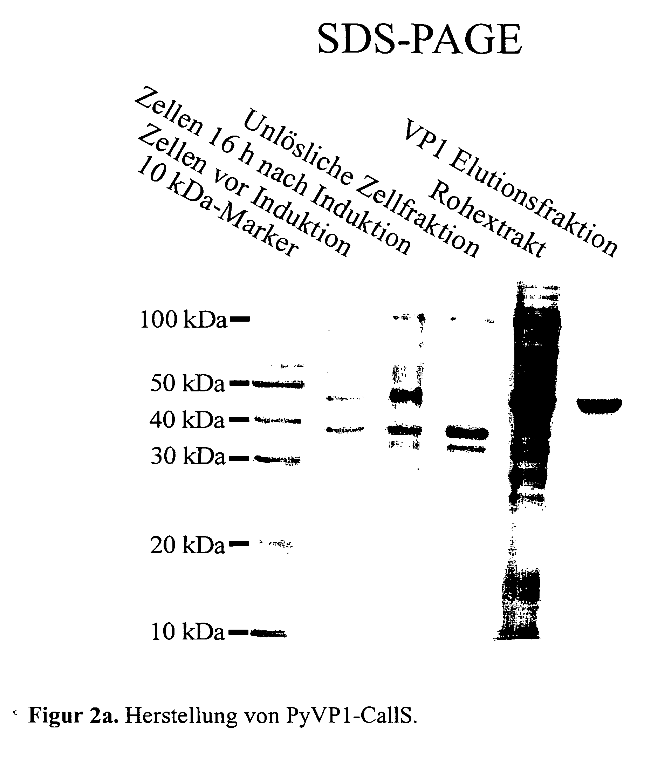

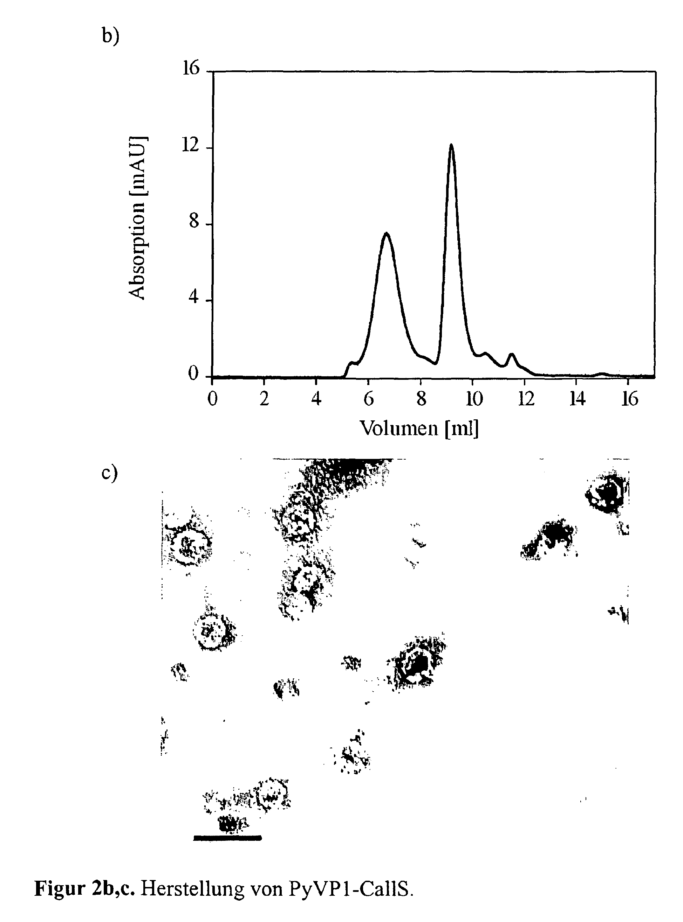

Figur 2 zeigt die Herstellung von PyVP1-CallS. Fig. 2a SDS-PAGE-Analyse der Expression und Reinigung der Variante PyVP1-CallS, nach den im Beispiel 1 angegebenen Bedingungen. (Fig. 2b) Gelfiltration zum Nachweis der Assemblierungskompetenz der PyVP1-CallS-Variante. Kapside eluieren zwischen 6 und 8 ml, freie Kapsomere zwischen 9 und 10 ml. (Fig. 2c) Elektronenmikroskopische Aufnahme von Kapsiden, die ausschließlich aus Untereinheiten der Variante PyVP1-CallS bestehen. Skalierungsbalken: 100 nm.

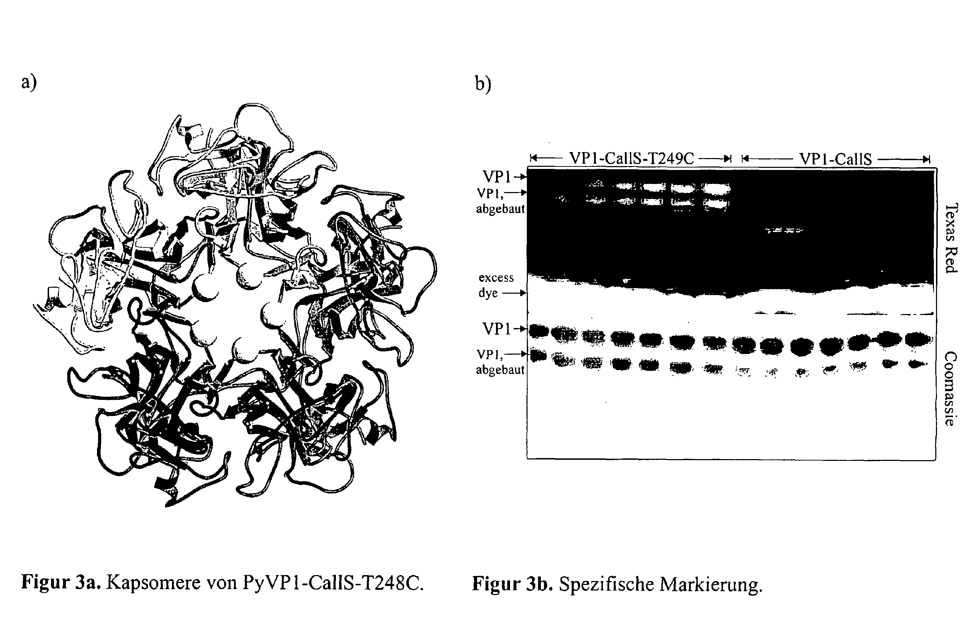

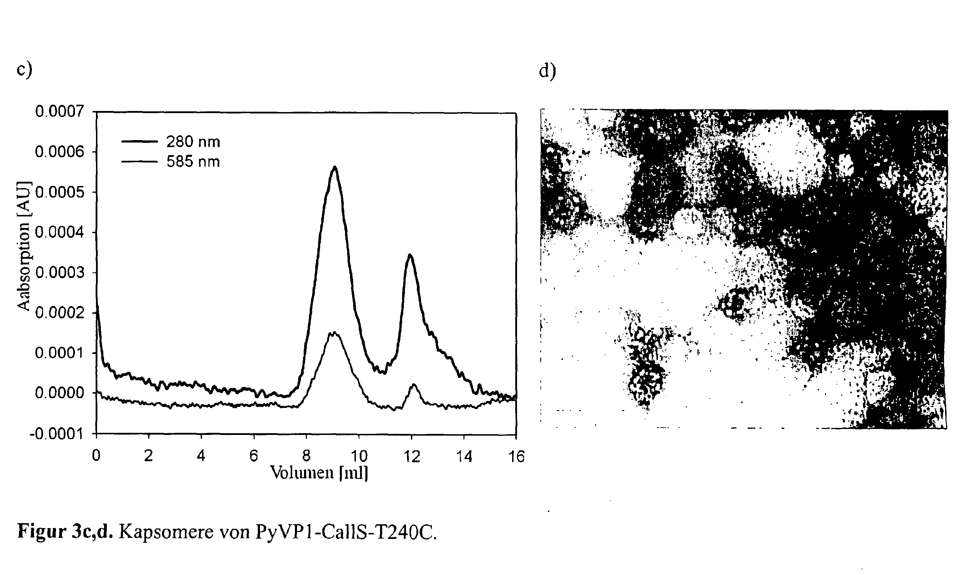

Figur 3 zeigt Kapsomere von PyVP1-CallS-T249C. (a) Aufsicht auf eine dreidimensionale Strukturdarstellung von pentameren Kapsomeren von PyVP1-CallS-T249C (Teilansicht). Die Aminosaureposition 249 in jeder Untereinheit ist durch eine Kugel markiert; an dieser geschützten Stelle ist eine spezifische, neutrale Markierung der Kapsomere möglich. (b) Spezifische Markierung des PyVP1-CallS-T249C-Proteins (linke Hälfte des SDS-Gels) an dem singulären Cystein gegenüber der Kontrolle PyVP1-CallS (rechte Hälfte des SDS-Gels). Die Färbung durch Coomassie-Farbstoff (unterer Teil der Fig. 3b) zeigt das Vorhandensein der Proteine an, aber nur die Variante mit Cystein an Position 249 kann durch einen Farbstoff wie Texas Red markiert werden (oberer Teil der Fig. 3b). (Fig. 3c) Gelfiltration zum Nachweis Die Beispiele 1 und 4 sind erfindungsgemäße Beispiele. Die übrigen Beispiele zeigen die Herstellung von VP1 Varianten mit anderen Modifikationen als die im Anspruch 1 spezifizierten. Sie sind als Vergleichsbeispiele zu betrachten und zeigen Modifikationen an Teileinheiten, die zusätzlich zu den in Anspruch 1 spezifizierten VP1-Modifikationen in die Virushülle aufgenommen werden können. der Assemblierungskompetenz der PyVP1-CallS-T249C-Variante und Einbau des Farbstoffs in Kapside. Die Kapside eluieren zwischen 8 und 10 ml, freie Kapsomere zwischen 11 und 12 ml (d) Elektronenmikroskopische Aufnahme von Kapsiden, die ausschließlich aus fluoreszenzmarkierten Untereinheiten der Variante PyVP1-CallS-T249C bestehen. Skalierungsbalken: 100 nm.

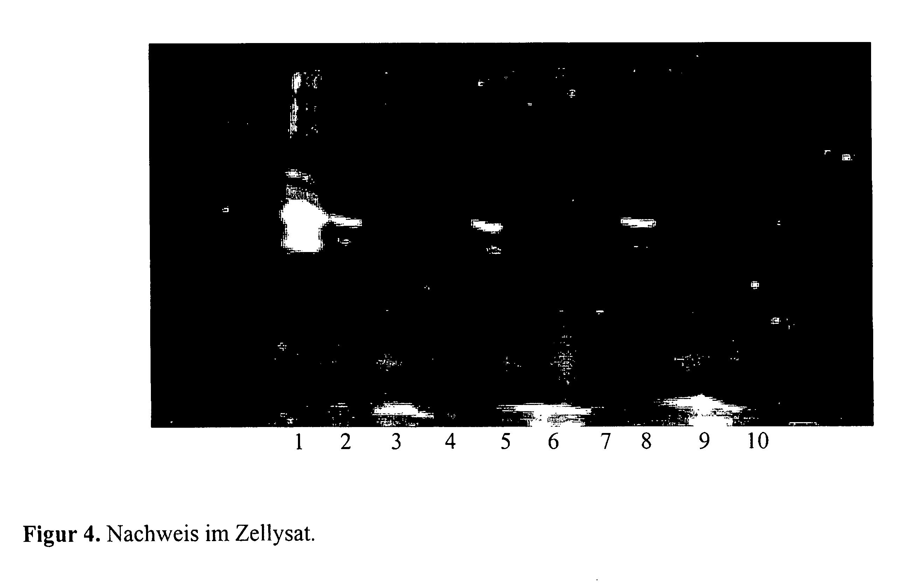

Figur 4 zeigt einen Nachweis im Zellysat. SDS-Gel (ungefärbt) von Zell-Lysat eukaryontischer C2C12-Zellen nach Inkubation für 1 Stunde mit fluoreszenzmarkierten PyVP1-CallS-T249C-Kapsiden. Die in die Zellen aufgenommenen Kapside sind weitgehend proteolytisch abgebaut, der Fluoreszenzfarbstoff jedoch deutlich sichtbar und damit die Aufnahme der Kapside in die Zellen nachweisbar. Bahn 1, VP1-CallS-T249C mit Texas Red markiert; Bahn 2, Medium (Überstand) über den Zellen, Bahn 3: Zellysat mit aufgenommenen Partikeln; Bahn 4: Waschfraktion der Zellen mit PBS (keine Kapside enthalten); Bahnen 5 bis 10: jeweils analog zu Bahnen 2 bis 4 aus gleichartigen Parallelexperimenten.

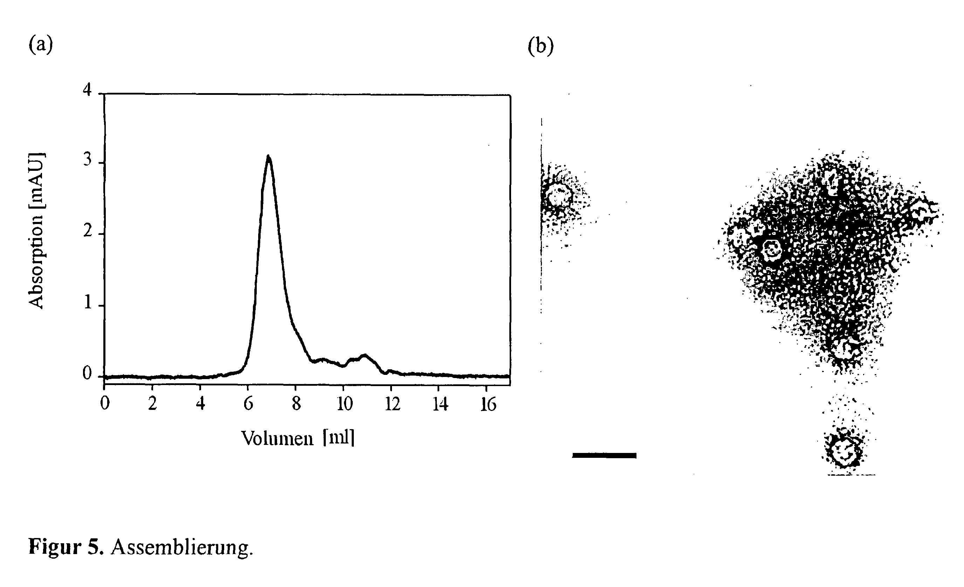

Figur 5 zeigt die Assemblierung. (a) Analyse der Assemblierung der Variante PyVPI-2C durch Gelfiltration. Die Assemblierung der Kapsomere zu Kapsiden (Elution bei 6 bis 8 ml) erfolgt vollständig, im Gegensatz zur Assemblierung der PyVP1-CallS-Variante (Fig. 2b) sind freien Pentamere (9 bis 10 ml) nicht mehr detektierbar. (b) Elektronenmikroskopische Aufnahme der Kapside, die vollständig aus PyVP1-2C gebildet werden.

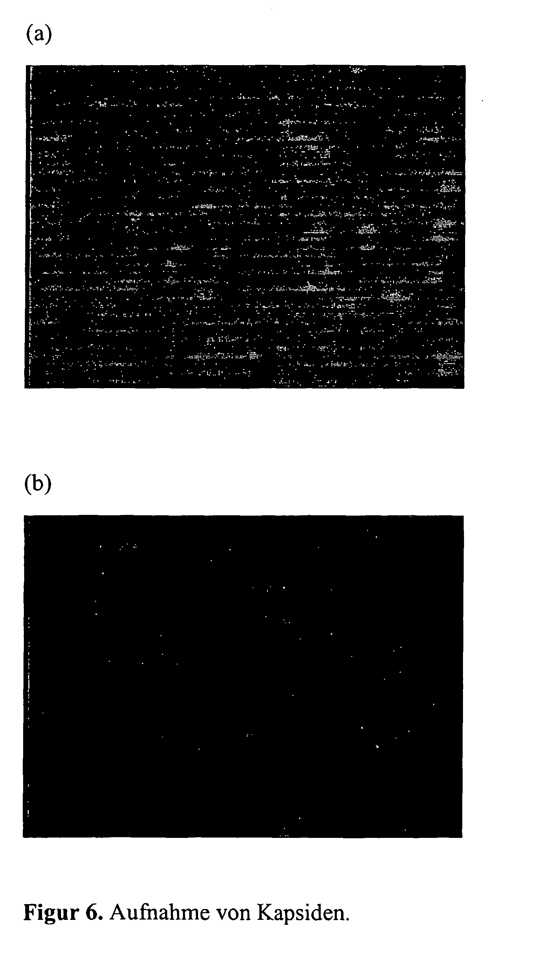

Figur 6 zeigt die Aufnahme von Kapsiden. (a) ohne RGD-Sequenzmotiv, (b) mit RGD-Sequenzmotiv, in eukaryontische Zellen vom Typ Caco-2, unter ansonsten gleichen Bedingungen. (a) Kapside vom Typ PyVP1-CallS-T249C sind mit Texas Red markiert und die Aufnahme der Kapside in die Zellen in einem Fluoreszenzmikroskop visualisiert. (b) Kapsidaufnahme unter identischen Bedingungen wie in (a), jedoch sind die fluoreszenzmarkierten Kapside vom Typ PyVP1-RGD148. Diese Kapside werden aufgrund des RGD-Motivs signifikant effizienter in die Zellen aufgenommen.

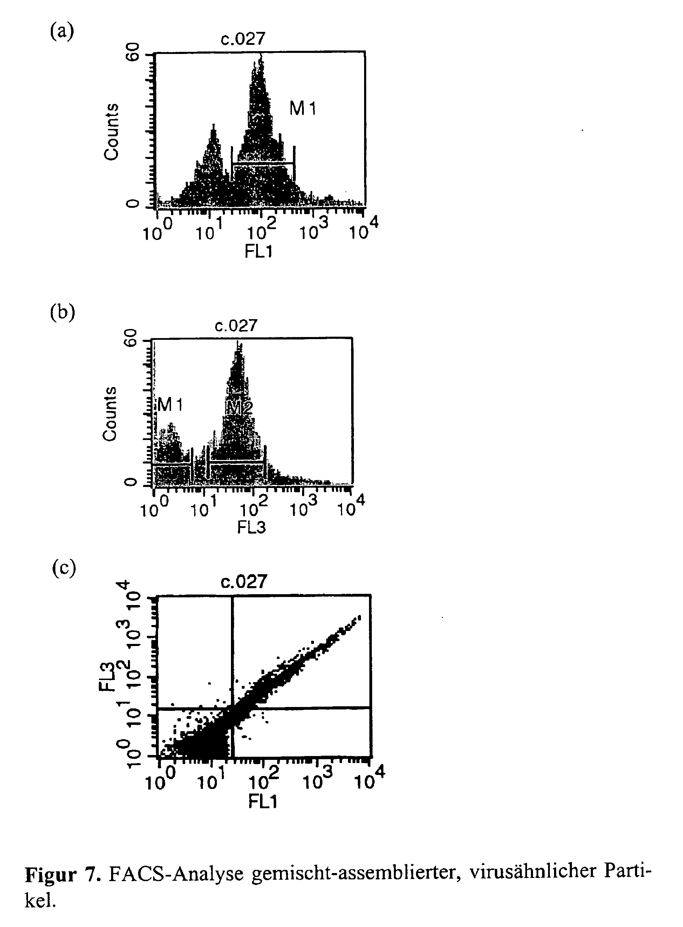

Figur 7 zeigt eine FACS-Analyse unterschiedlich markierter PyVP1-Varianten. Es werden Kapside aus PyVP1-CallS-T249C gebildet, die aus einer mit Fluorescein markierten und einer mit Texas Red markierten Spezies bestehen. Die Kapsidpopulation zeigt eine deutliche Fluorescein-Fluoreszenz (M1 in a), sowie auch eine Texas Red-Fluoreszenz (M2 in b). Aus der Auftragung Fluorescein- (FL1) gegen Texas Red- (FL3) Fluoreszenz wird ersichtlich, daß beide Farbstoffe auf einem Partikel lokalisiert sind (Quadrant oben rechts in c), Partikel, die nur einen Farbstoff enthalten, sind nicht vorhanden und werden daher nicht detektiert.

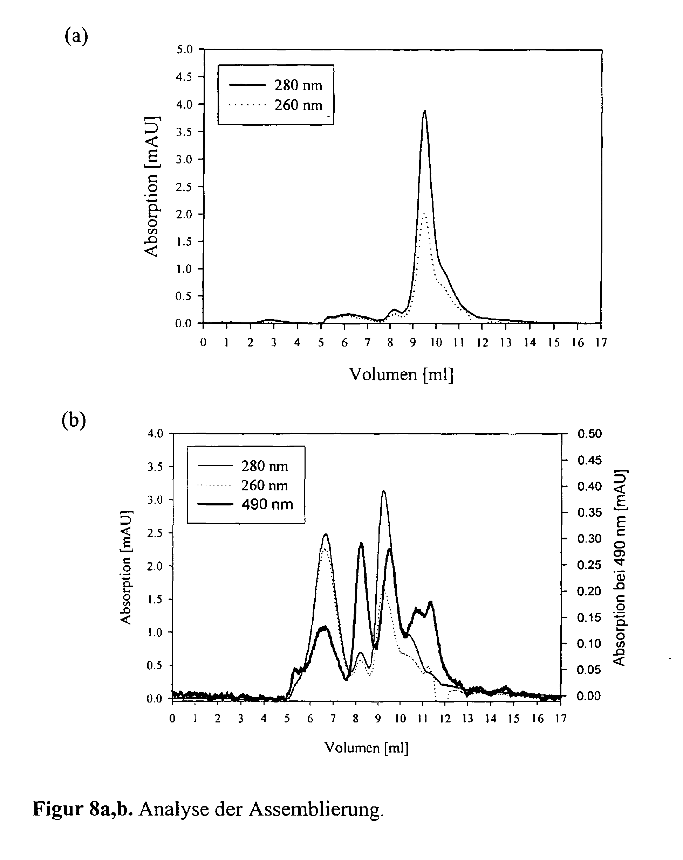

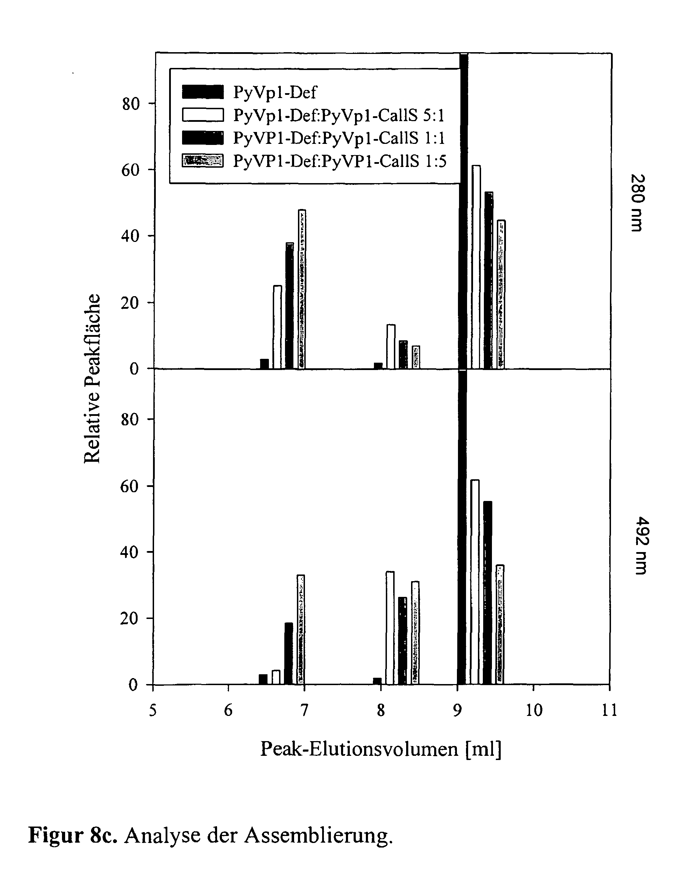

Figur 8 zeigt eine Analyse der Assemblierung. (a) Gelfiltrationsanalyse der assemblierungsdefizienten Komponente PyVP1-Def. Es werden unter Standard-Assemblierungsbedingungen keinerlei Kapside gebildet, sondern nur Kapsomere (Elution bei 9 bis 10 ml) detektiert. (b) Gelfiltrationsanalyse der gemischt-assemblierten Kapside, bestehend aus PyVP1-Def (fluoreszenzmarkiert) und PyVP1-CallS (stöchiometrisches Verhältnis 1:5). (c) Einbauraten von PyVp1-Def in Kapside unter verschiedenen stöchiometrischen Mengenverhältnissen der beteiligten Kapsomere.

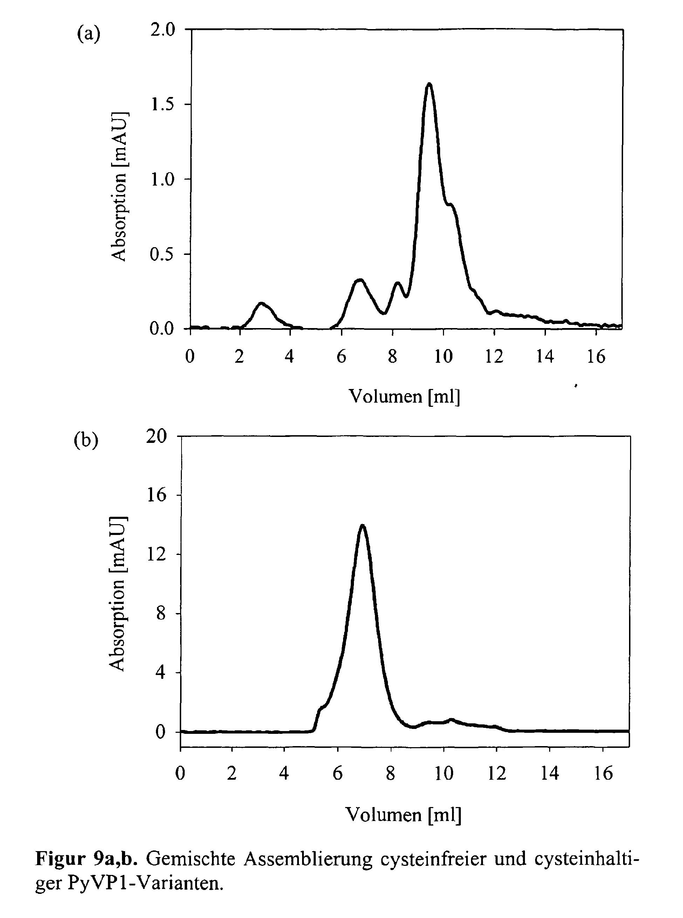

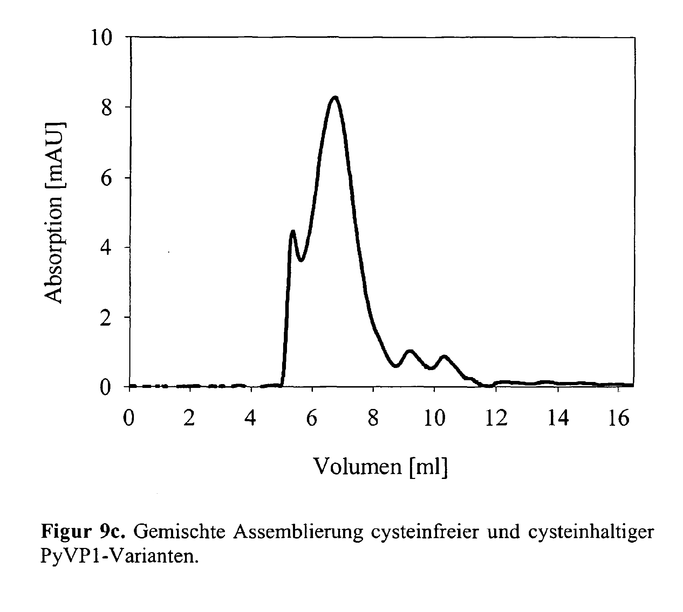

In Figur 9 ist die gemischte Assemblierung von cysteinfreiem PyVP1-CallS-WW150 und cysteinhaltigem PyVP1-wt dargestellt. (a) Die Gelfiltrationsanalyse zeigt, daß die Variante PyVP1-CallS-WW150 nur zu etwa 15 % assembliert werden kann. Kapside eluieren zwischen 6 und 8 ml, freie Kapsomere zwischen 11 und 12 ml. (b) Die Kapsomere der Variante PyVP1-wt bilden quantitativ Kapside. (c) Bei Assemblierung eines äquimolaren Gemisches beider Varianten bilden sich quantitativ gemischte Kapside, freie Kapsomere werden nicht mehr detektiert. Somit wird die Eigenschaft einer quantitativen Assemblierung von PyVP1-wt vollständig auf die gemischt zusammengesetzten (mosaikartigen) Viruskapside übertragen.

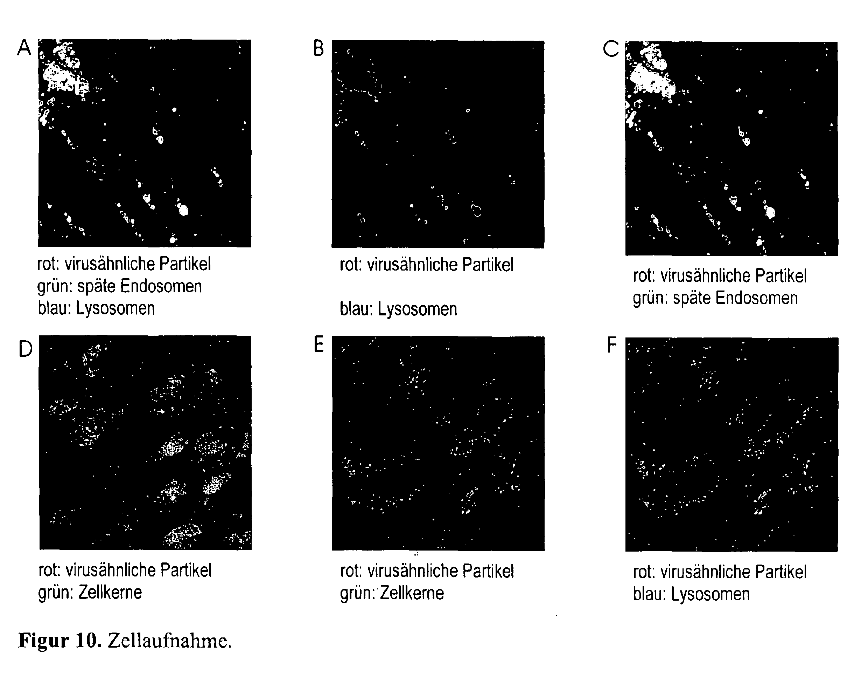

Figur 10 zeigt die Zellaufnahme. Kapside aus assembliertem PyVP1-CallS-T249C werden in C2C12-Zellen aufgenommen und mittels CLSM visualisiert. Zusätzlich zur Anfärbung der Kapside (rot, Farbstoff Texas Red, Fa. Molecular Probes) sind späte Endosomen (grün, Farbstoff Fluorescein-Dextran 70 kDa, Fa. Molecular Probes), Zellkerne (grün, Farbstoff SYTO-16, Fa. Molecular Probes) und Lysosomen (blau, Farbstoff LysoSensor Blue-Yellow, Fa. Molecular Probes) dargestellt. (a) bis (c), Lokalisation der Kapside 15 min nach Aufnahme, (d) bis (f), Lokalisation der Kapside 60 min nach Aufnahme. Die Kapside werden über Endozytose in die Zellen aufgenommen, durchlaufen frühe und späte (nach 15 min) Endosomen, und werden schließlich in Lysosomen (60 min) angereichert.

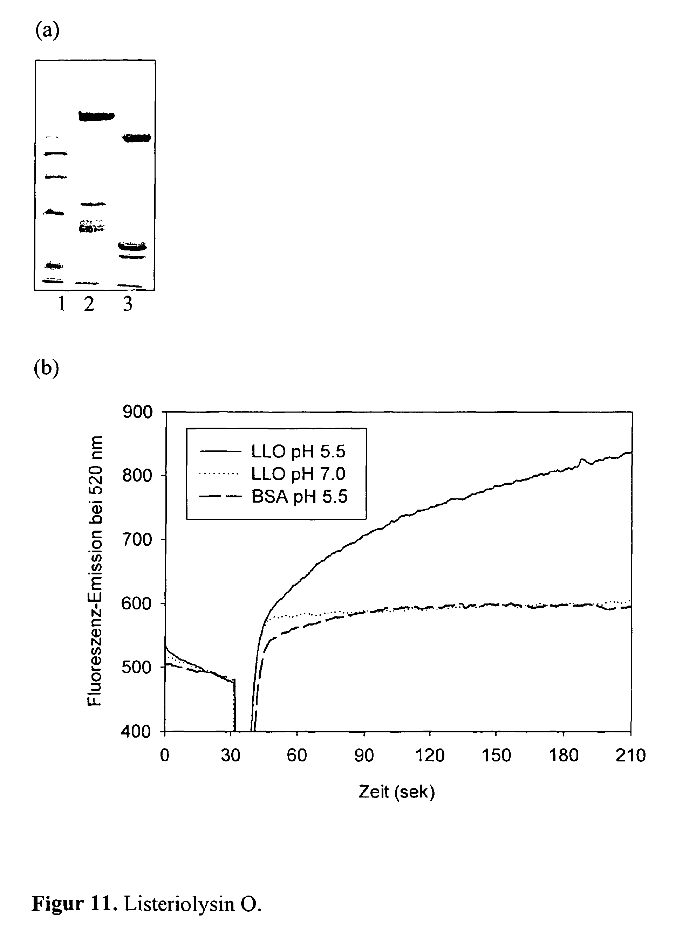

Figur 11 zeigt das Protein Listeriolysin O. (a) Reinigung des Proteins Listeriolysin O (LLO) aus *Listeria monocytogenes*. Bahn 1, Molekularmassenstandard (10 kDa-Leiter); Bahn 2, gereinigtes Fusionsprotein von LLO und Cellulose-Bindungsdomäne gemäß dem Beispiel 8; Bahn 3, Spaltung des Fusionsproteins mit Enterokinase und Freisetzung des LLO. (b) Aktivität des LLO-Proteins, gezeigt durch den zeitlichen Verlauf der Freisetzung eines Fluoreszenzfarbstoffs (Calcein, Fa. Sigma) aus cholesterinhaltigen Liposomen nach Zusetzung von LLO und Absenkung des pH-Wertes unter pH 6.0. In Kontrollexperimenten wurden BSA sowie LLO bei pH 7.0 verwendet, diese induzieren keine Freisetzung des Fluoreszenzfarbstoffes.

### Beispiel 1

### Herstellung, Assemblierung und Charakterisierung von cysteinfreiem Hüllprotein PyVP1 (PyVp1-CallS- Variante)

Das im gegebenen Beispiel verwendete virale Hüllprotein ist von dem in Lösung pentameren Polyomavirus-VP1-Protein abgeleitet, das nach dem Stand der Technik einfach *in vitro* zu einer Hülle assemblierbar ist. Im vorliegenden Beispiel wird eine Polyomavirus-Variante hergestellt, die keinerlei Cysteine in der Sequenz aufweist; die sechs Cysteine des Wildtyp-Proteins (Cys-12, Cys-16, Cys-20, Cys-115, Cys-274, und Cys-283) werden mit Hilfe von Mutagenisierungsverfahren nach dem Stand der Technik durch Serine ersetzt. Das hat unter anderem den Vorteil, daß die Redoxbedingungen der Lösung keinen Einfluß auf den Zustand des Proteins haben; es ist dadurch in vielen Anwendungen einfacher handhabbar.

Die Mutagenese wird mit Hilfe des QuickChange-Verfahrens (Fa. Stratagene), nach Angaben des Herstellers, durchgeführt. Zur Mutagenese werden die folgenden Oligonukleotide verwendet: C12S, C16S, C20S: 5'-GTC TCT AAA AGC GAG ACA AAA AGC ACA AAG GCT AGC CCA AGA CCC-3', und 5'-GGG TCT TGG GCT AGC CTT TGT GCT TTT TGT CTC GCT TTT AGA GAC-3', C115S: 5'-GAG GAC CTC ACG TCT GAC ACC CTA C-3' und 5'-GTA GGG TGT CAG ACG TGA GGT CCT C-3'; C274S, C283S: 5'-GGG CCC CTC AGC AAA GGA GAA GGT CTA TAC CTC TCG AGC GTA GAT ATA ATG-3' und 5'-CAT TAT ATC TAC GCT CGA GAG GTA TAG ACC TTC TCC TTT GCT GAG GGG CCC-3'.

Die Expression und Reinigung von PyVP1-CallS erfolgt als Fusionsprotein mit einer C-terminal fusionierten Inteindomäne und einer sich daran anschließenden Chitin-Bindungsdomäne (CBD). Dazu wird zunächst ein Plasmid hergestellt, das auf dem Vektor pCYB2 des IMPACT-Systems (Fa. New England Biolabs) beruht. Über die multiple Klonierungsstelle des pCYB2 wird mittels der Restriktionsschnittstellen NdeI - Xmal (Restriktionsenzyme von Fa. New England Biolabs) das DNA-Fragment nach Standardverfahren einkloniert, das für das PyVP1-CallS-Protein kodiert.

Für die PCR des DNA-Fragmentes werden die folgenden Oligonucleotide verwendet: vp1NImp (5'-TAT ACA TAT GGC CCC CAA AAG AAA AAG C-3'), und vp1CImp (5'-ATA TCC CGG GAG GAA ATA CAG TCT TTG TTT TTC C-3'). Bei dieser PCR werden gleichzeitig die C-terminalen Aminosäuren des Wildtyp-VP1-Proteins von Gly383-Asn384 in Pro383-Gly384 umgewandelt, da ein C-terminal lokalisiertes Asparagin für das Intein-Spaltsystem sehr ungünstig bezüglich der Spalteigenschaften ist. Die genannten Austausche beeinflussen im Weiteren nicht die wesentlichen Eigenschaften des PyVP1-Proteins zur Assemblierung.

Der *tac*-Promotor des pCYB2-Vektors liefert nur geringe Expressionsmengen des Fusionsproteins, daher wird das Fusionskonstrukt (PyVP1-CallS)-Intein-CBD über eine weitere PCR aus dem pCYB2-Vektor isoliert und in einen hochexprimierenden pET-Vektor mit *T7lac-*Promotor (Plasmid pET21a, Fa. Novagen), über NdeI - EcoRI - Restriktionsschnittstellen, kloniert. Die PCR erfolgt mit den folgenden Oligonucleotiden: vp1-NImp (5'-TAT ACA TAT GGC CCC CAA AAG AAA AAG C-3'), und 5'-ATA TCA ATT CCA GTC ATT GAA GCT GCC ACA AGG-3'.

Der dadurch hergestellte Vektor erlaubt die Expression des Fusionsproteins (PyVP1-CallS)-Intein-CBD mit Hilfe des hochexprimierenden *T7lac*-Promotors in *E. coli* BL21(DE3)-Zellen (Fa. Novagen). Dazu werden transformierte Zellen in 5 1 - Erlenmeyerkolben, die je 2 1 LB-Medium enthalten, bei 37 °C angezüchtet, bis die OD₆₀₀ der Kultur 2.0 bis 2.5 beträgt. Die Induktion der Proteinexpression erfolgt durch 1 mM IPTG im Medium. Die Kulturen werden danach für weitere 20 Stunden bei 15°C inkubiert; die niedrige Temperatur minimiert die Abspaltung des Intein-Anteils im Fusionsprotein unter *in vivo*-Bedingungen. Die Zellen werden durch Zentrifugation geerntet, in 70 ml Resuspensionspuffer (20 mM HEPES, 1 mM EDTA, 100 mM NaCl, 5 % (w/v) Glycerol, pH 8.0) aufgelöst, und durch Hochdruck-Homogenisation aufgeschlossen. Nach Zentrifugation des Rohextraktes für 60 min bei 48 000 g wird ein klarer Zellextrakt erhalten. Dieser Extrakt wird mit einer Flußrate von 0.5 ml/min bei einer Temperatur von 10 °C auf eine 10 ml Chitin-Affinitätsmatrix (New England Biolabs) aufgetragen. Die Säule wird anschließend mit 3 Säulenvolumina des Resuspensionspuffers, 15 Säulenvolumina eines Waschpuffers hoher Ionenstärke (20 mM HEPES, 1 mM EDTA, 2 M NaCl, 5 % (w/v) Glycerol, pH 8.0), und wiederum 3 Säulenvolumina des Resuspensionspuffers gewaschen; dabei werden alle unerwünschten *E. coli-*Wirtsproteine von der Chitin-Matrix entfernt.

Die Abspaltung des an der Chitinmatrix immobilisierten (PyVP1-CallS)-Kapsomers aus dem Fusionsprotein mit Hilfe der selbstspleißenden Inteinaktivität wird durch einen Puls (3 Säulenvolumina) mit jeweils 50 mM Dithiothreitol (DTT), 50 mM Hydroxylamin, oder 30 mM DTT zusammen mit 30 mM Hydroxylamin, im Resuspensionspuffer induziert. Dazu wird die beladene Chitinmatrix mit einer der angegebenen Lösungen für 14 Stunden bei 10 °C inkubiert. Das PyVP1-CallS-Protein wird dabei vollständig freigesetzt und kann mittels säulenchromatographischer Standardverfahren von der Chitinmatrix und den an der Matrix anhaftenden übrigen Bestandteilen des Fusionsproteins abgetrennt werden. Dazu wird geeigneterweise ein linearer Salzgradient mit einer Konzentration zwischen 0.1 und 2.0 M NaCl verwendet. Die Regeneration der Chitinmatrix erfolgt nach den Angaben des Herstellers durch Waschen des Chitin-Materials mit 3 Säulenvolumina eines SDS-haltigen Puffers (1 % SDS (w/v) in Resuspensionspuffer).

Die Assemblierung des PyVP1-CallS-Proteins erfolgt zunächst in Analogie zu bereits vorbeschriebenen Bedingungen nach dem Stand der Technik (vgl. Salunke, Caspar & Garcea, Biophys. J. 56, S. 887-900, 1989). Die virusähnlichen Kapside werden nach Dialyse des Proteins gegen 10 mM HEPES, 50 mM NaCl, 0.5 mM CaCl₂, 5 % Glycerin, pH 7.2, für 72 Stunden bei Raumtemperatur erhalten. Anhand von Gelfiltrationsuntersuchungen (Säule TSKGel G5000PWXL und TSKGel G6000PWXL, Fa. TosoHaas) lassen sich virusähnliche Kapsidhüllen nachweisen und von freien, nicht assemblierten Proteinbausteinen abtrennen.

Das PyVP1-CallS-Protein wird im beschriebenen Verfahren als lösliches Pentamer exprimiert und ist assemblierungskompetent. Fig. 2a zeigt ein SDS-Gel zur Darstellung der Herstellung und Reinigung von PyVP1-CallS. Fig. 2b stellt ein Gelfiltrationsexperiment dar, das zeigt, daß das PyVP1-CallS-Protein unter geeigneten Bedingungen zu kapsidähnlichen Strukturen assembliert werden kann. Fig. 2c beschreibt die entstandenen Kapside mit Hilfe einer elektronenmikroskopischen Aufnahme.

Das Beispiel zeigt, daß das PyVP1-Wildtyp-Protein so modifiziert werden kann, daß eine Assemblierung zu Kapsidstrukturen nach dem Stand der Technik auch dann möglich ist, wenn keinerlei Cysteine in der Proteinhülle vorhanden sind. Gleichzeitig dokumentiert das Beispiel die Möglichkeit der effizienten Herstellung von Kapsomeren mit Hilfe eines inteinbasierten Reinigungssystems.

### Beispiel 2

### Herstellung, Assemblierung und Charakterisierung von fluoreszenzmarkierbarem Hüllprotein PyVP1 (CallS-T249C-Variante)

Zur spezifischen Markierung der Kapsomere kann ein singuläres Cystein in eine spezielle Region im Protein eingeführt werden. Gemäß der in Fig. 3a dargestellten Tertiärstruktur des Proteins ist dies beispielsweise die Position des Threonin 249, das gegen ein Cystein ausgetauscht wird. Die Mutagenese erfolgt mit Hilfe des QuickChange-Verfahrens (Fa. Stratagene) nach Herstellerangaben, unter Verwendung der Oligonukleotide 5'-GGA CGG GTG GGG TGC ACG TGC GTG CAG TG-3' und 5'-CAC TGG AGG CAC GTG CAC CCC ACC CGT CC -3'. Expression und Reinigung erfolgen analog zum Beispiel 1.

Das gereinigte Protein wird mit den Farbstoffen Fluorescein-Maleimid oder Texas Red-Maleimid (Fa. Molecular Probes) nach Herstellerangaben markiert. Dabei erfolgt eine spezifische Kopplung an der Stelle des Cysteins 249; diese Spezifität ist in Fig. 3b gezeigt. Das Protein kann analog zu Beispiel 1 zu Kapsiden assembliert werden, wie aus Gelfiltrationsuntersuchungen (Fig. 3c) und anhand elektronenmikroskopischer Aufnahmen (Fig. 3d) ersichtlich ist.

Die so markierten Kapside werden für 1 Stunde mit eukaryontischen Zellen (C2C12-Zellen) inkubiert. Dabei wird ein 1000facher Überschuß von Viruspartikeln zu Zellen verwendet. Die adhärenten Zellen werden nach der Inkubation mit PBS gewaschen und mit Hilfe eines Zellschabers von der Gefäßwand abgetrennt. Die gelösten Zellen werden anschließend mit SDS-Probenpuffer versetzt und für 5 min auf 99 °C erhitzt. Danach wird das Zellysat über eine SDS-Gelelektrophorese nach Standardvorschrift aufgetrennt. Dabei werden die fluoreszenzmarkierten Proteinbestandteile der Kapsomere ohne die übliche Anfärbung des Gels deutlich sichtbar. Nach der angegebenen Inkubationsdauer hat bereits ein weitgehender Abbau des Proteins in den Zellen stattgefunden (Fig. 4).

Dieses Beispiel zeigt, daß ein modifiziertes PyVP1-CallS-Protein mit zusätzlichem, singulären Cystein in geschützter Position hergestellt, durch Fluoreszenzfarbstoffe markiert, und zu Kapsiden assembliert werden kann. Die Kapside aus dieser Proteinvariante können in das Innere von eukaryontischen Zellen aufgenommen werden. Die Aufnahme kann durch die Fluoreszenzfarbstoffe nachgewiesen werden. Die Markierung beeinflußt nicht die übrigen Eigenschaften des Proteins.

### Beispiel 3

### Herstellung, Assemblierung und Charakteristerung von cysteinhaltigem Hüllprotein PyVP1 mit und ohne Fluoreszenzmarkierungsoption (2C/3C-Variante)

Die Ausbildung einer intrapentameren Disulfidbrücke zwischen den Aminosäurepositionen 20 und 115 von PyVP1 kann vorteilhaft für die Assemblierung und die Stabilität der Kapside sein. Daher wird eine Variante von PyVP1-CallS hergestellt, die an diesen beiden Aminosäurepositionen anstelle der dort vorhandenen Serine jeweils Cysteine enthalten. Die Mutagenese wird mit Hilfe des QuickChange-Verfahrens (Fa. Stratagene) nach Angaben des Herstellers durchgeführt. Dazu werden die folgenden Oligonukleotide verwendet: S20C: 5'-GCA CAA AGG CTT GTC CAA GAC CCG C-3' und 5'-GCG GGT CTT GGA CAA GCC TTT GTG C-3'. Als Vorlage wird die Variante S115C verwendet, die als Zwischenprodukt bei der Herstellung von PyVP1-CallS gemäß Beispiel 1 entsteht. Die so hergestellte Variante PyVP1-CallS-S20C-S 115C besitzt zwei Cysteine in geeigneter Position zur intrapentameren Disulfidverbrückung und wird im Folgenden als PyVP1-2C bezeichnet.

Ausgehend von dieser Variante PyVP1-2C kann eine weitere Variante hergestellt werden, die ein zusätzliches Cystein an Position 249 enthält und damit analog zum PyVP1-CallS-T249C aus Beispiel 2 spezifisch und neutral markierbar ist. Die Mutagenese erfolgt mit Hilfe des QuickChange-Verfahrens (Fa. Stratagene) analog zu Beispiel 2 und mit den dort beschriebenen Oligonukleotiden.

Bei der Herstellung der beiden Varianten gemäß Beispiel 1 wird als zusätzliches Additiv 30 mM DTT in den Lösungsmitteln verwendet, um das Protein in reduziertem Zustand zu halten. Die Oxidation der Disulfidbrücke im Kapsomer erfolgt nach Abtrennung des DTT im Rahmen der Dialyse zur Assemblierung der Kapsomere zu Kapsiden. Fig. 5 zeigt die Assemblierungskompetenz der Variante PyVP1-2C, wobei die Assemblierung durch Dialyse im übrigen analog zu Beispiel 1 erfolgt.

Ein besonderes Merkmal dieser Variante gegenüber der PyVP1-CallS-Variante ist die vollständige Assemblierung der Kapsomere zu Kapsiden unter oxidierenden Bedingungen. Freie, nichtassemblierte Kapsomere des Proteins sind unter den genannten Bedingungen nicht mehr vorhanden. Mit Hilfe der beiden beschriebenen Varianten kann ein quantitativer Einbau von Komponenten in die virusähnliche Hülle erreicht werden.

### Beispiel 4

### Herstellung, Assemblierung und Charakterisierung von Hüllprotein PyVP1, das ein Arginin-Glycin-Aspartat-Sequenzmotiv an der Oberfläche enthält (PyVP1-RGD-Varianten)

Ausgehend von PyVP1-CallS-T249C aus Beispiel 2 wurden zwei Varianten hergestellt, die in je einer separaten Loopstruktur auf der Außenseite der Kapsidhülle neue Sequenzen tragen. Ein besonderes Merkmal dieser neuen Sequenzsegmente ist das Auftreten einer Abfolge Arg-Gly-Asp (RGD). Mit diesen Varianten soll ein zellulärer Aufnahmemechanismus in die artifiziellen Kapside implantiert werden, der dem Mechanismus von Adenoviren vergleichbar ist. Für diese Virenklasse ist nach dem Stand der Technik bekannt, daß eine Bindung an zelluläre Integrin-Oberflächenrezeptoren die Aufnahme der Adenoviren in die Zellen ermöglicht.

Die Insertion der neuen Sequenzmotive wird einerseits zwischen den Sequenzpositionen 148 und 149, und andererseits zwischen den Aminosäurepositionen 293 und 295 vorgenommen Die entsprechenden Bereiche befinden sich gemäß der Struktur an der Außenseite der Kapside.

Die Einführung eines neuen Loopsegments mit alternierenden, flexiblen Serin-Glycin-Motiven an Position 148/149 (zur nachfolgenden Herstellung der Variante PyVP1-RGD148) erfolgt mit Hilfe des QuickChange-Verfahrens (Fa. Stratagene) unter Verwendung der folgenden Oligonukleotide: 5'-CAA CAA ACC CAC AGA TAC AGT AAA CGG CAG CGG CAG CGG CAG CGG CAG CGG CAG TGC AAA AGG AAT TTC CAC TCC AGT G-3' und 5'-CAC TGG AGT GGA AAT TCC TTT TGC ACT GCC GCT GCC GCT GCT GCC GCT GCC GCT GCC GTT TAC TGT ATC TGT GGG TTT GTT G-3'. Für die Einführung eines analogen Loopsegments an Stelle 293/295 (zur nachfolgenden Herstellung der Variante PyVP1-RGD293) werden die folgenden Oligonukleotide eingesetzt: 5'-GAT ATA ATG GGC TGG AGA GTT ACC GGC AGC GGC AGC GGC AGC AGC GGC AGC GGC AGT GGC TAT GAT GTC CAT CAC TGG AG-3' und 5'-CTC CAG TGA TGG ACA TCA TAG CCA CTG CCG CTG CCG CTG CTG CCG CTG CCG CTG CCG GTA ACT CTC CAG CCC ATT ATA TC-3'. In einem zweiten Schritt werden jeweils die Oligonukleotide 5'-CGG CAG CGG CAG CGG CAG CGG TCG TGG CGA TAG CGG CAG CGG CAG CGG CAG TG-3' und 5'-CAC TGC CGC TGC CGC TGC CGC TAT CGC CAC GAC CGC TGC CGC TGC CGC TGC CGT-3' verwendet, um die Arg-Gly-Asp-Sequenz in die neu geschaffenen Loopsegmente in den beiden beschriebenen Varianten zu inserieren. Nach dieser finalen Klonierung sind die beiden Varianten PyVP1-RGD148 und PyVP1-RGD293 auf Genebene hergestellt.

Die Herstellung und Reinigung der beiden Varianten erfolgte analog zu Beispiel 1. Die Assemblierung der beiden Varianten zu Kapsiden unter den in Beispiel 1 angegebenen Assemblierungsbedingungen ist erfolgreich, die Kapsomere sind nativ und assemblierungskompetent. Ferner ist bei diesen Varianten eine Markierung mit Fluoreszenzfarbstoffen am singulären Cystein C249, in Analogie zu Beispiel 2, möglich. Die assemblierten Kapside, bestehend aus fluoreszenzmarkierten Kapsomeren, können mit eukaryontischen Zellen (Typ Caco-2) inkubiert werden. Die Aufnahme der Kapside in die Zellen kann über die Fluoreszenzmarkierung mit Hilfe eines Fluoreszenz-Mikroskops verfolgt werden, eine Fixierung der Zellen ist dabei nicht erforderlich. Wie aus Fig. 6 ersichtlich ist, erfolgt eine Aufnahme der Kapside in die Zellen. Die PyVP1-RGD148-Variante (Fig. 6b) wird dabei bedeutend effizienter in die Zellen aufgenommen, als die vergleichbare Kontrollvariante PyVP1-CallS-T249C (Fig. 6a) ohne das RGD-Sequenzmotiv. Das implantierte RGD-Motiv bewirkt damit die Kapsidaufnahme über den effizienten Aufnahmeweg mittels integrinrezeptor-vermittelter Endozytose. Das Beispiel zeigt weiterhin, daß eine Steuerung der Aufnahme der Kapside in Zellen durch Verwendung geeigneter Komponenten möglich ist.

### Beispiel 5

### Herstellung, Assemblierung und Charakterisierung von Hüllprotein PyVP1, das eine Veränderung in der natürlichen Sialyllaktosebindungsstelle aufweist (PyVP1-R78W-Mutante)

Auf Basis der Variante PyVP1-3C wird eine weitere Variante hergestellt, die eine Mutation der Aminosäure Arginin 78 in Tryptophan enthält (PyVP1-3C-R78W). Die Position des Arginin 78 ist bei der Bindung des natürlichen Virus an Sialyllactose auf der Zelloberfläche, dem natürlichen Rezeptor des Polyomavirus, wesentlich beteiligt. Die Unterdrückung dieser Wechselwirkung durch die Mutation R78W, also einem Austausch des Arginins gegen ein strukturell inkompatibles Tryptophan, soll damit eine Aufnahme der Viruspartikel in die Zielzellen über die natürliche Rezeptorbindung verhindern.

Die angegebene Mutation wird durch das QuickChange-Verfahren (Fa. Stratagene) nach Angaben des Herstellers durchgeführt. Dazu werden die folgenden Oligonukleotide verwendet: 5'-CTA TGG TTG GAG CTG GGG GAT TAA TTT G-3' und 5'-CAA ATT AAT CCC CCA GCT CCA ACC ATA G-3'. Die Herstellung und Reinigung sowie die Assemblierung des resultierenden PyVp1-R78W-Variante erfolgt analog zu Beispiel 1.

Die Variante PyVP1-R78W ist wie die übrigen dokumentierten Varianten dazu in der Lage, zu Kapsiden zu assemblieren. Das Beispiel zeigt, daß der Zelltropismus der Kapside durch Variation der Oberflächen der Kapsidstrukturen manipuliert werden kann. Dabei können sowohl neue Zelltropismen eingeführt werden, als auch bestehende Zelltropismen beseitigt werden.

### Beispiel 6

### Herstellung und Charakterisierung von gemischten Kapsiden I

Die Herstellung von gemischten Kapsiden, d. h. Partikel, die mosaikartig aus mehreren verschiedenen molekularen Substanzen aufgebaut sind, ist eine besonders wichtige Eigenschaft der vorliegenden Erfindung. Zum Nachweis gemischter, aus unterschiedlichen Hüllproteinen aufgebauter Kapside, wird wie in Beispiel 2 beschrieben, die Variante PyVP1-CallS-T249C des Hüllproteins in einem Ansatz mit dem Fluoreszenzfarbstoff Fluorescein-Maleimid und in einem zweiten Ansatz mit Texas Red-Maleimid an das singuläre Cystein 249 gekoppelt. Die verschieden markierten Kapside werden in einem äquimolarem Verhältnis miteinander gemischt und assembliert. Die Analyse der Kapsidbildung erfolgt mittels Durchtluls-Cytometrie (FACS). Diese Technik ermöglicht eine Detektion unterschiedlicher Fluoreszenzen innerhalb eines Partikels. Figur 7 zeigt die Analyse äquimolar assemblierter Kapside. Es zeigt sich jeweils eine Population fluoreszenzmarkierter Kapside und freier nichtassemblierter Kapsomere (Figur 7a/b). Bei der Auftragung Fluorescein- gegen Texas Red-Fluoreszenz (Figur 7c) wird eine Population von Partikeln beobachtet, die gleichzeitig beide Fluoreszenzen tragen. Partikel, die nur mit einem Farbstoff markiert sind, können nicht detektiert werden, da sie offenbar nicht gebildet werden.

Mit diesem Beispiel wird gezeigt, daß sich Polyomavirus VP1-Hüllproteine, die unterschiedliche Eigenschaften aufweisen, mosaikartig assemblieren lassen, so daß sich Viruskapside bilden, die spezifisch die Eigenschaften von beiden Hüllproteinen aufweisen. Gleichzeitig wird eine Möglichkeit zur hochsensitiven Bestimmung und Analyse der Zusammensetzung der Viruskapside aufgezeigt.

### Beispiel 7

### Herstellung und Charakterisierung von gemischten Kapsiden II

Zur weiteren Demonstration der Vorteile der Erfindung wird eine Variante von PyVP1 hergestellt, die vollständig assemblierungsdefizient ist. Dazu wird mit Hilfe von PCR nach dem Stand der Technik zwischen die Positionen 293 und 295 der Aminosäuresequenz von PyVP1-CallS-T249C eine künstliche Peptidsequenz eingefügt (Sequenzabfolge GSGSG WTEHK SPDGR TYYYN TETKQ STWEK PDDGS GSG). Die Herstellung und Reinigung der Variante erfolgt gemäß den Angaben in Beispiel 1. Die produzierte Variante PyVP1-Def ist ein natives, pentameres Protein. Sie ist jedoch vollständig assemblierungsdefizient und kann unter den Assemblierungs-Standardbedingungen aus Beispiel 1 virusähnliche Kapside nicht ausbilden. Diese Assemblierungsdefizienz ist anhand von Gelfiltrationsuntersuchungen (Fig. 8a) gezeigt.

Die assemblierungsdefiziente Variante PyVP1-Def wird gemäß dem Beispiel 2 über das singuläre Cystein an Position 249 mit dem Fluoreszenzfarbstoff Fluorescein-Maleimid (Fa. Molecular Probes) markiert. Anschließend erfolgt eine Assemblierung in Gegenwart der Variante PyVP1-CallS in verschiedenen stöchiometrischen Verhältnissen der beiden Komponenten zueinander. Die Assemblierung wird durch Dialyse gemäß dem Beispiel 1 durchgeführt. Die Messung der Absorption des Fluoresceins bei 490 nm in einer Gelfiltrations-Analyse (Fig. 8b) zeigt, daß PyVP1-Def in die entstehenden Kapside eingebaut wird. Die Variation der stöchiometrischen Verhältnisse der beiden Kapsid-Komponenten bei der Assemblierung (Fig. 8c) demonstriert, daß der Einbau der assemblierungsdefizienten Variante PyVP1-Def in die gemischten Kapside proportional den Mengenverhältnissen der Varianten erfolgt.

Dieses Beispiel zeigt ebenfalls, daß gemischte Kapside verschiedener Varianten von Polyomavirus VP 1 unter Assemblierungsbedingungen gebildet werden können. Die in die Kapside eingebauten Kapsomere spiegeln darüberhinaus dabei die stöchiometrischen Mengenverhältnisse der Ausgangsbedingungen wieder. Das beschriebene Verfahren kann ferner dazu genutzt werden, Kapsomere in die Kapsidstrukturen einzubauen, die andernfalls assemblierungsdefizient sind.

### Beispiel 8

### Herstellung und Charakterisierung von gemischten Kapsiden III

Die Assemblierung cysteinfreier VP1-Varianten, wie beispielsweise PyVP1-CallS aus Beispiel 2, kann den Nachteil haben, daß nicht alle, beispielsweise etwa 50 % der eingesetzten Kapsomere, Kapside bilden. Außerdem können diese Kapside relativ instabil sein und teilweise nach Isolierung dissemblieren. Dieser Nachteil läßt sich durch eine gemischte Assemblierung mit cysteinhaltigen Varianten kompensieren.

Die cysteinfreie Variante PyVP1-CallS-WW150 zeigt eine verminderte Assemblierungsfähigkeit; ca. 15 % gegenüber 50 % bei PyVP1-CallS (Figur 9a), wohingegen cysteinhaltige Kapsomere der Variante VP1-wt ähnlich zu PyVP1-2C und PyVP1-3C aus Beispiel 3 unter geeigneten Bedingungen vollständig zu Viruskapsiden assemblieren (Figur 9b). Eine äquimolare Mischung der Varianten PyVP1-CallS-WW150 und PyVP1-wt unter Assemblierungsbedingungen führt zu mosaikartig gemischten Viruskapsiden. Das wird dadurch deutlich, daß die Mischung vollständig assembliert und keine freien Kapsomere mehr detektiert werden können (Figur 9c).

Auch in diesem Beispiel wird die Bildung mosaikartig aufgebauter Viruskapside belegt. Weiterhin wird die Möglichkeit demostriert, cysteinhaltige mit cysteinfreien Varianten zu kombinieren, wobei die Eigenschaften der cysteinhaltigen Kapsomere, eine vollständige Assemblierung zu stabilen Viruskapsiden, auf das gesamte Kapsid übertragen wird. Dieser Effekt ermöglicht es, ansonsten cysteinfreie Kapsomere an einem an definierter Stelle eingeführten Cysteinrest hochspezifisch zu modifizieren (beispielsweise mit PyVP1-CallS) und diese neue Funktion in ein Viruskapsid einzuführen, wobei die Nachteile cysteinfreier Assemblierung (geringere Assemblierungseffizienz, instabilere Kapside) umgangen werden.

### Beispiel 9

### Transfektion von Zellen mit virusähnlichen Kapsiden

Die Variante PyVP1-CallS-T249C kann nach Beispiel 2 hergestellt, assembliert, und fluoreszenzmarkiert werden. Die markierten Kapside konnen mit Hilfe von konfokaler Laserscan-Mikroskopie (CLSM) nach Aufnahme in eukaryontische Zellen vom Typ C2C12 intrazellulär dargestellt werden. Somit bietet diese PyVP1-Variante die Möglichkeit, Effizienz und Aufnahmemechanismus von homogen oder heterogen (gemischt) assemblierten, mosaikartig aufgebauten Kapsiden zu untersuchen. Dazu wird fluoreszenzmarkiertes PyVP1-CallS-T249C in die Kapsidpartikel mit eingebaut.

In Fig. 10 ist eine Serie von Experimenten zur Aufnahme von Kapsiden, bestehend aus assembliertem PyVP1-CallS-T249C, in C2C12-Zellen dargestellt. Zusätzlich zur Anfärbung der Kapside (rot, Farbstoff Texas Red, Fa. Molecular Probes) sind späte Endosomen (grün, Farbstoff Fluorescein-Dextran 70 kDa, Fa. Molecular Probes), Zellkerne (grün, Farbstoff SYTO-16, Fa. Molecular Probes), und Lysosomen (blau, Farbstoff LysoSensor Blue-Yellow, Fa. Molecular Probes) visualisiert. Die Kapside werden über Endozytose in die Zellen aufgenommen, durchlaufen frühe und späte (nach 15 min) Endosomen, und werden schließlich in Lysosomen (60 min) angereichert.

Das Beispiel demonstriert, daß mit Hilfe der vorstehend beschriebenen Varianten eine Analyse der Eigenschaften der Komponenten möglich ist, und diese Untersuchungen können auch zelluläre Lokalisationen und Wirkmechanismen der Kapside umfassen. Damit ist eine Möglichkeit dargestellt, die biologischen Eigenschaften der artifiziell hergestellten, mosaikartigen Kapside durch den Einsatz von Markierungen zu untersuchen und zu beschreiben, wobei die Markierung selbst neutral ist und keinen Einfluß auf diese Eigenschaften hat.

### Beispiel 10

### Transfektion von Zellen mit Komplexen aus DNA und virusähnlichen Kapsiden

Zur Demonstration des Transports von DNA mittels virusähnlicher Kapside in eukaryontische Zellen werden 100 µl einer Lösung des Proteins PyVP1-3C (1 mg/ml in 20 mM HEPES pH 7.2, 100 mM NaCl, 10 mM DTT, 1 mM EDTA, 5 % Glycerol) mit 7.5 µl einer Lösung des Plasmids pEGFP-N1 (Fa. Clontech) sowie 100 µl Dialysepuffer (20 mM Natriumacetat, pH 5.0, 100 mM NaCl, 1 mM CaCl₂, 5% Glycerol) versetzt und über 4 Tage bei Raumtemperatur unter häufigem Pufferwechsel gegen Dialysepuffer dialysiert. Zur Herstellung des Transfektionsmediums werden 100 µl dieser Reaktionsmischung mit 300 µl Dulbecco's Modified Eagle Medium (DMEM) + 1 µM Chloroquin gemischt. Für den Transfektionstest werden NIH-3T3-Zellen eingesetzt, die am Vortag in einer 12-Well-Platte in einer Dichte von 10 000 Zellen pro Well ausgesät wurden. Zur Transfektion werden die Zellen mit PBS (Phosphate buffered saline) gewaschen und mit 400 µl Transfektionsmedium pro Well versetzt. Die Zellen werden für 1 h bei 37 °C und mit 5 % CO₂ inkubiert, anschließend mit PBS gewaschen und mit Vollmedium (DMEM + 10 % FBS) für weitere 48 h bei 37 °C und 5 % CO₂ inkubiert. Nach dieser Zeit kann eine erfolgreiche Transfektion anhand der Expression eines Reportergens detektiert werden. Das hier verwendete Plasmid pEGFP-N1 erlaubt die Expression von GFP (Green Fluorescent Protein), das anhand seiner grünen Fluoreszenz in den Zellen detektiert werden kann. Mit dem hier beschriebenen Protokoll können durchschnittlich etwa 20 Zellen pro Well identifiziert werden, die eindeutig das Reporterprotein GFP produzieren. Dieses Beispiel zeigt, daß mit dem hier beschriebenen Transportsystem, bestehend aus PyVP1-3C, erfolgreich DNA in eukaryontische Zellen transportiert werden kann. Zusätzlich kann die eingebrachte DNA in den Zellen freigesetzt und ein Reportergen korrekt exprimiert werden.

### Beispiel 11

### Herstellung und Charakterisierung von Listeriolysin O (LLO)

Wie aus Beispiel 7 ersichtlich wird, wird ein großer Anteil der hergestellten virusanalogen Kapside nach der Aufnahme in eukaryontische Zellen in zellulären Lysosomen angereichert und schließlich abgebaut. Eine Freisetzung der Partikel aus dem endosomalen Kompartiment kann durch die Zugabe von Zytolysinen bewirkt werden. Ein Modell hierfür ist das Listeriolysin O (LLO) aus dem Organismus *Listeria monocytogenes*.

Das LLO-Gen wird mit Hilfe von PCR nach Standardverfahren aus einem Fragment genomischer DNA von *Listeria monocytogenes* amplifiziert. Dazu wird mit Hilfe der Oligonukleotide 5'-TAT AGA CGT CCG ATG CAT CTG CAT TCA ATA AAG AAA ATT-3' und 5'-TAG TTA AGG CTG CGA TTG GAT TAT CTA CAC TAT TAC TA-3' eine Klonierung in den Vektor pTIP vorgenommen. Dieser Vektor pTIP ist ein Derivat des in Beispiel 1 dokumentierten Intein-Expressionsvektors auf Basis von pET21 a, mit zusätzlich eingeführten prolinreichen Sequenzen. Der Vektor ist dabei so konstruiert, daß wahlweise am 5'- oder 3'-Ende eines über eine multiple Klonierungsstelle eingefügten Gens eine prolinreiche Sequenz anfusioniert werden kann. Die prolinreiche Sequenz enthält dabei vorwiegend Pro-Pro-Pro-Pro-Pro-Pro-Pro-Pro-Leu-Pro.

In einer zweiten PCR wird das Genfragment aus dem pTIP-Vektor amplifiziert und in einen pET34b-Vektor (Fa. Novagen) kloniert. Als Oligonukleotide hierfür werden 5'-GCC GCC ACC TCC ACC GCC AC-3' und 5'-ATT AGG GTT CGA TTG GAT TAT CTA CAC TAT TAC-3' verwendet. Der Vektor wird mit Srt 1 (Fa. Stratagene) *blunt end* geschnitten und das DNA-Fragment wird *blunt end* in den Vektor pET34b ligiert. Das hergestellte Konstrukt ermöglicht eine Expression des mittels prolinreicher Sequenz markierten LLO-Proteins als N-terminales Fusionsprotein mit einer Cellulose-Bindungsdomäne. Diese Bindungsdomäne kann nach erfolgreicher Affinitätsreinigung mit Hilfe von Enterokinase proteolytisch abgetrennt werden. Die Herstellung des Fusionsproteins erfolgt durch Anzucht von transformierten BL21(DE3)-Zellen bei 25 °C nach Induktion mit 1 mM IPTG. Der Zellaufschluß erfolgt gemäß Beispiel 1. Als Resuspensionspuffer wird dabei 20 mM HEPES, 200 mM NaCl, pH 7.0 verwendet. Zur Entfernung bakterieller DNA wird der Zellextrakt mit 5 mM MgCl₂ und 0.1 U Benzonase versetzt und für 30 min bei 25 °C inkubiert.

Anschließend erfolgt die Reinigung des Fusionsproteins durch Auftragen des Zellextraktes auf eine Cellulosematrix (Fa. Novagen) gemäß Herstellerangaben. Die Elution des Fusionsproteins erfolgt mit 1 Säulenvolumen Ethylenglykol (Fa. Merck). Das eluierte Protein wird sofort gegen Resuspensionspuffer dialysiert. Die Abspaltung der Cellulose-Bindungsdomäne aus dem Fusionsprotein wird mit Hilfe von Enterokinase nach Herstellerangaben durchgeführt.

Fig. 11a zeigt ein SDS-Elektrophoresegel, das die Herstellung und Reinigung des LLO dokumentiert. Die Aktivität des Proteins ist in Fig. 11b demonstriert. Das Protein kann unter geeigneten Lösungsmittelbedingungen (pH < 6.0) Poren in cholesterinhaltige Lipiddoppelschichten einfügen. Dies ist anhand synthetischer, cholesterinhaltiger Liposomen gezeigt, die nach Standardverfahren hergestellt wurden. Dabei wird ein Fluoreszenzfarbstoff (Calcein) aus den synthetischen Liposomen freigesetzt, und es erfolgt eine meßbare Erhöhung des Fluoreszenzsignals in der Lösung (Fig. 11b).

Das Beispiel zeigt, daß das Protein LLO rekombinant in aktiver Form hergestellt werden kann. Darüber hinaus ist gezeigt, daß LLO biologische Membranen, wie sie bei Endosomen vorkommen, aufzulösen vermag. In Zusammenhang mit den Kapsiden aus Beispiel 1 bis 7 kann diese Eigenschaft zur Endosomenfreisetzung aufgenommener Kapside eingesetzt werden.

## Patentansprüche

1. Transportsystem für molekulare Substanzen, enthaltend rekombinant hergestellte Teileinheiten auf Aminosäurebasis, das folgende Merkmale aufweist:
- mindestens zwei voneinander einfach verschieden modifizierte Teileinheiten, und/oder
- eine oder mehrere zumindest zweifach verschieden modifizierte Teileinheiten,
wobei die Teileinheiten mosaikartig zu dem Transportsystem zusammensetzbar sind und molekulare Substanzen in das Transportsystem einschließbar sind, und
wobei es sich bei den modifizierten Teileinheiten um Polyomavirus VP1-Protein Teileinheiten handelt, die als eine Modifikation eine Einführung von ein oder mehreren Aminosäuren, Peptid- oder Proteinsequenzen oder Proteindomänen in einer Loop-Region an der Außenseite des Proteins zwischen den Aminosäurepositionen 148 und 149 und/ oder 293 und 295 aufweist.

2. Transportsystem gemäß Anspruch 1,
**dadurch gekennzeichnet,**
**daß** das Transportsystem mindestens eine VP1-Teileinheit aufweist, die als Modifikation zwischen den Aminosäurepositionen 148 und 149 und/oder 293 und 295 Proteine oder Proteindomänen, Peptide und Peptidhormone, Glykoproteine, Antikörper oder modifizierte Antikörper, Antigene oder isolierte Rezeptorbindungsdomänen von Liganden aufweist, durch die eine verbesserte Aufnahme in Zielzellen erreicht wird.

3. Transportsystem gemäß Anspruch 1,
**dadurch gekennzeichnet,**
**daß** das Transportsystem mindestens eine VP1-Teileinheit aufweist, die eine Modifikation zwischen den Aminosäurepositionen 148 und 149 und/oder 293 und 295 aufweist, wodurch ein Transport in bestimmte Zellorganellen der Zielzellen oder aus den Zielzellen hinaus bewirkt wird.

4. Transportsystem nach einem oder mehreren der Ansprüche 1 bis 3,
**dadurch gekennzeichnet,**
**daß** das Transportsystem mindestens eine VP1-Teileinheit aufweist, die als Modifikation zwischen den Aminosäurepositionen 148 und 149 und/oder 293 und 295 eine RGD-Sequenz, eine prolinreiche Sequenz, eine WW-Sequenz, SH3-Domäne, Biotin, Avidin, Streptavidin oder polyionische Sequenzen aufweist.

5. Transportsystem nach einem oder mehreren der vorhergehenden Ansprüche,
**dadurch gekennzeichnet,**
**daß** die rekombinant hergestellten modifizierten VP1-Teileinheiten als weitere Modifikation Punktmutationen aufweisen.

6. Transportsystem nach einem oder mehreren der vorhergehenden Ansprüche,
**dadurch gekennzeichnet,**
**daß** die rekombinant hergestellten modifizierten VP1-Teileinheiten als weitere Modifikation mit einem Fluoreszenzfarbstoff, Oligonukleotiden, Peptiden, Peptidhormonen, Lipiden, Fettsäuren, oder Kohlenhydraten markiert sind.

7. Transportsystem nach einem oder mehreren der vorhergehenden Ansprüche,
**dadurch gekennzeichnet,**
**daß** die rekombinant hergestellten modifizierten VP1-Teileinheiten als weitere Modifikation eine Veränderung der Cystein-Zusammensetzung aufweisen.

8. Transportsystem nach Anspruch 1,
**dadurch gekennzeichnet,**
**daß** wahlweise unmodifizierte Teileinheiten enthalten sind.

9. Transportsystem nach einem oder mehreren der vorhergehenden Ansprüche,
**dadurch gekennzeichnet,**
**daß** Proteine, die Bestandteil der Hülle sind, durch die Fusion von Glycin-Alanin-reichen Sequenzen am aminoterminalen Ende eine verminderte zelluläre Abbaurate aufweisen, wodurch eine Stimulierung des Immunsystems in lebenden Organismen unterbleibt oder verringert wird.

10. Transportsystem nach einem oder mehreren der vorhergehenden Ansprüche,
**dadurch gekennzeichnet,**
**daß** das Transportsystem mit einer vor einer Immunantwort des Organismus schützenden Hülle überzogen wird.

11. Transportsystem nach einem oder mehreren der vorhergehenden Ansprüche,
**dadurch gekennzeichnet,**
**daß** die Hülle aus Polyethylenglykol besteht oder in Form einer synthetisch hergestellten Lipidmembran ausgeführt ist.

12. Transportsystem nach einem oder mehreren der vorhergehenden Ansprüche,
**dadurch gekennzeichnet,**
**daß** die rekombinant hergestellten Teileinheiten durch Punktmutationen oder durch Einführung, Entfernung oder Austausch von ein oder mehreren Peptid- oder Proteinsequenzen oder Proteindomänen an dem Terminus/den Termini und/oder in der Sequenz der Teileinheit modifiziert sind, oder daß die rekombinant hergestellten Teileinheiten so modifiziert sind, daß sie effizient und/oder gezielt durch Zielzellen aufnehmbar sind und/oder die molekularen Substanzen besser an die Teileinheiten bind- oder anlagerbar sind.

13. Transportsystem nach Anspruch 12,
**dadurch gekennzeichnet,**
**daß** die rekombinant hergestellten Teileinheiten mit bakteriellen Cytolysinen oder viralen Proteinen und/oder mit Translokationsdomänen bakterieller Toxine modifiziert werden.

14. Verfahren zur Herstellung von Transportsystemen nach einem oder mehreren der vorhergehenden Ansprüche mit den folgenden Schritten:
- Rekombinante Expression der Polyomavirus VP1-Protein Teileinheiten,
- Freisetzung der Teileinheiten durch Lyse der Wirtszellen,
- Inkontaktbringen der Teileinheiten in den gewünschten stöchiometrischen Verhältnissen, um das Transportsystem mosaikartig zusammenzusetzen/zu assemblieren.

15. Verfahren nach Anspruch 14, mit folgendem weiteren Schritt:
- vor oder während der Assemblierung Inkontaktbringen mit molekularen Substanzen, um die molekularen Substanzen in das Transportsystem einzuschließen.

16. Verwendung der Transportsysteme nach einem der Ansprüche 1 bis 13 zum Transfer von molekularen Substanzen in Zellen.

17. Verwendung nach Anspruch 16 zum Transfer von DNA in eukaryontische Zellen.

18. Verwendung nach Anspruch 16 oder 17,
**dadurch gekennzeichnet,**
**daß** als molekulare Substanzen einzelsträngige oder doppelsträngige DNA, einzelsträngige oder doppelsträngige RNA, Peptide, Peptidhormone, Proteine, Proteindomänen, Glykoproteine, Ribozyme, PNA (Peptide nucleic acid), Nukleotide, Hormone, Lipide oder Kohlenhydrate transferiert werden.

19. Verwendung nach einem oder mehreren der Ansprüche 16 bis 18,
**dadurch gekennzeichnet,**
**daß** die in das Transportsystem eingeschlossenen Substanzen mit einem Signalmolekül versehen sind, so daß sie wahlweise in den Zellkern, die Mitochondrien, das Endoplasmatische Reticulum, oder aus der Zelle transportiert werden.

20. Verwendung nach einem oder mehreren der Ansprüche 16 bis 19,
**dadurch gekennzeichnet,**
**daß** Cytolysine, bevorzugt Listeriolysin O, zugegeben werden, um die Transportsysteme aus den zellulären Lysosomen freizusetzen.

21. Verwendung nach einem oder mehreren der Ansprüche 16 bis 20,
**dadurch gekennzeichnet,**
**daß** Teileinheiten des molekularen Transportsystems mit Fluoreszenzfarbstoffen markiert sind.

22. Pharmazeutische Zusammensetzung, enthaltend ein oder mehrere Transportsysteme nach einem oder mehreren der Ansprüche 1 bis 13, zusammen mit üblichen pharmazeutisch verträglichen Träger- und Hilfsstoffen.

## Claims

1. Transport system for molecular substances, containing recombinantly produced partial units based on amino acids, including the following features:
- at least two partial units modified once differently from each other, and/or
- one or several partial units modified differently at least twice
wherein the partial units are able to assemble to said transport system like a mosaic and
wherein molecular substances can be encapsidated into the transport system, and
wherein the modified partial units are partial units from the Polyomavirus VP1 protein which contain as a modification an introduction of one or more amino acids, peptide or protein sequences or protein domains in a loop region at the outside of the protein between amino acid positions 148 and 149 and/or 293 and 295.

2. Transport system according to claim 1,
**characterized in that** the transport system contains at least one VP1 partial unit comprising as modification between amino acid positions 148 and 149 and/or 293 and 295 proteins or protein domains, peptides and peptide hormones, glycoproteins, antibodies or modified antibodies, antigens or isolated receptor binding domains of ligands by which an improved uptake into target cells is achieved.

3. Transport system according to claim 1, **characterized in that**
the transport system comprises at least one VP1 partial unit which comprises a modification between amino acid positions 148 and 149 and/or 293 and 295 by which a transport in particular cell organelles of the target cells or out of the target cells is effected.

4. Transport system in accordance with one or more of claims 1 to 3, **characterized in that**
the transport system comprises at least one VP1 partial unit which comprises as a modification between amino acid positions 148 and 149 and/or 293 and 295 an RGD-sequence, a proline rich sequence, a WW-sequence, SH3-domain, biotin, avidin, streptavidin or polyionic sequences.

5. Transport system in accordance with one or more of the preceding claims, **characterized in that**
the recombinantly prepared modified VP1 partial units comprise as further modification point mutations.

6. Transport system in accordance with one or more of the preceding claims, **characterized in that**
the recombinantly prepared modified VP1 partial units are labelled as a further modification with a fluorescence dye, oligonucleotides, peptides, peptide hormones, lipids, fatty acids or carbohydrates.

7. Transport system in accordance with one or more of the preceding claims, **characterized in that**
the recombinantly prepared modified VP1 partial units comprise as a further modification a modification of the cysteine composition.

8. Transport system in accordance with claim 1, **characterized in that** it includes optionally unmodified partial units.

9. Transport system in accordance with one or more of the preceding claims, **characterized in that**
proteins which are a component of the envelope provide a reduced cellular degradation rate due to the fusion with glycine-alanine-rich sequences whereby a stimulation of the immune system in living organisms is suppressed or reduced.

10. Transport system in accordance with one or more of the preceding claims, **characterized in that**
the transport system is covered by a coating that protects it against an immune response of the organism.

11. Transport system according to one or several of the previous claims,
**characterized by** the fact,
that the coating consists of polyethylene glycol or is carried out in the form of a synthetically produced lipid membrane.

12. Transport system according to one or several of the previous claims,
**characterized by** the fact,
that the recombinantly produced partial units are modified by point mutations or by insertion, deletion or exchange of one or several peptide or protein sequences or protein domains at the terminus/the termini and/or in the sequence of the partial unit, or that the recombinantly produced partial units are modified in such a way that they may be taken up efficiently and/or directed against target cells, and/or the molecular substances can be better bound and associated to the partial units.

13. Transport system according to claim 12,
**characterized by** the fact,
that the recombinantly prepared partiaul units are modified by bacterial cytolysins or viral proteins and/or translocation domains of bacterial toxins.

14. Method for the production of transport systems according to one or several of the previous claims with the following steps:
- recombinant expression of the partial units of the polyomavirus VP1 protein,
- release of the partial units by lysis of the host cells,
- creating contact between the partial units in the desired stoichiometric relations in order to compose/to assemble the transport system in a mosaic-like fashion.

15. Method according to claim 14 with the following further step:
- creating contact with molecular substances, either before or during the assembly, in order to encapsidate the molecular substances into the transport system.

16. Use of the transport systems according to one of claims 1 to 13 for the transfer of molecular substances into cells.

17. Use according to claim 16 for the transfer of DNA into eukaryotic cells.

18. Use according to claim 16 or 17, **characterized in that**
single-stranded or double-stranded DNA, single-stranded or double-stranded RNA, peptides, peptide hormones, proteins, protein domains, glycoproteins, ribozymes, PNA (peptide nucleic acid), nucleotides, hormones, lipids or carbohydrates can be transferred as molecular substances.

19. Use according to one or more of claims 16 to 18, **characterized in that**
the substances encapsidated in the transport system are supplied with a signal molecule so that they are transported either into the nucleus, the mitochondria, the endoplasmatic reticulum, or out of the cell.

20. Use according to one or more of claims 16 to 19, **characterized in that**
cytolysins, preferably listeriolysin O, are added to release the transport systems out of the cellular lysosomes.

21. Use in accordance with one or more of claims 16 to 20, **characterized in that** the partial units of the molecular transport system are labelled with fluorescence dyes.

22. Pharmaceutical composition comprising one or more of the transport systems in accordance with one or more of claims 1 to 13, in combination with conventional pharmaceutically acceptable carriers and excipients.

## Revendications

1. Système de transport pour des substances moléculaires contenant des motifs partiels fabriqués de manière recombinante à base d'acides aminés, qui présente les caractéristiques suivantes :
- au moins deux motifs partiels modifiés une fois différemment l'un de l'autre,
- un ou plusieurs motifs modifiés au moins deux fois différemment,
où les motifs partiels peuvent être assemblés à la manière d'une mosaïque en le système de transport et des substances moléculaires peuvent être incluses dans le système de transport, et
où les motifs partiels modifiés sont des motifs partiels de protéine de polyomavirus VP1, qui présentent comme modification une introduction d'un ou de plusieurs acides aminés, séquences peptidiques ou protéiques ou domaines de protéine dans une région de boucle sur la façade externe de la protéine entre les positions des acides aminés 148 et 149 et/ou 293 et 295.

2. Système de transport selon la revendication 1, **caractérisé en ce que** le système de transport présente au moins un motif partiel de VP1, qui présente comme modification entre les positions des acides aminés 148 et 149 et/ou 293 et 295 des protéines ou domaines de protéine, peptides et hormones de peptides, glycoprotéines, anticorps ou anticorps modifiés, antigènes ou domaines de liaison au récepteur de ligands isolés, grâce auxquels un accueil amélioré dans les cellules cibles est obtenu.

3. Système de transport selon la revendication 1,
**caractérisé en ce que** le système de transport présente au moins un motif partiel de VP1, qui présente une modification entre les positions des acides aminés 148 et 149 et/ou 293 et 295, moyennant quoi un transport vers certaines organelles cellulaires des cellules cibles ou en dehors des cellules cibles est déclenché.

4. Système de transport selon l'une ou plusieurs des revendications 1 à 3, **caractérisé en ce que** le système de transport présente au moins un motif partiel de VP1, qui présente comme modification entre les positions des acides aminés 148 et 149 et/ou 293 et 295 une séquence RGD, une séquence riche en proline, une séquence WW, un domaine SH3, de la biotine, de l'avidine, de la streptavidine ou des séquences polyioniques.

5. Système de transport selon l'une ou plusieurs des revendications précédente**s, caractérisé en ce que** les motifs partiels de VP1 modifiés et fabriqués de manière recombinante présentent comme modification des mutations ponctuelles.

6. Système de transport selon l'une ou plusieurs des revendications précédentes, **caractérisé en ce que** les motifs partiels de VP1 modifiés et fabriqués de manière recombinante sont marqués, comme autre modification, avec un colorant par fluorescence, des oligonucléotides, des peptides, des hormones de peptides, des lipides, des acides gras, ou des hydrates de carbone.

7. Système de transport selon l'une ou plusieurs des revendications précédentes, **caractérisé en ce que** les motifs partiels de VP1 modifiés et fabriqués de manière recombinante présentent comme autre modification une modification de la composition de la cystéine.

8. Système de transport selon la revendication 1, **caractérisé en ce qu'**il contient optionnellement des motifs partiels non modifiés.

9. Système de transport selon l'une ou plusieurs des revendications précédentes, **caractérisé en ce que** les protéines qui font partie de l'enveloppe présentent un taux de dégradation cellulaire réduit grâce à la fusion des séquences riches en glycine - alanine sur l'extrémité aminoterminale, moyennant quoi une stimulation du système immunitaire dans les organismes vivants est réprimée ou réduite.

10. Système de transport selon l'une ou plusieurs des revendications précédentes, **caractérisé en ce que** le système de transport est revêtu d'une enveloppe protégeant d'une réponse immunitaire de l'organisme.

11. Système de transport selon l'une ou plusieurs des revendications précédentes, **caractérisé en ce que** l'enveloppe est constituée de polyéthylène glycol ou est mise en oeuvre sous la forme d'une membrane lipidique fabriquée par synthèse.

12. Système de transport selon l'une ou plusieurs des revendications précédentes, **caractérisé en ce que** les motifs partiels fabriqués de manière recombinante sont modifiés par mutations ponctuelles ou par introduction, élimination ou échange d'une ou de plusieurs séquences de peptide ou de protéine ou d'un ou de plusieurs domaines de protéine sur la terminaison/sur les terminaisons et/ou dans la séquence du motif partiel, ou que les motifs partiels fabriqués de manière recombinante sont modifiés de façon à pouvoir être accueillis efficacement et/ou de manière ciblée par des cellules cibles et/ou les substances moléculaires peuvent être mieux liées ou additionnées aux motifs partiels.

13. Système de transport selon la revendication 12, **caractérisé en ce que** les motifs partiels fabriqués de manière recombinante sont modifiés avec des cytolysines bactériennes ou des protéines virales et/ou avec des domaines de translocation de toxines bactériennes.

14. Procédé de fabrication de systèmes de transport selon l'une ou plusieurs des revendications précédentes, comprenant les étapes suivantes :
- expression recombinante des motifs partiels de protéine de polyomavirus VP1,
- libération des motifs partiels par lyse des cellules hôtes,
- mise en contact des motifs partiels selon les rapports stoechiométriques voulus pour assembler/monter le système de transport à la manière d'une mosaïque.

15. Procédé selon la revendication 14, comprenant l'autre étape suivante :
- mise en contact, avant ou pendant l'assemblage, avec des substances moléculaires pour inclure les substances moléculaires dans le système de transport.

16. Utilisation des systèmes de transport selon l'une des revendications 1 à 13 pour le transfert de substances moléculaires dans des cellules.

17. Utilisation selon la revendication 16 pour le transfert de l'ADN dans des cellules eucaryotes.

18. Utilisation selon la revendication 16 ou 17, **caractérisée en ce que** l'on transfère, à titre de substances moléculaires, de l'ADN simple brin ou double brin, de l'ARN simple brin ou double brin, des peptides, des hormones de peptides, des protéines, des domaines de protéine, des glycoprotéines, des ribozymes, de l'APN (acide nucléique peptidique), des nucléotides, des hormones, des lipides ou des hydrates de carbone.

19. Utilisation selon l'une ou plusieurs des revendications 16 à 18, **caractérisée en ce que** les substances incluses dans le système de transport sont dotées d'une molécule signal, pour qu'elles soient transportées au choix dans le noyau cellulaire, les mitochondries, le réticulum endoplasmique ou hors de la cellule.

20. Utilisation selon l'une ou plusieurs des revendications 16 à 19, **caractérisée en ce que** des cytolysines, de préférence la listériolysine O, sont additionnées pour libérer les systèmes de transport hors des lysosomes cellulaires.

21. Utilisation selon l'une ou plusieurs des revendications 16 à 20, **caractérisée en ce que** les motifs partiels du système de transport moléculaire sont marqués avec des colorants par fluorescence.

22. Composition pharmaceutique, contenant un ou plusieurs systèmes de transport selon l'une ou plusieurs des revendications 1 à 13, conjointement avec les excipients et auxiliaires usuels pharmaceutiquement acceptables.


### SEQUENZPROTOKOLL

<110> ACGT ProGenomics AG
<120> Modulare Transportsysteme für molekulare Substanzen und deren Herstellung und Verwendung
<130> P12605
<140>
   <141>
<150> DE - 199 52 957.4
   <151> 1999-11-03
<160> 22
<170> PatentIn Ver. 2.1
<210> 1
   <211> 1152
   <212> DNA
   <213> Künstliche Sequenz
<220>
   <223> Beschreibung der künstlichen Sequenz: Variante des Polyomavirus Hüllproteins VP1 (PyVP1-CallS)
<220>
   <221> CDS
   <222> (1)..(1152)
<400> 1
   atg gcc ccc aaa aga aaa agc ggc gtc tct aaa agc gag aca aaa agc    48
   Met Ala Pro Lys Arg Lys Ser Gly Val Ser Lys Ser Glu Thr Lys Ser
    1               5                  10                  15
   aca aag gct agc cca aga ccc gca ccc gtt ccc aaa ctg ctt att aaa    96
   Thr Lys Ala Ser Pro Arg Pro Ala Pro Val Pro Lys Leu Leu Ile Lys
               20                  25                  30
   ggg ggt atg gag gtg ctg gac ctt gtg aca ggg cca gac agt gtg aca   144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct   192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc aga ggg att     240
   Glu Ser Leu Thr Glu Gly Gly Gln Tyr Tyr Gly Trp Ser Arg Gly Ile
    65                  70                  75                  80
   aat ttg gct aca tca gat aca gag gat tcc cca gga aat aat aca ctt     288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac     336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc     384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa     432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc     480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag     528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta     576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc     624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca     672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac     720
   Val Glu Ile Trp His Pro Asp Pro Ala Lys Asn Glu Asn Thr Arg Tyr
   225                 230                 235                 240
   ttt ggc aat tac act gga ggc acg aca acc cca ccc gtc ctg cag ttc     768
   Phe Gly Asn Tyr Thr Gly Gly Thr Thr Thr Pro Pro Val Leu Gln Phe
                   245                 250                 255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc    912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat    960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
       370                 375                 380
<210> 2
   <211> 384
   <212> PRT
   <213> Künstliche Sequenz
   <223> Beschreibung der künstlichen Sequenz: Variante des Polyomavirus Hüllproteins VP1 (PyVP1-CallS)
<400> 2
   Met Ala Pro Lys Arg Lys Ser Gly Val Ser Lys Ser Glu Thr Lys Ser
   1               5                   10                  15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
   35 40 45
   Glu Ile Glu Ala Phe Leu Asn Pro Arg Met Gly Gln Pro Pro Thr Pro
   50 55 60
   Glu Ser Leu Thr Glu Gly Gly Gln Tyr Tyr Gly Trp Ser Arg Gly Ile
   65 70 75 80
   Asn Leu Ala Thr Ser Asp Thr Glu Asp Ser Pro Gly Asn Asn Thr Leu
   85 90 95
   Pro Thr Trp Ser Met Ala Lys Leu Gln Leu Pro Met Leu Asn Glu Asp
   100 105 110
   Leu Thr Ser Asp Thr Leu Gln Met Trp Glu Ala Val Ser Val Lys Thr
   115 120 125
   Glu Val Val Gly Ser Gly Ser Leu Leu Asp Val His Gly Phe Asn Lys
   130 135 140
   Pro Thr Asp Thr Val Asn Thr Lys Gly Ile Ser Thr Pro Val Glu Gly
   145 150 155 160
   Ser Gln Tyr His Val Phe Ala Val Gly Gly Glu Pro Leu Asp Leu Gln
   165 170 175
   Gly Leu Val Thr Asp Ala Arg Thr Lys Tyr Lys Glu Glu Gly Val Val
   180 185 190
   Thr Ile Lys Thr Ile Thr Lys Lys Asp Met Val Asn Lys Asp Gln Val
   195 200 205
   Leu Asn Pro Ile Ser Lys Ala Lys Leu Asp Lys Asp Gly Met Tyr Pro
   210 215 220
   Val Glu Ile Trp His Pro Asp Pro Ala Lys Asn Glu Asn Thr Arg Tyr
   225 230 235 240
   Phe Gly Asn Tyr Thr Gly Gly Thr Thr Thr Pro Pro Val Leu Gln Phe
   245 250 255
   Thr Asn Thr Leu Thr Thr Val Leu Leu Asp Glu Asn Gly Val Gly Pro
   260 265 270
   Leu Ser Lys Gly Glu Gly Leu Tyr Leu Ser Ser Val Asp Ile Met Gly
   275 280 285
   Trp Arg Val Thr Arg Asn Tyr Asp Val His His Trp Arg Gly Leu Pro
   290 295 300
   Arg Tyr Phe Lys Ile Thr Leu Arg Lys Arg Trp Val Lys Asn Pro Tyr
   305 310 315 320
   Pro Met Ala Ser Leu Ile Ser Ser Leu Phe Asn Asn Met Leu Pro Gln
   325 330 335
   Val Gln Gly Gln Pro Met Glu Gly Glu Asn Thr Gln Val Glu Glu Val
   340 345 350
   Arg Val Tyr Asp Gly Thr Glu Pro Val Pro Gly Asp Pro Asp Met Thr
   355 360 365
   Arg Tyr Val Asp Arg Phe Gly Lys Thr Lys Thr Val Phe Pro Pro Gly
   370 375 380
<210> 3
   <211> 1152
   <212> DNA
   <213> Künstliche Sequenz
<220>
   <221> CDS
   <222> (1)..(1152)
<220>
   <223> Beschreibung der künstlichen Sequenz: Variation des Polyomavirus Hüllproteins VP1 (PyVP1-CallS-T249C)
<400> 3
   atg gcc ccc aaa aga aaa agc ggc gtc tct aaa agc gag aca aaa agc 48
   Met Ala Pro Lys Arg Lys Ser Gly Val Ser Lys Ser Glu Thr Lys Ser
   1 5 10 15
   aca aag gct agc cca aga ccc gca ccc gtt ccc aaa ctg ctt att aaa 96
   Thr Lys Ala Ser Pro Arg Pro Ala Pro Val Pro Lys Leu Leu Ile Lys
   20 25 30
   ggg ggt atg gag gtg ctg gac ctt gtg aca ggg cca gac agt gtg aca 144
   Gly Gly Met Glu Val Leu Asp Leu Val Thr Gly Pro Asp Ser Val Thr
   35 40 45
   gaa ata gaa gct ttt ctg aac ccc aga atg ggg cag cca ccc acc cct 192
   Glu Ile Glu Ala Phe Leu Asn Pro Arg Met Gly Gln Pro Pro Thr Pro
        50                  55                  60
   gaa agc cta aca gag gga ggg caa tac tat ggt tgg agc aga ggg att    240
   Glu Ser Leu Thr Glu Gly Gly Gln Tyr Tyr Gly Trp Ser Arg Gly Ile
    65                  70                  75                  80
   aat ttg gct aca tca gat aca gag gat tcc cca gga aat aat aca ctt    288
   Asn Leu Ala Thr Ser Asp Thr Glu Asp Ser Pro Gly Asn Asn Thr Leu
                    85                  90                  95
   ccc aca tgg agt atg gca aag ctc cag ctt ccc atg ctc aat gag gac    336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc    384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc    480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag    528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta    576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc    768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc    912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat    960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
       370                 375                 380
<210> 4
   <211> 384
   <212> PRT
   <213> Künstliche Sequenz
   <223> Beschreibung der künstlichen Sequenz: Variation des Polyomavirus Hüllproteins VP1 (PyVP1-CallS-T249C)
<400> 4
   Met Ala Pro Lys Arg Lys Ser Gly Val Ser Lys Ser Glu Thr Lys Ser
    1               5                  10                  15
   Thr Lys Ala Ser Pro Arg Pro Ala Pro Val Pro Lys Leu Leu Ile Lys
   20 25 30
   Gly Gly Met Glu Val Leu Asp Leu Val Thr Gly Pro Asp Ser Val Thr
   35 40 45
   Glu Ile Glu Ala Phe Leu Asn Pro Arg Met Gly Gln Pro Pro Thr Pro
   50 55 60
   Glu Ser Leu Thr Glu Gly Gly Gln Tyr Tyr Gly Trp Ser Arg Gly Ile
   65 70 75 80
   Asn Leu Ala Thr Ser Asp Thr Glu Asp Ser Pro Gly Asn Asn Thr Leu
   85 90 95
   Pro Thr Trp Ser Met Ala Lys Leu Gln Leu Pro Met Leu Asn Glu Asp
   100 105 110
   Leu Thr Ser Asp Thr Leu Gln Met Trp Glu Ala Val Ser Val Lys Thr
   115 120 125
   Glu Val Val Gly Ser Gly Ser Leu Leu Asp Val His Gly Phe Asn Lys
   130 135 140
   Pro Thr Asp Thr Val Asn Thr Lys Gly Ile Ser Thr Pro Val Glu Gly
   145 150 155 160
   Ser Gln Tyr His Val Phe Ala Val Gly Gly Glu Pro Leu Asp Leu Gln
   165 170 175
   Gly Leu Val Thr Asp Ala Arg Thr Lys Tyr Lys Glu Glu Gly Val Val
   180 185 190
   Thr Ile Lys Thr Ile Thr Lys Lys Asp Met Val Asn Lys Asp Gln Val
   195 200 205
   Leu Asn Pro Ile Ser Lys Ala Lys Leu Asp Lys Asp Gly Met Tyr Pro
   210 215 220
   Val Glu Ile Trp His Pro Asp Pro Ala Lys Asn Glu Asn Thr Arg Tyr
   225 230 235 240
   Phe Gly Asn Tyr Thr Gly Gly Thr Cys Thr Pro Pro Val Leu Gln Phe
   245 250 255
   Thr Asn Thr Leu Thr Thr Val Leu Leu Asp Glu Asn Gly Val Gly Pro
   260 265 270
   Leu Ser Lys Gly Glu Gly Leu Tyr Leu Ser Ser Val Asp Ile Met Gly
   275 280 285
   Trp Arg Val Thr Arg Asn Tyr Asp Val His His Trp Arg Gly Leu Pro
   290 295 300
   Arg Tyr Phe Lys Ile Thr Leu Arg Lys Arg Trp Val Lys Asn Pro Tyr
   305 310 315 320
   Pro Met Ala Ser Leu Ile Ser Ser Leu Phe Asn Asn Met Leu Pro Gln
   325 330 335
   Val Gln Gly Gln Pro Met Glu Gly Glu Asn Thr Gln Val Glu Glu Val
   340 345 350
   Arg Val Tyr Asp Gly Thr Glu Pro Val Pro Gly Asp Pro Asp Met Thr
   355 360 365
   Arg Tyr Val Asp Arg Phe Gly Lys Thr Lys Thr Val Phe Pro Pro Gly
   370 375 380
<210> 5
   <211> 1152
   <212> DNA
   <213> Künstliche Sequenz
<220>
   <221> CDS
   <222> (1)..(1152)
<220>
   <223> Beschreibung der künstlichen Sequenz: Variation des Polyomavirus Hüllproteins VP1 (PyVP1-2C)
<400> 5
   atg gcc ccc aaa aga aaa agc ggc gtc tct aaa agc gag aca aaa agc 48
   Met Ala Pro Lys Arg Lys Ser Gly Val Ser Lys Ser Glu Thr Lys Ser
   1 5 10 15
   aca aag gcc tgt cca aga ccc gca ccc gtt ccc aaa ctg ctt att aaa 96
   Thr Lys Ala Cys Pro Arg Pro Ala Pro Val Pro Lys Leu Leu Ile Lys
   20 25 30
   ggg ggt atg gag gtg ctg gac ctt gtg aca ggg cca gac agt gtg aca 144
   Gly Gly Met Glu Val Leu Asp Leu Val Thr Gly Pro Asp Ser Val Thr
   35 40 45
   gaa ata gaa gct ttt ctg aac ccc aga atg ggg cag cca ccc acc cct    192
   Glu Ile Glu Ala Phe Leu Asn Pro Arg Met Gly Gln Pro Pro Thr Pro
        50                  55                  60
   gaa agc cta aca gag gga ggg caa tac tat ggt tgg agc aga ggg att    240
   Glu Ser Leu Thr Glu Gly Gly Gln Tyr Tyr Gly Trp Ser Arg Gly Ile
    65                  70                  75                  80
   aat ttg gct aca tca gat aca gag gat tcc cca gga aat aat aca ctt    288
   Asn Leu Ala Thr Ser Asp Thr Glu Asp Ser Pro Gly Asn Asn Thr Leu
                    85                  90                  95
   ccc aca tgg agt atg gca aag ctc cag ctt ccc atg ctc aat gag gac    336
   Pro Thr Trp Ser Met Ala Lys Leu Gln Leu Pro Met Leu Asn Glu Asp
               100                 105                 110
   ctc acc tgt gac acc cta caa atg tgg gag gca gtc tca gtg aaa acc    384
   Leu Thr Cys Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc    480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag    528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta    576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc acg aca acc cca ccc gtc ctg cag ttc     768
   Phe Gly Asn Tyr Thr Gly Gly Thr Thr Thr Pro Pro Val Leu Gln Phe
                   245                 250                 255
   aca aac acc ctg aca act gtg ctc cta gat gaa aat gga gtt ggg ccc     816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc     864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc     912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat     960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
       370                 375                 380
<210> 6
   <211> 384
   <212> PRT
   <213> Künstliche Sequenz
   <223> Beschreibung der künstlichen Sequenz: Variation des Polyomavirus Hüllproteins VP1 (PyVP1-2C)
<400> 6
   Met Ala Pro Lys Arg Lys Ser Gly Val Ser Lys Ser Glu Thr Lys Ser
   1 5 10 15
   Thr Lys Ala Cys Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Cys Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Val Asn Thr Lys Gly Ile Ser Thr Pro Val Glu Gly
   145                 150                 155                 160
   Ser Gln Tyr His Val Phe Ala Val Gly Gly Glu Pro Leu Asp Leu Gln
                   165                 170                 175
   Gly Leu Val Thr Asp Ala Arg Thr Lys Tyr Lys Glu Glu Gly Val Val
               180                 185                 190
   Thr Ile Lys Thr Ile Thr Lys Lys Asp Met Val Asn Lys Asp Gln Val
           195                 200                 205
   Leu Asn Pro Ile Ser Lys Ala Lys Leu Asp Lys Asp Gly Met Tyr Pro
       210                 215                 220
   Val Glu Ile Trp His Pro Asp Pro Ala Lys Asn Glu Asn Thr Arg Tyr
   225                 230                 235                 240
   Phe Gly Asn Tyr Thr Gly Gly Thr Thr Thr Pro Pro Val Leu Gln Phe
                   245                 250                 255
   Thr Asn Thr Leu Thr Thr Val Leu Leu Asp Glu Asn Gly Val Gly Pro
               260                 265                 270
   Leu Ser Lys Gly Glu Gly Leu Tyr Leu Ser Ser Val Asp Ile Met Gly
           275                 280                 285
   Trp Arg Val Thr Arg Asn Tyr Asp Val His His Trp Arg Gly Leu Pro
       290                 295                 300
   Arg Tyr Phe Lys Ile Thr Leu Arg Lys Arg Trp Val Lys Asn Pro Tyr
   305                 310                 315                 320
   Pro Met Ala Ser Leu Ile Ser Ser Leu Phe Asn Asn Met Leu Pro Gln
                   325                 330                 335
   Val Gln Gly Gln Pro Met Glu Gly Glu Asn Thr Gln Val Glu Glu Val
               340                 345                 350
   Arg Val Tyr Asp Gly Thr Glu Pro Val Pro Gly Asp Pro Asp Met Thr
           355                 360                 365
   Arg Tyr Val Asp Arg Phe Gly Lys Thr Lys Thr Val Phe Pro Pro Gly
       370                 375                 380
<210> 7
   <211> 1152
   <212> DNA
   <213> Künstliche Sequenz
<220>
   <221> CDS
   <222> (1)..(1152)
<220>
   <223> Beschreibung der künstlichen Sequenz: Variation des Polyomavirus Hüllproteins VP1 (PyVP1-3C)
<400> 7
   atg gcc ccc aaa aga aaa agc ggc gtc tct aaa agc gag aca aaa agc      48
   Met Ala Pro Lys Arg Lys Ser Gly Val Ser Lys Ser Glu Thr Lys Ser
     1               5                  10                  15
   aca aag gcc tgt cca aga ccc gca ccc gtt ccc aaa ctg ctt att aaa      96
   Thr Lys Ala Cys Pro Arg Pro Ala Pro Val Pro Lys Leu Leu Ile Lys
                20                  25                  30
   ggg ggt atg gag gtg ctg gac ctt gtg aca ggg cca gac agt gtg aca     144
   Gly Gly Met Glu Val Leu Asp Leu Val Thr Gly Pro Asp Ser Val Thr
               35                  40                  45
   gaa ata gaa gct ttt ctg aac ccc aga atg ggg cag cca ccc acc cct    192
   Glu Ile Glu Ala Phe Leu Asn Pro Arg Met Gly Gln Pro Pro Thr Pro
           50                  55                  60
   gaa agc cta aca gag gga ggg caa tac tat ggt tgg agc aga ggg att    240
   Glu Ser Leu Thr Glu Gly Gly Gln Tyr Tyr Gly Trp Ser Arg Gly Ile
   65                  70                  75                  80
   aat ttg gct aca tca gat aca gag gat tcc cca gga aat aat aca ctt    288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac    336
   Pro Thr Trp Ser Met Ala Lys Leu Gln Leu Pro Met Leu Asn Glu Asp
               100                 105                 110
   ctc acc tgt gac acc cta caa atg tgg gag gca gtc tca gtg aaa acc    384
   Leu Thr Cys Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc    480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag    528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta    576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                     230                     235                     240
   ttt ggc aat tac act gga ggc acg tgc acc cca ccc gtc ctg cag ttc    768
   Phe Gly Asn Tyr Thr Gly Gly Thr Cys Thr Pro Pro Val Leu Gln Phe
                   245                     250                     255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                     265                     270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                     280                     285
   tgg aga gtt aca aga aac tat gat gtc cat cac tgg aga ggg ctt ccc    912
   Trp Arg Val Thr Arg Asn Tyr Asp Val His His Trp Arg Gly Leu Pro
       290                     295                     300
   aga tat ttc aaa atc acc ctg aga aaa aga tgg gtc aaa aat ccc tat    960
   Arg Tyr Phe Lys Ile Thr Leu Arg Lys Arg Trp Val Lys Asn Pro Tyr
   305                     310                     315                     320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                     330                     335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                     345                     350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                     360                     365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
       370                     375                     380
<210> 8
   <211> 384
   <212> PRT
   <213> Künstliche Sequenz
   <223> Beschreibung der künstlichen Sequenz: Variation des Polyomavirus Hüllproteins VP1 (PyVP1-3C)
<400> 8
   Met Ala Pro Lys Arg Lys Ser Gly Val Ser Lys Ser Glu Thr Lys Ser
   1               5                   10                  15
   Thr Lys Ala Cys Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Cys Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Val Asn Thr Lys Gly Ile Ser Thr Pro Val Glu Gly
   145                 150                 155                 160
   Ser Gln Tyr His Val Phe Ala Val Gly Gly Glu Pro Leu Asp Leu Gln
                   165                 170                 175
   Gly Leu Val Thr Asp Ala Arg Thr Lys Tyr Lys Glu Glu Gly Val Val
               180                 185                 190
   Thr Ile Lys Thr Ile Thr Lys Lys Asp Met Val Asn Lys Asp Gln Val
           195                 200                 205
   Leu Asn Pro Ile Ser Lys Ala Lys Leu Asp Lys Asp Gly Met Tyr Pro
       210                 215                 220
   Val Glu Ile Trp His Pro Asp Pro Ala Lys Asn Glu Asn Thr Arg Tyr
   225                 230                 235                 240
   Phe Gly Asn Tyr Thr Gly Gly Thr Cys Thr Pro Pro Val Leu Gln Phe
                   245                 250                 255
   Thr Asn Thr Leu Thr Thr Val Leu Leu Asp Glu Asn Gly Val Gly Pro
               260                 265                 270
   Leu Ser Lys Gly Glu Gly Leu Tyr Leu Ser Ser Val Asp Ile Met Gly
           275                 280                 285
   Trp Arg Val Thr Arg Asn Tyr Asp Val His His Trp Arg Gly Leu Pro
       290                 295                 300
   Arg Tyr Phe Lys Ile Thr Leu Arg Lys Arg Trp Val Lys Asn Pro Tyr
   305                 310                 315                 320
   Pro Met Ala Ser Leu Ile Ser Ser Leu Phe Asn Asn Met Leu Pro Gln
                   325                 330                 335
   Val Gln Gly Gln Pro Met Glu Gly Glu Asn Thr Gln Val Glu Glu Val
               340                 345                 350
   Arg Val Tyr Asp Gly Thr Glu Pro Val Pro Gly Asp Pro Asp Met Thr
           355                 360                 365
   Arg Tyr Val Asp Arg Phe Gly Lys Thr Lys Thr Val Phe Pro Pro Gly
       370                 375                 380
<210> 9
   <211> 1188
   <212> DNA
   <213> Künstliche Sequenz
<220>
   <221> CDS
   <222> (1)..(1188)
<220>
   <223> Beschreibung der künstlichen Sequenz: Variation des Polyomavirus Hüllproteins VP1 (PyVP1-RGD148)
<220>
   <221> misc_feature
   <222> (469)..(477)
   <223> RGD-haltiges Peptid inseriert
<400> 9
   atg gcc ccc aaa aga aaa agc ggc gtc tct aaa agc gag aca aaa agc    48
   Met Ala Pro Lys Arg Lys Ser Gly Val Ser Lys Ser Glu Thr Lys Ser
   1               5                   10                  15
   aca aag gct agc cca aga ccc gca ccc gtt ccc aaa ctg ctt att aaa    96
   Thr Lys Ala Ser Pro Arg Pro Ala Pro Val Pro Lys Leu Leu Ile Lys
               20                  25                  30
   ggg ggt atg gag gtg ctg gac ctt gtg aca ggg cca gac agt gtg aca   144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct   192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc aga ggg att   240
   Glu Ser Leu Thr Glu Gly Gly Gln Tyr Tyr Gly Trp Ser Arg Gly Ile
   65                  70                  75                  80
   aat ttg gct aca tca gat aca gag gat tcc cca gga aat aat aca ctt   288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac   336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc   384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa   432
   Glu Val Val Gly Ser Gly Ser Leu Leu Asp Val His Gly Phe Asn Lys
       130                 135                 140
   ccc aca gat aca gta aac ggc agc ggc agc ggc agc aga ggc gac agc   480
   Pro Thr Asp Thr Val Asn Gly Ser Gly Ser Gly Ser Arg Gly Asp Ser
   145                 150                 155                 160
   ggc agt gca aaa gga att tcc act cca gtg gaa ggc agc caa tat cat   528
   Gly Ser Ala Lys Gly Ile Ser Thr Pro Val Glu Gly Ser Gln Tyr His
                   165                 170                 175
   gtg ttt gct gtg ggc ggg gaa ccg ctt gac ctc cag gga ctt gtg aca   576
   Val Phe Ala Val Gly Gly Glu Pro Leu Asp Leu Gln Gly Leu Val Thr
               180                 185                 190
   gat gcc aga aca aaa tac aag gaa gaa ggg gta gta aca atc aaa aca   624
   Asp Ala Arg Thr Lys Tyr Lys Glu Glu Gly Val Val Thr Ile Lys Thr
           195                 200                 205
   atc aca aag aag gac atg gtc aac aaa gac caa gtc ctg aat cca att    672
   Ile Thr Lys Lys Asp Met Val Asn Lys Asp Gln Val Leu Asn Pro Ile
       210                 215                 220
   agc aag gcc aag ctg gat aag gac gga atg tat cca gtt gaa atc tgg    720
   Ser Lys Ala Lys Leu Asp Lys Asp Gly Met Tyr Pro Val Glu Ile Trp
   225                 230                 235                 240
   cat cca gat cca gca aaa aat gag aac aca agg tac ttt ggc aat tac    768
   His Pro Asp Pro Ala Lys Asn Glu Asn Thr Arg Tyr Phe Gly Asn Tyr
                   245                 250                 255
   act gga ggc acg tgc acc cca ccc gtc ctg cag ttc aca aac acc ctg    816
   Thr Gly Gly Thr Cys Thr Pro Pro Val Leu Gln Phe Thr Asn Thr Leu
               260                 265                 270
   aca act gtg ctc cta gat gaa aat gga gtt ggg ccc ctc agc aaa gga    864
   Thr Thr Val Leu Leu Asp Glu Asn Gly Val Gly Pro Leu Ser Lys Gly
           275                 280                 285
   gaa ggt cta tac ctc tcg agc gta gat ata atg ggc tgg aga gtt aca    912
   Glu Gly Leu Tyr Leu Ser Ser Val Asp Ile Met Gly Trp Arg Val Thr
       290                 295                 300
   aga aac tat gat gtc cat cac tgg aga ggg ctt ccc aga tat ttc aaa    960
   Arg Asn Tyr Asp Val His His Trp Arg Gly Leu Pro Arg Tyr Phe Lys
   305                 310                 315                 320
   atc acc ctg aga aaa aga tgg gtc aaa aat ccc tat ccc atg gcc tcc    1008
   Ile Thr Leu Arg Lys Arg Trp Val Lys Asn Pro Tyr Pro Met Ala Ser
                   325                 330                 335
   ctc ata agt tcc ctt ttc aac aac atg ctc ccc caa gtg cag ggc caa    1056
   Leu Ile Ser Ser Leu Phe Asn Asn Met Leu Pro Gln Val Gln Gly Gln
               340                 345                 350
   ccc atg gaa ggg gag aac acc cag gta gag gag gtt aga gtg tat gat    1104
   Pro Met Glu Gly Glu Asn Thr Gln Val Glu Glu Val Arg Val Tyr Asp
           355                 360                 365
   ggg act gaa cct gta ccg ggg gac cct gat atg acg cgc tat gtt gac    1152
   Gly Thr Glu Pro Val Pro Gly Asp Pro Asp Met Thr Arg Tyr Val Asp
       370                 375                 380
   cgc ttt gga aaa aca aag act gta ttt cct ccc ggg                    1188
   Arg Phe Gly Lys Thr Lys Thr Val Phe Pro Pro Gly
   385                 390                 395
<210> 10
   <211> 396
   <212> PRT
   <213> Künstliche Sequenz
<223> Beschreibung der künstlichen Sequenz: Variation des Polyomavirus Hüllproteins VP1 (PyVP1-RGD148)
<400> 10
   Met Ala Pro Lys Arg Lys Ser Gly Val Ser Lys Ser Glu Thr Lys Ser
   1 5 10 15
   Thr Lys Ala Ser Pro Arg Pro Ala Pro Val Pro Lys Leu Leu Ile Lys
   20 25 30
   Gly Gly Met Glu Val Leu Asp Leu Val Thr Gly Pro Asp Ser Val Thr
   35 40 45
   Glu Ile Glu Ala Phe Leu Asn Pro Arg Met Gly Gln Pro Pro Thr Pro
   50 55 60
   Glu Ser Leu Thr Glu Gly Gly Gln Tyr Tyr Gly Trp Ser Arg Gly Ile
   65 70 75 80
   Asn Leu Ala Thr Ser Asp Thr Glu Asp Ser Pro Gly Asn Asn Thr Leu
   85 90 95
   Pro Thr Trp Ser Met Ala Lys Leu Gln Leu Pro Met Leu Asn Glu Asp
   100 105 110
   Leu Thr Ser Asp Thr Leu Gln Met Trp Glu Ala Val Ser Val Lys Thr
   115 120 125
   Glu Val Val Gly Ser Gly Ser Leu Leu Asp Val His Gly Phe Asn Lys
   130 135 140
   Pro Thr Asp Thr Val Asn Gly Ser Gly Ser Gly Ser Arg Gly Asp Ser
   145 150 155 160
   Gly Ser Ala Lys Gly Ile Ser Thr Pro Val Glu Gly Ser Gln Tyr His
   165 170 175
   Val Phe Ala Val Gly Gly Glu Pro Leu Asp Leu Gln Gly Leu Val Thr
   180 185 190
   Asp Ala Arg Thr Lys Tyr Lys Glu Glu Gly Val Val Thr Ile Lys Thr
   195 200 205
   Ile Thr Lys Lys Asp Met Val Asn Lys Asp Gln Val Leu Asn Pro Ile
       210                 215                 220
   Ser Lys Ala Lys Leu Asp Lys Asp Gly Met Tyr Pro Val Glu Ile Trp
   225                 230                 235                 240
   His Pro Asp Pro Ala Lys Asn Glu Asn Thr Arg Tyr Phe Gly Asn Tyr
                   245                 250                 255
   Thr Gly Gly Thr Cys Thr Pro Pro Val Leu Gln Phe Thr Asn Thr Leu
               260                 265                 270
   Thr Thr Val Leu Leu Asp Glu Asn Gly Val Gly Pro Leu Ser Lys Gly
           275                 280                 285
   Glu Gly Leu Tyr Leu Ser Ser Val Asp Ile Met Gly Trp Arg Val Thr
       290                 295                 300
   Arg Asn Tyr Asp Val His His Trp Arg Gly Leu Pro Arg Tyr Phe Lys
   305                 310                 315                 320
   Ile Thr Leu Arg Lys Arg Trp Val Lys Asn Pro Tyr Pro Met Ala Ser
                   325                 330                 335
   Leu Ile Ser Ser Leu Phe Asn Asn Met Leu Pro Gln Val Gln Gly Gln
               340                 345                 350
   Pro Met Glu Gly Glu Asn Thr Gln Val Glu Glu Val Arg Val Tyr Asp
           355                 360                 365
   Gly Thr Glu Pro Val Pro Gly Asp Pro Asp Met Thr Arg Tyr Val Asp
       370                 375                 380
   Arg Phe Gly Lys Thr Lys Thr Val Phe Pro Pro Gly
   385                 390                 395
<210> 11
   <211> 1200
   <212> DNA
   <213> Künstliche Sequenz
<220>
   <221> CDS
   <222> (1)..(1200)
<220>
   <223> Beschreibung der künstlichen Sequenz: Variation des Polyomavirus Hüllproteins VP1 (PyVP1-RGD293)
<220>
   <221> misc_feature
   <222> (898)..(906)
   <223> RGD-haltiges Peptid inseriert
<400> 11
   atg gcc ccc aaa aga aaa agc ggc gtc tct aaa agc gag aca aaa agc      48
   Met Ala Pro Lys Arg Lys Ser Gly Val Ser Lys Ser Glu Thr Lys Ser
   1               5                   10                  15
   aca aag gct agc cca aga ccc gca ccc gtt ccc aaa ctg ctt att aaa      96
   Thr Lys Ala Ser Pro Arg Pro Ala Pro Val Pro Lys Leu Leu Ile Lys
               20                  25                  30
   ggg ggt atg gag gtg ctg gac ctt gtg aca ggg cca gac agt gtg aca     144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct     192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc aga ggg att     240
   Glu Ser Leu Thr Glu Gly Gly Gln Tyr Tyr Gly Trp Ser Arg Gly Ile
   65                  70                  75                  80
   aat ttg gct aca tca gat aca gag gat tcc cca gga aat aat aca ctt     288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac     336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc     384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa     432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc     480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag    528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta    576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc    768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                 280                 285
   tgg aga gtt acc ggc agc ggc agc ggc agc ggt cgt ggc gat agc ggc    912
   Trp Arg Val Thr Gly Ser Gly Ser Gly Ser Gly Arg Gly Asp Ser Gly
       290                 295                 300
   agc ggc agc ggc agt ggc tat gat gtc cat cac tgg aga ggg ctt ccc    960
   Ser Gly Ser Gly Ser Gly Tyr Asp Val His His Trp Arg Gly Leu Pro
   305                 310                 315                 320
   aga tat ttc aaa atc acc ctg aga aaa aga tgg gtc aaa aat ccc tat    1008
   Arg Tyr Phe Lys Ile Thr Leu Arg Lys Arg Trp Val Lys Asn Pro Tyr
                   325                 330                 335
   ccc atg gcc tcc ctc ata agt tcc ctt ttc aac aac atg ctc ccc caa    1056
   Pro Met Ala Ser Leu Ile Ser Ser Leu Phe Asn Asn Met Leu Pro Gln
               340                 345                 350
   gtg cag ggc caa ccc atg gaa ggg gag aac acc cag gta gag gag gtt    1104
   Val Gln Gly Gln Pro Met Glu Gly Glu Asn Thr Gln Val Glu Glu Val
           355                 360                 365
   aga gtg tat gat ggg act gaa cct gta ccg ggg gac cct gat atg acg    1152
   Arg Val Tyr Asp Gly Thr Glu Pro Val Pro Gly Asp Pro Asp Met Thr
       370                 375                 380
   cgc tat gtt gac cgc ttt gga aaa aca aag act gta ttt cct ccc ggg    1200
   Arg Tyr Val Asp Arg Phe Gly Lys Thr Lys Thr Val Phe Pro Pro Gly
   385                 390                 395                 400
<210> 12
   <211> 400
   <212> PRT
   <213> Künstliche Sequenz
   <223> Beschreibung der künstlichen Sequenz: Variation des Polyomavirus Hüllproteins VP1 (PyVP1-RGD293)
<400> 12
   Met Ala Pro Lys Arg Lys Ser Gly Val Ser Lys Ser Glu Thr Lys Ser
   1               5                   10                  15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Ser Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Val Asn Thr Lys Gly Ile Ser Thr Pro Val Glu Gly
   145 150 155 160
   Ser Gln Tyr His Val Phe Ala Val Gly Gly Glu Pro Leu Asp Leu Gln
   165 170 175
   Gly Leu Val Thr Asp Ala Arg Thr Lys Tyr Lys Glu Glu Gly Val Val
   180 185 190
   Thr Ile Lys Thr Ile Thr Lys Lys Asp Met Val Asn Lys Asp Gln Val
   195 200 205
   Leu Asn Pro Ile Ser Lys Ala Lys Leu Asp Lys Asp Gly Met Tyr Pro
   210 215 220
   Val Glu Ile Trp His Pro Asp Pro Ala Lys Asn Glu Asn Thr Arg Tyr
   225 230 235 240
   Phe Gly Asn Tyr Thr Gly Gly Thr Cys Thr Pro Pro Val Leu Gln Phe
   245 250 255
   Thr Asn Thr Leu Thr Thr Val Leu Leu Asp Glu Asn Gly Val Gly Pro
   260 265 270
   Leu Ser Lys Gly Glu Gly Leu Tyr Leu Ser Ser Val Asp Ile Met Gly
   275 280 285
   Trp Arg Val Thr Gly Ser Gly Ser Gly Ser Gly Arg Gly Asp Ser Gly
   290 295 300
   Ser Gly Ser Gly Ser Gly Tyr Asp Val His His Trp Arg Gly Leu Pro
   305 310 315 320
   Arg Tyr Phe Lys Ile Thr Leu Arg Lys Arg Trp Val Lys Asn Pro Tyr
   325 330 335
   Pro Met Ala Ser Leu Ile Ser Ser Leu Phe Asn Asn Met Leu Pro Gln
   340 345 350
   Val Gln Gly Gln Pro Met Glu Gly Glu Asn Thr Gln Val Glu Glu Val
   355 360 365
   Arg Val Tyr Asp Gly Thr Glu Pro Val Pro Gly Asp Pro Asp Met Thr
   370 375 380
   Arg Tyr Val Asp Arg Phe Gly Lys Thr Lys Thr Val Phe Pro Pro Gly
   385 390 395 400
<210> 13
   <211> 1152
   <212> DNA
   <213> Künstliche Sequenz
<220>
   <221> CDS
   <222> (1)..(1152)
<220>
   <223> Beschreibung der künstlichen Sequenz: Variation des Polyomavirus Hüllproteins VP1 (PyVP1-R78W)
<220>
   <221> misc_feature
   <222> (232)..(234)
   <223> Austausch an einer Rezeptorbindungsstelle
<400> 13
   atg gcc ccc aaa aga aaa agc ggc gtc tct aaa agc gag aca aaa agc   48
   Met Ala Pro Lys Arg Lys Ser Gly Val Ser Lys Ser Glu Thr Lys Ser
   1               5                   10                  15
   aca aag gct tgt cca aga ccc gca ccc gtt ccc aaa ctg ctt att aaa   96
   Thr Lys Ala Cys Pro Arg Pro Ala Pro Val Pro Lys Leu Leu Ile Lys
               20                  25                  30
   ggg ggt atg gag gtg ctg gac ctt gtg aca ggg cca gac agt gtg aca   144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct   192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc tgg ggg att   240
   Glu Ser Leu Thr Glu Gly Gly Gln Tyr Tyr Gly Trp Ser Trp Gly Ile
   65                  70                  75                  80
   aat ttg gct aca tca gat aca gag gat tcc cca gga aat aat aca ctt   288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac   336
   Pro Thr Trp Ser Met Ala Lys Leu Gln Leu Pro Met Leu Asn Glu Asp
               100                 105                 110
   ctc acg tgt gac acc cta caa atg tgg gag gca gtc tca gtg aaa acc    384
   Leu Thr Cys Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc    480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag    528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta    576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc    768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                 280                 285
   tgg aga gtt aca aga aac tat gat gtc cat cac tgg aga ggg ctt ccc    912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat    960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
       370                 375                 380
<210> 14
   <211> 384
   <212> PRT
   <213> Künstliche Sequenz
   <223> Beschreibung der künstlichen Sequenz: Variation des Polyomavirus Hüllproteins VP1 (PyVP1-R78W)
<400> 14
   Met Ala Pro Lys Arg Lys Ser Gly Val Ser Lys Ser Glu Thr Lys Ser
    1               5                   10                  15
   Thr Lys Ala Cys Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Trp Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Cys Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Val Asn Thr Lys Gly Ile Ser Thr Pro Val Glu Gly
   145                 150                 155                 160
   Ser Gln Tyr His Val Phe Ala Val Gly Gly Glu Pro Leu Asp Leu Gln
                   165                 170                 175
   Gly Leu Val Thr Asp Ala Arg Thr Lys Tyr Lys Glu Glu Gly Val Val
               180                 185                 190
   Thr Ile Lys Thr Ile Thr Lys Lys Asp Met Val Asn Lys Asp Gln Val
           195                 200                 205
   Leu Asn Pro Ile Ser Lys Ala Lys Leu Asp Lys Asp Gly Met Tyr Pro
       210                 215                 220
   Val Glu Ile Trp His Pro Asp Pro Ala Lys Asn Glu Asn Thr Arg Tyr
   225                 230                 235                 240
   Phe Gly Asn Tyr Thr Gly Gly Thr Cys Thr Pro Pro Val Leu Gln Phe
                   245                 250                 255
   Thr Asn Thr Leu Thr Thr Val Leu Leu Asp Glu Asn Gly Val Gly Pro
               260                 265                 270
   Leu Ser Lys Gly Glu Gly Leu Tyr Leu Ser Ser Val Asp Ile Met Gly
           275                 280                 285
   Trp Arg Val Thr Arg Asn Tyr Asp Val His His Trp Arg Gly Leu Pro
       290                 295                 300
   Arg Tyr Phe Lys Ile Thr Leu Arg Lys Arg Trp Val Lys Asn Pro Tyr
   305                 310                 315                 320
   Pro Met Ala Ser Leu Ile Ser Ser Leu Phe Asn Asn Met Leu Pro Gln
                   325                 330                 335
   Val Gln Gly Gln Pro Met Glu Gly Glu Asn Thr Gln Val Glu Glu Val
               340                 345                 350
   Arg Val Tyr Asp Gly Thr Glu Pro Val Pro Gly Asp Pro Asp Met Thr
           355                 360                 365
   Arg Tyr Val Asp Arg Phe Gly Lys Thr Lys Thr Val Phe Pro Pro Gly
       370                 375                 380
<210> 15
   <211> 1263
   <212> DNA
   <213> Künstliche Sequenz
<220>
   <221> CDS
   <222> (1)..(1263)
<220>
   <223> Beschreibung der künstlichen Sequenz: Variation des Polyomavirus Hüllproteins VP1 (PyVP1-Def)
<220>
   <221> misc_feature
   <222> (868)..(981)
   <223> Insertion einer Peptidsequenz
<400> 15
   atg gcc ccc aaa aga aaa agc ggc gtc tct aaa agc gag aca aaa agc    48
   Met Ala Pro Lys Arg Lys Ser Gly Val Ser Lys Ser Glu Thr Lys Ser
   1               5                   10                  15
   aca aag gct agc cca aga ccc gca ccc gtt ccc aaa ctg ctt att aaa    96
   Thr Lys Ala Ser Pro Arg Pro Ala Pro Val Pro Lys Leu Leu Ile Lys
               20                  25                  30
   ggg ggt atg gag gtg ctg gac ctt gtg aca ggg cca gac agt gtg aca    144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct    192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc aga ggg att    240
   Glu Ser Leu Thr Glu Gly Gly Gln Tyr Tyr Gly Trp Ser Arg Gly Ile
   65                  70                  75                  80
   aat ttg gct aca tca gat aca gag gat tcc cca gga aat aat aca ctt      288
   Asn Leu Ala Thr Ser Asp Thr Glu Asp Ser Pro Gly Asn Asn Thr Leu
                       85                  90                  95
   ccc aca tgg agt atg gca aag ctc cag ctt ccc atg ctc aat gag gac      336
   Pro Thr Trp Ser Met Ala Lys Leu Gln Leu Pro Met Leu Asn Glu Asp
                   100                 105                 110
   ctc acg tct gac acc cta caa atg tgg gag gca gtc tca gtg aaa acc      384
   Leu Thr Ser Asp Thr Leu Gln Met Trp Glu Ala Val Ser Val Lys Thr
               115                 120                 125
   gag gtg gtg ggc tct ggc tca ctg tta gat gtg cat ggg ttc aac aaa      432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc      480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag      528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta      576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc      624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca      672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac      720
   Val Glu Ile Trp His Pro Asp Pro Ala Lys Asn Glu Asn Thr Arg Tyr
   225                 230                 235                 240
   ttt ggc aat tac act gga ggc acg tgc acc cca ccc gtc ctg cag ttc      768
   Phe Gly Asn Tyr Thr Gly Gly Thr Cys Thr Pro Pro Val Leu Gln Phe
                   245                 250                 255
   aca aac acc ctg aca act gtg ctc cta gat gaa aat gga gtt ggg ccc      816
   Thr Asn Thr Leu Thr Thr Val Leu Leu Asp Glu Asn Gly Val Gly Pro
               260                 265                 270
   ctc agc aaa gga gaa ggt cta tac ctc tcg agc gta gat ata atg ggc    864
   Leu Ser Lys Gly Glu Gly Leu Tyr Leu Ser Ser Val Asp Ile Met Gly
           275                 280                 285
   tgg ggc agc ggc agc ggc tgg aca gaa cat aaa tca cct gat gga agg    912
   Trp Gly Ser Gly Ser Gly Trp Thr Glu His Lys Ser Pro Asp Gly Arg
       290                 295                 300
   act tat tat tac aat act gaa aca aaa cag tct acc tgg gaa aag cca    960
   Thr Tyr Tyr Tyr Asn Thr Glu Thr Lys Gln Ser Thr Trp Glu Lys Pro
   305                 310                 315                 320
   gat gat ggt agt ggt agc ggc gtt aca aga aac tat gat gtc cat cac    1008
   Asp Asp Gly Ser Gly Ser Gly Val Thr Arg Asn Tyr Asp Val His His
                   325                 330                 335
   tgg aga ggg ctt ccc aga tat ttc aaa atc acc ctg aga aaa aga tgg    1056
   Trp Arg Gly Leu Pro Arg Tyr Phe Lys Ile Thr Leu Arg Lys Arg Trp
               340                 345                 350
   gtc aaa aat ccc tat ccc atg gcc tcc ctc ata agt tcc ctt ttc aac    1104
   Val Lys Asn Pro Tyr Pro Met Ala Ser Leu Ile Ser Ser Leu Phe Asn
           355                 360                 365
   aac atg ctc ccc caa gtg cag ggc caa ccc atg gaa ggg gag aac acc    1152
   Asn Met Leu Pro Gln Val Gln Gly Gln Pro Met Glu Gly Glu Asn Thr
       370                 375                 380
   cag gta gag gag gtt aga gtg tat gat ggg act gaa cct gta ccg ggg    1200
   Gln Val Glu Glu Val Arg Val Tyr Asp Gly Thr Glu Pro Val Pro Gly
   385                 390                 395                 400
   gac cct gat atg acg cgc tat gtt gac cgc ttt gga aaa aca aag act    1248
   Asp Pro Asp Met Thr Arg Tyr Val Asp Arg Phe Gly Lys Thr Lys Thr
                   405                 410                 415
   gta ttt cct ccc ggg                                                1263
   Val Phe Pro Pro Gly
               420
<210> 16
   <211> 421
   <212> PRT
   <213> Künstliche Sequenz
   <223> Beschreibung der künstlichen Sequenz: Variation des Polyomavirus Hüllproteins VP1 (PyVP1-Def)
<400> 16
   Met Ala Pro Lys Arg Lys Ser Gly Val Ser Lys Ser Glu Thr Lys Ser
    1               5                  10                  15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Ser Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Val Asn Thr Lys Gly Ile Ser Thr Pro Val Glu Gly
   145                 150                 155                 160
   Ser Gln Tyr His Val Phe Ala Val Gly Gly Glu Pro Leu Asp Leu Gln
                   165                 170                 175
   Gly Leu Val Thr Asp Ala Arg Thr Lys Tyr Lys Glu Glu Gly Val Val
               180                 185                 190
   Thr Ile Lys Thr Ile Thr Lys Lys Asp Met Val Asn Lys Asp Gln Val
           195                 200                 205
   Leu Asn Pro Ile Ser Lys Ala Lys Leu Asp Lys Asp Gly Met Tyr Pro
       210                 215                 220
   Val Glu Ile Trp His Pro Asp Pro Ala Lys Asn Glu Asn Thr Arg Tyr
   225                 230                 235                 240
   Phe Gly Asn Tyr Thr Gly Gly Thr Cys Thr Pro Pro Val Leu Gln Phe
                   245                 250                 255
   Thr Asn Thr Leu Thr Thr Val Leu Leu Asp Glu Asn Gly Val Gly Pro
   260 265 270
   Leu Ser Lys Gly Glu Gly Leu Tyr Leu Ser Ser Val Asp Ile Met Gly
   275 280 285
   Trp Gly Ser Gly Ser Gly Trp Thr Glu His Lys Ser Pro Asp Gly Arg
   290 295 300
   Thr Tyr Tyr Tyr Asn Thr Glu Thr Lys Gln Ser Thr Trp Glu Lys Pro
   305 310 315 320
   Asp Asp Gly Ser Gly Ser Gly Val Thr Arg Asn Tyr Asp Val His His
   325 330 335
   Trp Arg Gly Leu Pro Arg Tyr Phe Lys Ile Thr Leu Arg Lys Arg Trp
   340 345 350
   Val Lys Asn Pro Tyr Pro Met Ala Ser Leu Ile Ser Ser Leu Phe Asn
   355 360 365
   Asn Met Leu Pro Gln Val Gln Gly Gln Pro Met Glu Gly Glu Asn Thr
   370 375 380
   Gln Val Glu Glu Val Arg Val Tyr Asp Gly Thr Glu Pro Val Pro Gly
   385 390 395 400
   Asp Pro Asp Met Thr Arg Tyr Val Asp Arg Phe Gly Lys Thr Lys Thr
   405 410 415
   Val Phe Pro Pro Gly
   420
<210> 17
   <211> 1581
   <212> DNA
   <213> Künstliche Sequenz
<220>
   <221> CDS
   <222> (1)..(1581)
<220>
   <223> Beschreibung der künstlichen Sequenz: Variation des Listeriolysin O (LLO)
<220>
   <221> misc_feature
   <222> (1)..(63)
   <223> Anfügen einer prolinreichen Sequenz
<400> 17
   atg ccg cca cct cca ccg cca cct ccg tta cca ggc cta ggc cgg cgt    48
   Met Pro Pro Pro Pro Pro Pro Pro Pro Leu Pro Gly Leu Gly Arg Arg
    1               5                  10                  15
   ggg cta gcg acg tcc gat gca tct gca ttc aat aaa gaa aat tta att    96
   Gly Leu Ala Thr Ser Asp Ala Ser Ala Phe Asn Lys Glu Asn Leu Ile
               20                  25                  30
   tca tcc atg gca cca cca gca tct ccg cct gca agt cct aag acg cca   144
   Ser Ser Met Ala Pro Pro Ala Ser Pro Pro Ala Ser Pro Lys Thr Pro
           35                  40                  45
   atc gaa aag aaa cac gcg gat gaa atc gat aag tat ata caa gga ttg   192
   Ile Glu Lys Lys His Ala Asp Glu Ile Asp Lys Tyr Ile Gln Gly Leu
       50                  55                  60
   gat tac aat aaa aac aat gta tta gta tac cac gga gat gca gtg aca   240
   Asp Tyr Asn Lys Asn Asn Val Leu Val Tyr His Gly Asp Ala Val Thr
   65                  70                  75                  80
   aat gtg ccg cca aga aaa ggt tat aaa gat gga aat gaa tat atc gtt   288
   Asn Val Pro Pro Arg Lys Gly Tyr Lys Asp Gly Asn Glu Tyr Ile Val
                   85                  90                  95
   gtg gag aaa aag aag aaa tcc atc aat caa aat aat gca gat atc caa   336
   Val Glu Lys Lys Lys Lys Ser Ile Asn Gln Asn Asn Ala Asp Ile Gln
               100                 105                 110
   gtt gtg aat gca att tcg agc cta aca tat cca ggt gct ctc gtg aaa   384
   Val Val Asn Ala Ile Ser Ser Leu Thr Tyr Pro Gly Ala Leu Val Lys
           115                 120                 125
   gcg aat tcg gaa tta gta gaa aat caa ccc gat gtt ctt cct gtc aaa   432
   Ala Asn Ser Glu Leu Val Glu Asn Gln Pro Asp Val Leu Pro Val Lys
       130                 135                 140
   cgt gat tca tta aca ctt agc att gat ttg cca gga atg act aat caa   480
   Arg Asp Ser Leu Thr Leu Ser Ile Asp Leu Pro Gly Met Thr Asn Gln
   145                 150                 155                 160
   gac aat aaa att gtt gta aaa aat gct act aaa tcg aac gtt aac aac   528
   Asp Asn Lys Ile Val Val Lys Asn Ala Thr Lys Ser Asn Val Asn Asn
                   165                 170                 175
   gca gta aat aca tta gtg gaa aga tgg aat gaa aaa tat gct caa gct    576
   Ala Val Asn Thr Leu Val Glu Arg Trp Asn Glu Lys Tyr Ala Gln Ala
               180                 185                 190
   tat cca aat gta agt gca aaa att gat tat gat gac gaa atg gct tac    624
   Tyr Pro Asn Val Ser Ala Lys Ile Asp Tyr Asp Asp Glu Met Ala Tyr
           195                 200                 205
   agt gaa tcg caa tta att gca aaa ttt ggt acg gca ttt aaa gct gta    672
   Ser Glu Ser Gln Leu Ile Ala Lys Phe Gly Thr Ala Phe Lys Ala Val
       210                 215                 220
   aat aat agc ttg aat gta aac ttc ggc gca atc agt gaa ggg aaa atg    720
   Asn Asn Ser Leu Asn Val Asn Phe Gly Ala Ile Ser Glu Gly Lys Met
   225                 230                 235                 240
   caa gaa gaa gtc att agt ttt aaa caa att tac tat aac gtg aat gtt    768
   Gln Glu Glu Val Ile Ser Phe Lys Gln Ile Tyr Tyr Asn Val Asn Val
                   245                 250                 255
   aat gaa cct aca aga cct tcc aga ttt ttc ggc aaa gct gtt act aaa    816
   Asn Glu Pro Thr Arg Pro Ser Arg Phe Phe Gly Lys Ala Val Thr Lys
               260                 265                 270
   gag cag ttg caa gcg ctt gga gtg aat gca gaa aat cct cct gca tat    864
   Glu Gln Leu Gln Ala Leu Gly Val Asn Ala Glu Asn Pro Pro Ala Tyr
           275                 280                 285
   atc tca agt gtg gca tat ggc cgt caa gtt tat ttg aaa tta tca act    912
   Ile Ser Ser Val Ala Tyr Gly Arg Gln Val Tyr Leu Lys Leu Ser Thr
       290                 295                 300
   aat tcc cat agt acc aaa gta aaa gct gct ttt gac gct gcc gta agt    960
   Asn Ser His Ser Thr Lys Val Lys Ala Ala Phe Asp Ala Ala Val Ser
   305                 310                 315                 320
   ggg aaa tct gtc tca ggt gat gta gaa ctg aca aat atc atc aaa aat    1008
   Gly Lys Ser Val Ser Gly Asp Val Glu Leu Thr Asn Ile Ile Lys Asn
                   325                 330                 335
   tct tcc ttc aaa gcc gta att tac ggt ggc tcc gca aaa gat gaa gtt    1056
   Ser Ser Phe Lys Ala Val Ile Tyr Gly Gly Ser Ala Lys Asp Glu Val
               340                 345                 350
   caa atc atc gac ggt aac ctc gga gac tta cga gat att ttg aaa aaa    1104
   Gln Ile Ile Asp Gly Asn Leu Gly Asp Leu Arg Asp Ile Leu Lys Lys
           355                 360                 365
   ggt gct act ttt aac cgg gaa aca cca gga gtt ccc att gcc tat aca      1152
   Gly Ala Thr Phe Asn Arg Glu Thr Pro Gly Val Pro Ile Ala Tyr Thr
       370                 375                 380
   aca aac ttc tta aaa gac aat gaa tta gct gtt att aaa aac aac tca      1200
   Thr Asn Phe Leu Lys Asp Asn Glu Leu Ala Val Ile Lys Asn Asn Ser
   385                 390                 395                 400
   gaa tat att gaa aca act tca aaa gct tat aca gat gga aaa atc aac      1248
   Glu Tyr Ile Glu Thr Thr Ser Lys Ala Tyr Thr Asp Gly Lys Ile Asn
                   405                 410                 415
   atc gat cac tct gga gga tac gtt gct caa ttc aac atc tct tgg gat      1296
   Ile Asp His Ser Gly Gly Tyr Val Ala Gln Phe Asn Ile Ser Trp Asp
               420                 425                 430
   gaa ata aat tat gat cct gaa ggt aac gaa att gtt caa cat aaa aac      1344
   Glu Ile Asn Tyr Asp Pro Glu Gly Asn Glu Ile Val Gln His Lys Asn
           435                 440                 445
   tgg agc gaa aac aat aaa agt aag cta gct cat ttc aca tcg tcc atc      1392
   Trp Ser Glu Asn Asn Lys Ser Lys Leu Ala His Phe Thr Ser Ser Ile
       450                 455                 460
   tat ttg cca ggt aac gca aga aat att aat gtt tac gct aaa gaa tgc      1440
   Tyr Leu Pro Gly Asn Ala Arg Asn Ile Asn Val Tyr Ala Lys Glu Cys
   465                 470                 475                 480
   act ggt tta gct tgg gaa tgg tgg aga acg gta att gat gac cgg aac      1488
   Thr Gly Leu Ala Trp Glu Trp Trp Arg Thr Val Ile Asp Asp Arg Asn
                   485                 490                 495
   cta ccg ctt gtg aaa aat aga aat atc tcc atc tgg ggc act aca ctt      1536
   Leu Pro Leu Val Lys Asn Arg Asn Ile Ser Ile Trp Gly Thr Thr Leu
               500                 505                 510
   tat ccg aaa tat agt aat agt gta gat aat cca atc gaa ccc ggg          1581
   Tyr Pro Lys Tyr Ser Asn Ser Val Asp Asn Pro Ile Glu Pro Gly
           515                 520                 525
<210> 18
   <211> 527
   <212> PRT
   <213> Künstliche Sequenz
   <223> Beschreibung der künstlichen Sequenz: Variation des Listeriolysin O (LLO)
<400> 18
   Met Pro Pro Pro Pro Pro Pro Pro Pro Leu Pro Gly Leu Gly Arg Arg
   1 5 10 15
   Gly Leu Ala Thr Ser Asp Ala Ser Ala Phe Asn Lys Glu Asn Leu Ile
   20 25 30
   Ser Ser Met Ala Pro Pro Ala Ser Pro Pro Ala Ser Pro Lys Thr Pro
   35 40 45
   Ile Glu Lys Lys His Ala Asp Glu Ile Asp Lys Tyr Ile Gln Gly Leu
   50 55 60
   Asp Tyr Asn Lys Asn Asn Val Leu Val Tyr His Gly Asp Ala Val Thr
   65 70 75 80
   Asn Val Pro Pro Arg Lys Gly Tyr Lys Asp Gly Asn Glu Tyr Ile Val
   85 90 95
   Val Glu Lys Lys Lys Lys Ser Ile Asn Gln Asn Asn Ala Asp Ile Gln
   100 105 110
   Val Val Asn Ala Ile Ser Ser Leu Thr Tyr Pro Gly Ala Leu Val Lys
   115 120 125
   Ala Asn Ser Glu Leu Val Glu Asn Gln Pro Asp Val Leu Pro Val Lys
   130 135 140
   Arg Asp Ser Leu Thr Leu Ser Ile Asp Leu Pro Gly Met Thr Asn Gln
   145 150 155 160
   Asp Asn Lys Ile Val Val Lys Asn Ala Thr Lys Ser Asn Val Asn Asn
   165 170 175
   Ala Val Asn Thr Leu Val Glu Arg Trp Asn Glu Lys Tyr Ala Gln Ala
   180 185 190
   Tyr Pro Asn Val Ser Ala Lys Ile Asp Tyr Asp Asp Glu Met Ala Tyr
   195 200 205
   Ser Glu Ser Gln Leu Ile Ala Lys Phe Gly Thr Ala Phe Lys Ala Val
   210 215 220
   Asn Asn Ser Leu Asn Val Asn Phe Gly Ala Ile Ser Glu Gly Lys Met
   225 230 235 240
   Gln Glu Glu Val Ile Ser Phe Lys Gln Ile Tyr Tyr Asn Val Asn Val
   245 250 255
   Asn Glu Pro Thr Arg Pro Ser Arg Phe Phe Gly Lys Ala Val Thr Lys
   260 265 270
   Glu Gln Leu Gln Ala Leu Gly Val Asn Ala Glu Asn Pro Pro Ala Tyr
   275 280 285
   Ile Ser Ser Val Ala Tyr Gly Arg Gln Val Tyr Leu Lys Leu Ser Thr
   290 295 300
   Asn Ser His Ser Thr Lys Val Lys Ala Ala Phe Asp Ala Ala Val Ser
   305 310 315 320
   Gly Lys Ser Val Ser Gly Asp Val Glu Leu Thr Asn Ile Ile Lys Asn
   325 330 335
   Ser Ser Phe Lys Ala Val Ile Tyr Gly Gly Ser Ala Lys Asp Glu Val
   340 345 350
   Gln Ile Ile Asp Gly Asn Leu Gly Asp Leu Arg Asp Ile Leu Lys Lys
   355 360 365
   Gly Ala Thr Phe Asn Arg Glu Thr Pro Gly Val Pro Ile Ala Tyr Thr
   370 375 380
   Thr Asn Phe Leu Lys Asp Asn Glu Leu Ala Val Ile Lys Asn Asn Ser
   385 390 395 400
   Glu Tyr Ile Glu Thr Thr Ser Lys Ala Tyr Thr Asp Gly Lys Ile Asn
   405 410 415
   Ile Asp His Ser Gly Gly Tyr Val Ala Gln Phe Asn Ile Ser Trp Asp
   420 425 430
   Glu Ile Asn Tyr Asp Pro Glu Gly Asn Glu Ile Val Gln His Lys Asn
   435 440 445
   Trp Ser Glu Asn Asn Lys Ser Lys Leu Ala His Phe Thr Ser Ser Ile
   450 455 460
   Tyr Leu Pro Gly Asn Ala Arg Asn Ile Asn Val Tyr Ala Lys Glu Cys
   465 470 475 480
   Thr Gly Leu Ala Trp Glu Trp Trp Arg Thr Val Ile Asp Asp Arg Asn
   485 490 495
   Leu Pro Leu Val Lys Asn Arg Asn Ile Ser Ile Trp Gly Thr Thr Leu
   500 505 510
   Tyr Pro Lys Tyr Ser Asn Ser Val Asp Asn Pro Ile Glu Pro Gly
   515 520 525
<210> 19
   <211> 1266
   <212> DNA
   <213> Künstliche Sequenz
<220>
   <221> CDS
   <222> (1)..(1266)
<220>
   <221> misc_feature
   <222> (445)..(558)
   <223> Insertion einer WW-Domäne
<220>
   <223> Beschreibung der künstlichen Sequenz: Variation des Polyomavirus Hüllproteins VP1 (PyVP1-WW150)
<400> 19
   atg gcc ccc aaa aga aaa agc ggc gtc tct aaa agc gag aca aaa agc 48
   Met Ala Pro Lys Arg Lys Ser Gly Val Ser Lys Ser Glu Thr Lys Ser
   1 5 10 15
   aca aag gct agc cca aga ccc gca ccc gtt ccc aaa ctg ctt att aaa 96
   Thr Lys Ala Ser Pro Arg Pro Ala Pro Val Pro Lys Leu Leu Ile Lys
   20 25 30
   ggg ggt atg gag gtg ctg gac ctt gtg aca ggg cca gac agt gtg aca 144
   Gly Gly Met Glu Val Leu Asp Leu Val Thr Gly Pro Asp Ser Val Thr
   35 40 45
   gaa ata gaa gct ttt ctg aac ccc aga atg ggg cag cca ccc acc cct 192
   Glu Ile Glu Ala Phe Leu Asn Pro Arg Met Gly Gln Pro Pro Thr Pro
   50 55 60
   gaa agc cta aca gag gga ggg caa tac tat ggt tgg agc aga ggg att 240
   Glu Ser Leu Thr Glu Gly Gly Gln Tyr Tyr Gly Trp Ser Arg Gly Ile
   65 70 75 80
   aat ttg gct aca tca gat aca gag gat tcc cca gga aat aat aca ctt    288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac    336
   Pro Thr Trp Ser Met Ala Lys Leu Gln Leu Pro Met Leu Asn Glu Asp
               100                 105                 110
   ctc acg tct gac acc cta caa atg tgg gag gca gtc tca gtg aaa acc    384
   Leu Thr Ser Asp Thr Leu Gln Met Trp Glu Ala Val Ser Val Lys Thr
           115                 120                 125
   gag gtg gtg ggc tct ggc tca ctg tta gat gtg cat ggg ttc aac aaa    432
   Glu Val Val Gly Ser Gly Ser Leu Leu Asp Val His Gly Phe Asn Lys
       130                 135                 140
   ccc aca gat aca ggc agc ggc agc ggc tgg aca gaa cat aaa tca cct    480
   Pro Thr Asp Thr Gly Ser Gly Ser Gly Trp Thr Glu His Lys Ser Pro
   145                 150                 155                 160
   gat gga agg act tat tat tac aat act gaa aca aaa cag tct acc tgg    528
   Asp Gly Arg Thr Tyr Tyr Tyr Asn Thr Glu Thr Lys Gln Ser Thr Trp
                   165                 170                 175
   gaa aag cca gat gat ggt agt ggt agc ggc gta aac aca aaa gga att    576
   Glu Lys Pro Asp Asp Gly Ser Gly Ser Gly Val Asn Thr Lys Gly Ile
               180                 185                 190
   tcc act cca gtg gaa ggc agc caa tat cat gtg ttt gct gtg ggc ggg    624
   Ser Thr Pro Val Glu Gly Ser Gln Tyr His Val Phe Ala Val Gly Gly
           195                 200                 205
   gaa ccg ctt gac ctc cag gga ctt gtg aca gat gcc aga aca aaa tac    672
   Glu Pro Leu Asp Leu Gln Gly Leu Val Thr Asp Ala Arg Thr Lys Tyr
       210                 215                 220
   aag gaa gaa ggg gta gta aca atc aaa aca atc aca aag aag gac atg    720
   Lys Glu Glu Gly Val Val Thr Ile Lys Thr Ile Thr Lys Lys Asp Met
   225                 230                 235                 240
   gtc aac aaa gac caa gtc ctg aat cca att agc aag gcc aag ctg gat    768
   Val Asn Lys Asp Gln Val Leu Asn Pro Ile Ser Lys Ala Lys Leu Asp
                   245                 250                 255
   aag gac gga atg tat cca gtt gaa atc tgg cat cca gat cca gca aaa    816
   Lys Asp Gly Met Tyr Pro Val Glu Ile Trp His Pro Asp Pro Ala Lys
               260                 265                 270
   aat gag aac aca agg tac ttt ggc aat tac act gga ggc acg tgc acc     864
   Asn Glu Asn Thr Arg Tyr Phe Gly Asn Tyr Thr Gly Gly Thr Cys Thr
           275                 280                 285
   cca ccc gtc ctg cag ttc aca aac acc ctg aca act gtg ctc cta gat     912
   Pro Pro Val Leu Gln Phe Thr Asn Thr Leu Thr Thr Val Leu Leu Asp
       290                 295                 300
   gaa aat gga gtt ggg ccc ctc agc aaa gga gaa ggt cta tac ctc tcg     960
   Glu Asn Gly Val Gly Pro Leu Ser Lys Gly Glu Gly Leu Tyr Leu Ser
   305                 310                 315                 320
   agc gta gat ata atg ggc tgg aga gtt aca aga aac tat gat gtc cat     1008
   Ser Val Asp Ile Met Gly Trp Arg Val Thr Arg Asn Tyr Asp Val His
                   325                 330                 335
   cac tgg aga ggg ctt ccc aga tat ttc aaa atc acc ctg aga aaa aga     1056
   His Trp Arg Gly Leu Pro Arg Tyr Phe Lys Ile Thr Leu Arg Lys Arg
               340                 345                 350
   tgg gtc aaa aat ccc tat ccc atg gcc tcc ctc ata agt tcc ctt ttc     1104
   Trp Val Lys Asn Pro Tyr Pro Met Ala Ser Leu Ile Ser Ser Leu Phe
           355                 360                 365
   aac aac atg ctc ccc caa gtg cag ggc caa ccc atg gaa ggg gag aac     1152
   Asn Asn Met Leu Pro Gln Val Gln Gly Gln Pro Met Glu Gly Glu Asn
       370                 375                 380
   acc cag gta gag gag gtt aga gtg tat gat ggg act gaa cct gta ccg     1200
   Thr Gln Val Glu Glu Val Arg Val Tyr Asp Gly Thr Glu Pro Val Pro
   385                 390                 395                 400
   ggg gac cct gat atg acg cgc tat gtt gac cgc ttt gga aaa aca aag     1248
   Gly Asp Pro Asp Met Thr Arg Tyr Val Asp Arg Phe Gly Lys Thr Lys
                   405                 410                 415
   act gta ttt cct ccc ggg                                             1266
   Thr Val Phe Pro Pro Gly
               420
<210> 20
   <211> 422
   <212> PRT
   <213> Künstliche Sequenz
   <223> Beschreibung der künstlichen Sequenz: Variation des Polyomavirus Hüllproteins VP1 (PyVP1-WW150)
<400> 20
   Met Ala Pro Lys Arg Lys Ser Gly Val Ser Lys Ser Glu Thr Lys Ser
    1               5                  10                  15
   Thr Lys Ala Ser Pro Arg Pro Ala Pro Val Pro Lys Leu Leu Ile Lys
               20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
   65                  70                  75                  80
   Asn Leu Ala Thr Ser Asp Thr Glu Asp Ser Pro Gly Asn Asn Thr Leu
                   85                  90                  95
   Pro Thr Trp Ser Met Ala Lys Leu Gln Leu Pro Met Leu Asn Glu Asp
               100                 105                 110
   Leu Thr Ser Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Gly Ser Gly Ser Gly Trp Thr Glu His Lys Ser Pro
   145                 150                 155                 160
   Asp Gly Arg Thr Tyr Tyr Tyr Asn Thr Glu Thr Lys Gln Ser Thr Trp
                   165                 170                 175
   Glu Lys Pro Asp Asp Gly Ser Gly Ser Gly Val Asn Thr Lys Gly Ile
               180                 185                 190
   Ser Thr Pro Val Glu Gly Ser Gln Tyr His Val Phe Ala Val Gly Gly
           195                 200                 205
   Glu Pro Leu Asp Leu Gln Gly Leu Val Thr Asp Ala Arg Thr Lys Tyr
       210                 215                 220
   Lys Glu Glu Gly Val Val Thr Ile Lys Thr Ile Thr Lys Lys Asp Met
   225                 230                 235                 240
   Val Asn Lys Asp Gln Val Leu Asn Pro Ile Ser Lys Ala Lys Leu Asp
                   245                 250                 255
   Lys Asp Gly Met Tyr Pro Val Glu Ile Trp His Pro Asp Pro Ala Lys
               260                 265                 270
   Asn Glu Asn Thr Arg Tyr Phe Gly Asn Tyr Thr Gly Gly Thr Cys Thr
           275                 280                 285
   Pro Pro Val Leu Gln Phe Thr Asn Thr Leu Thr Thr Val Leu Leu Asp
       290                 295                 300
   Glu Asn Gly Val Gly Pro Leu Ser Lys Gly Glu Gly Leu Tyr Leu Ser
   305                 310                 315                 320
   Ser Val Asp Ile Met Gly Trp Arg Val Thr Arg Asn Tyr Asp Val His
                   325                 330                 335
   His Trp Arg Gly Leu Pro Arg Tyr Phe Lys Ile Thr Leu Arg Lys Arg
               340                 345                 350
   Trp Val Lys Asn Pro Tyr Pro Met Ala Ser Leu Ile Ser Ser Leu Phe
           355                 360                 365
   Asn Asn Met Leu Pro Gln Val Gln Gly Gln Pro Met Glu Gly Glu Asn
       370                 375                 380
   Thr Gln Val Glu Glu Val Arg Val Tyr Asp Gly Thr Glu Pro Val Pro
   385                 390                 395                 400
   Gly Asp Pro Asp Met Thr Arg Tyr Val Asp Arg Phe Gly Lys Thr Lys
                   405                 410                 415
   Thr Val Phe Pro Pro Gly
               420
<210> 21
   <211> 1152
   <212> DNA
   <213> Künstliche Sequenz
<220>
   <221> CDS
   <222> (1)..(1152)
<220>
   <223> Beschreibung der künstlichen Sequenz: Sequenz des Polyomavirus Hüllproteins VP1 (PyVP1-wt) mit Austausch der beiden letzten Aminosäuren 383 / 384
<400> 21
   atg gcc ccc aaa aga aaa agc ggc gtc tct aaa tgc gag aca aaa tgt    48
   Met Ala Pro Lys Arg Lys Ser Gly Val Ser Lys Cys Glu Thr Lys Cys
    1               5                  10                  15
   aca aag gcc tgt cca aga ccc gca ccc gtt ccc aaa ctg ctt att aaa    96
   Thr Lys Ala Cys Pro Arg Pro Ala Pro Val Pro Lys Leu Leu Ile Lys
               20                  25                  30
   ggg ggt atg gag gtg ctg gac ctt gtg aca ggg cca gac agt gtg aca   144
   Gly Gly Met Glu Val Leu Asp Leu Val Thr Gly Pro Asp Ser Val Thr
           35                  40                  45
   gaa ata gaa gct ttt ctg aac ccc aga atg ggg cag cca ccc acc cct   192
   Glu Ile Glu Ala Phe Leu Asn Pro Arg Met Gly Gln Pro Pro Thr Pro
       50                  55                  60
   gaa agc cta aca gag gga ggg caa tac tat ggt tgg agc aga ggg att   240
   Glu Ser Leu Thr Glu Gly Gly Gln Tyr Tyr Gly Trp Ser Arg Gly Ile
   65                  70                  75                  80
   aat ttg gct aca tca gat aca gag gat tcc cca gga aat aat aca ctt   288
   Asn Leu Ala Thr Ser Asp Thr Glu Asp Ser Pro Gly Asn Asn Thr Leu
                   85                  90                  95
   ccc aca tgg agt atg gca aag ctc cag ctt ccc atg ctc aat gag gac   336
   Pro Thr Trp Ser Met Ala Lys Leu Gln Leu Pro Met Leu Asn Glu Asp
               100                 105                 110
   ctc acc tgt gac acc cta caa atg tgg gag gca gtc tca gtg aaa acc   384
   Leu Thr Cys Asp Thr Leu Gln Met Trp Glu Ala Val Ser Val Lys Thr
           115                 120                 125
   gag gtg gtg ggc tct ggc tca ctg tta gat gtg cat ggg ttc aac aaa   432
   Glu Val Val Gly Ser Gly Ser Leu Leu Asp Val His Gly Phe Asn Lys
       130                 135                 140
   ccc aca gat aca gta aac aca aaa gga att tcc act cca gtg gaa ggc   480
   Pro Thr Asp Thr Val Asn Thr Lys Gly Ile Ser Thr Pro Val Glu Gly
   145                 150                 155                 160
   agc caa tat cat gtg ttt gct gtg ggc ggg gaa ccg ctt gac ctc cag   528
   Ser Gln Tyr His Val Phe Ala Val Gly Gly Glu Pro Leu Asp Leu Gln
                   165                 170                 175
   gga ctt gtg aca gat gcc aga aca aaa tac aag gaa gaa ggg gta gta   576
   Gly Leu Val Thr Asp Ala Arg Thr Lys Tyr Lys Glu Glu Gly Val Val
               180                 185                 190
   aca atc aaa aca atc aca aag aag gac atg gtc aac aaa gac caa gtc    624
   Thr Ile Lys Thr Ile Thr Lys Lys Asp Met Val Asn Lys Asp Gln Val
           195                 200                 205
   ctg aat cca att agc aag gcc aag ctg gat aag gac gga atg tat cca    672
   Leu Asn Pro Ile Ser Lys Ala Lys Leu Asp Lys Asp Gly Met Tyr Pro
       210                 215                 220
   gtt gaa atc tgg cat cca gat cca gca aaa aat gag aac aca agg tac    720
   Val Glu Ile Trp His Pro Asp Pro Ala Lys Asn Glu Asn Thr Arg Tyr
   225                 230                 235                 240
   ttt ggc aat tac act gga ggc aca aca act cca ccc gtc ctg cag ttc    768
   Phe Gly Asn Tyr Thr Gly Gly Thr Thr Thr Pro Pro Val Leu Gln Phe
                   245                 250                 255
   aca aac acc ctg aca act gtg ctc cta gat gaa aat gga gtt ggg ccc    816
   Thr Asn Thr Leu Thr Thr Val Leu Leu Asp Glu Asn Gly Val Gly Pro
               260                 265                 270
   ctc tgt aaa gga gag ggc cta tac ctc tcc tgt gta gat ata atg ggc    864
   Leu Cys Lys Gly Glu Gly Leu Tyr Leu Ser Cys Val Asp Ile Met Gly
           275                 280                 285
   tgg aga gtt aca aga aac tat gat gtc cat cac tgg aga ggg ctt ccc    912
   Trp Arg Val Thr Arg Asn Tyr Asp Val His His Trp Arg Gly Leu Pro
       290                 295                 300
   aga tat ttc aaa atc acc ctg aga aaa aga tgg gtc aaa aat ccc tat    960
   Arg Tyr Phe Lys Ile Thr Leu Arg Lys Arg Trp Val Lys Asn Pro Tyr
   305                 310                 315                 320
   ccc atg gcc tcc ctc ata agt tcc ctt ttc aac aac atg ctc ccc caa    1008
   Pro Met Ala Ser Leu Ile Ser Ser Leu Phe Asn Asn Met Leu Pro Gln
                   325                 330                 335
   gtg cag ggc caa ccc atg gaa ggg gag aac acc cag gta gag gag gtt    1056
   Val Gln Gly Gln Pro Met Glu Gly Glu Asn Thr Gln Val Glu Glu Val
               340                 345                 350
   aga gtg tat gat ggg act gaa cct gta ccg ggg gac cct gat atg acg    1104
   Arg Val Tyr Asp Gly Thr Glu Pro Val Pro Gly Asp Pro Asp Met Thr
           355                 360                 365
   cgc tat gtt gac cgc ttt gga aaa aca aag act gta ttt cct ccc ggg    1152
   Arg Tyr Val Asp Arg Phe Gly Lys Thr Lys Thr Val Phe Pro Pro Gly
   370 375 380
<210> 22
   <211> 384
   <212> PRT
   <213> Künstliche Sequenz
   <223> Beschreibung der künstlichen Sequenz: Sequenz des Polyomavirus Hüllproteins VP1 (PyVP1-wt) mit Austausch der beiden letzten Aminosäuren 383 / 384
<400> 22
   Met Ala Pro Lys Arg Lys Ser Gly Val Ser Lys Cys Glu Thr Lys Cys
     1               5                  10                  15
   Thr Lys Ala Cys Pro Arg Pro Ala Pro Val Pro Lys Leu Leu Ile Lys
                   20                  25                  30
   Gly Gly Met Glu Val Leu Asp Leu Val Thr Gly Pro Asp Ser Val Thr
           35                  40                  45
   Glu Ile Glu Ala Phe Leu Asn Pro Arg Met Gly Gln Pro Pro Thr Pro
       50                  55                  60
   Glu Ser Leu Thr Glu Gly Gly Gln Tyr Tyr Gly Trp Ser Arg Gly Ile
    65                  70                  75                  80
   Asn Leu Ala Thr Ser Asp Thr Glu Asp Ser Pro Gly Asn Asn Thr Leu
                    85                  90                  95
   Pro Thr Trp Ser Met Ala Lys Leu Gln Leu Pro Met Leu Asn Glu Asp
               100                 105                 110
   Leu Thr Cys Asp Thr Leu Gln Met Trp Glu Ala Val Ser Val Lys Thr
           115                 120                 125
   Glu Val Val Gly Ser Gly Ser Leu Leu Asp Val His Gly Phe Asn Lys
       130                 135                 140
   Pro Thr Asp Thr Val Asn Thr Lys Gly Ile Ser Thr Pro Val Glu Gly
   145                 150                 155                 160
   Ser Gln Tyr His Val Phe Ala Val Gly Gly Glu Pro Leu Asp Leu Gln
                   165                 170                 175
   Gly Leu Val Thr Asp Ala Arg Thr Lys Tyr Lys Glu Glu Gly Val Val
               180                 185                 190
   Thr Ile Lys Thr Ile Thr Lys Lys Asp Met Val Asn Lys Asp Gln Val
           195                 200                 205
   Leu Asn Pro Ile Ser Lys Ala Lys Leu Asp Lys Asp Gly Met Tyr Pro
       210                 215                 220
   Val Glu Ile Trp His Pro Asp Pro Ala Lys Asn Glu Asn Thr Arg Tyr
   225                 230                 235                 240
   Phe Gly Asn Tyr Thr Gly Gly Thr Thr Thr Pro Pro Val Leu Gln Phe
                   245                 250                 255
   Thr Asn Thr Leu Thr Thr Val Leu Leu Asp Glu Asn Gly Val Gly Pro
               260                 265                 270
   Leu Cys Lys Gly Glu Gly Leu Tyr Leu Ser Cys Val Asp Ile Met Gly
           275                 280                 285
   Trp Arg Val Thr Arg Asn Tyr Asp Val His His Trp Arg Gly Leu Pro
       290                 295                 300
   Arg Tyr Phe Lys Ile Thr Leu Arg Lys Arg Trp Val Lys Asn Pro Tyr
   305                 310                 315                 320
   Pro Met Ala Ser Leu Ile Ser Ser Leu Phe Asn Asn Met Leu Pro Gln
                   325                 330                 335
   Val Gln Gly Gln Pro Met Glu Gly Glu Asn Thr Gln Val Glu Glu Val
               340                 345                 350
   Arg Val Tyr Asp Gly Thr Glu Pro Val Pro Gly Asp Pro Asp Met Thr
           355                 360                 365
   Arg Tyr Val Asp Arg Phe Gly Lys Thr Lys Thr Val Phe Pro Pro Gly
       370                 375                 380